# EUROPEAN PATENT SPECIFICATION

(11) **EP 4 087 836 B1**
(45) Date of publication and mention of the grant of the patent: **08.10.2025**
(21) Application number: 21704972.5
(22) Date of filing: 07.01.2021
(51) Int. Cl.: C07D 249/04, A61K 31/4192, A61P 13/00, A61P 13/10, A61P 13/12

(54) **PROCESSES FOR PREPARING TRIAZOLE GLYCOLATE OXIDASE INHIBITORS**
VERFAHREN ZUR HERSTELLUNG VON TRIAZOLGLYKOLAT-OXIDASE-INHIBITOREN
PROCÉDÉS DE PRÉPARATION D'INHIBITEURS DE GLYCOLATE OXYDASE DE TRIAZOLE

(30) Priority: 08.01.2020 US 202062958443 P; 08.01.2020 US 202062958445 P
(43) Date of publication of application: 16.11.2022
(73) Proprietor: Cantero Therapeutics, Inc., Palo Alto, CA 94304 (US)
(72) Inventor: AKBARIROMANI, Elham, Palo Alto, California 94301 (US); CHAND, Pooran, Palo Alto, California 94301 (US); CHOUDHARY, Sunil Kumar, Palo Alto, California 94301 (US); FERNANDES, Miguel Xavier, Palo Alto, California 94301 (US); LEBLANC, Yves, Palo Alto, California 94301 (US); MAAG, Hans, Palo Alto, California 94301 (US); REVU, Omkar, Palo Alto, California 94301 (US); SHEKHAWAT, Pavan, Palo Alto, California 94301 (US)
(74) Representative: J A Kemp LLP
(86) International application number: PCT/US2021/012545
(87) International publication number: WO 2021/142155

(56) References cited:
- WO-A1-2019/165159
- WO-A1-2020/010309
- WO-A2-2019/133770

## Description

### CROSS-REFERENCES TO RELATED APPLICATIONS

This application claims priority benefit under 35 U.S.C. § 119(e) to each of U.S. Provisional Application No. 62/958,443 filed January 8, 2020 and 62/958,445, filed January 8, 2020.

### BACKGROUND

Kidney stone disease (KSD) has a prevalence of approximately 10% in developed countries with lifetime recurrence rates of up to 50% [Johri, et al. (2010) Nephron Clin Pract. 116: c159]. KSD patients present with hematuria and renal colic and medical treatment is essentially symptomatic. The administration of drugs to facilitate stone passage is effective for small stones (< 5mm). For bigger stones, extracorporeal sound waves or minimally invasive surgery are used to break the stone into small pieces that can more easily pass the urinary tract [Coe et al. (2005) J. Clin. Invest. 115: 2598].

Approximately 75% of kidney stones contain primarily calcium oxalate and elevated levels of urinary oxalate are found in up to 50% of KSD patients. Furthermore, increased levels of urinary oxalate increase the risk of forming kidney stones [Moe (2006) Lancet 367: 333; Sakhaee (2009) Kidney Int. 75: 585; Kaufman et al. (2008) J Am Soc Nephrol. 19: 1197]. In mammals, calcium has vital physiological roles in so many processes that its levels are tightly regulated. Oxalate, however, is a metabolic end-product with no known physiological role. Oxalate is a divalent anion that must be eliminated with the urine and tends to precipitate as tissue-damaging insoluble calcium oxalate crystals.

Primary hyperoxalurias (PH) are a group of rare metabolic diseases, with autosomal recessive inheritance, affecting the glyoxylate or the hydroxyproline pathways. All of them have in common an overproduction of oxalate. So far, three forms of primary hyperoxaluria have been identified. They are referred as primary hyperoxaluria types 1, 2, and 3. Primary hyperoxaluria type 1 (PH1) is caused by mutation of liver-specific enzyme alanine-glyoxylate aminotransferase (AGT). Primary hyperoxaluria type 2 (PH2) is caused by mutation of glyoxylate reductase-hydroxypyruvate reductase (GRHPR). Primary hyperoxaluria type 3 (PH3) is caused by mutation of 4-hydroxy-2-oxoglutarate aldolase (HOGA1). PH1 eventually leads to renal failure after several years. PH2 and PH3 have a less severe course. Approximately 80% of PH patients suffer PH1, the most severe PH type. Considering its statistical predominance, most studies on PH essentially refer to PH1 [Salido et al. (2012) Biochim Biophys Acta. 1822: 1453].

Since calcium levels are so tightly regulated in the organism, changing them in urine is extremely difficult, and it may also produce undesired effects in vital physiological processes. Minor increases in urinary oxalate can produce large effect on calcium oxalate crystal formation, and elevated levels of urinary oxalate are a major risk factor for the formation of calcium oxalate kidney stones [Pak, et al. (2004) Kidney Int. 66: 2032]. Consequently, a small decrease in oxalate concentration could lower the calcium oxalate level below saturation, and thus prevent calcium oxalate stone formation. Irrespective of the urinary oxalate (UOx) levels in individuals with kidney stone disease, primary hyperoxaluria, or secondary hyperoxaluria, lowering UOx levels will decrease the contribution of oxalate to calcium oxalate formation, and thus lower the probability of stone formation and/or alleviate the severity of excessive calcium oxalate deposition related conditions [Marengo et al. (2008) Nat Clin Pract Nephrol. 4: 368].

The development of an effective drug that reduces urinary oxalate levels can be a valuable therapeutic option in the prophylaxis and treatment of conditions related to calcium oxalate. Common approaches for treatment of urolithiasis due to calcium oxalate include surgical removal of stones, dietary changes increase fluid intake and to restrict oxalate intake, urine alkalization, diuretics, and crystallization inhibitors such as citrate, bicarbonate, and magnesium [Moe, *supra*]. However, none of these therapeutic approaches tackles the origin of the conditions. No drug which specifically inhibits the endogenous biosynthetic formation of oxalate is commercially available for the prophylaxis and treatment of calcium oxalate deposition related conditions.

In humans, dietary oxalate contributes only 10-50% to the amount of excreted urinary oxalate [Holmes, et al. (2001) Kidney Int. 59: 270]. Most urinary oxalate is derived from the endogenous metabolism, mainly in liver. In humans, the major precursor of oxalate is glyoxylate. Therefore, approaches to reduce the production of oxalate must block the conversion of glyoxylate into oxalate, or block the production of glyoxylate from its precursors. In humans, the major precursor of glyoxylate is glycolate in a reaction catalyzed by the peroxisomal liver enzyme glycolate oxidase (GO), also termed hydroxyacid oxidase 1. Pharmacological inhibition of GO activity with small molecules will diminish endogenous oxalate production and lead to a reduction of calcium oxalate levels in the urine, thus providing a specific approach for prophylaxis and treatment of calcium oxalate deposition and related conditions. There is evidence that GO is a safe therapeutic target in humans. A report describes a finding where a defective splice variant of human GO in an individual simply causes isolated asymptomatic glycolic aciduria with no apparent ill effects [Frishberg, et al. (2014) J Med Genet. 51: 526].

Recently, a series of 1,2,3-triazole-4-carboxylic acids have been described for the treatment of GO related diseases. While initial processes for preparing such compounds have also been described, there remains a need for improvements in those processes which can be run on a large scale and increased yield.

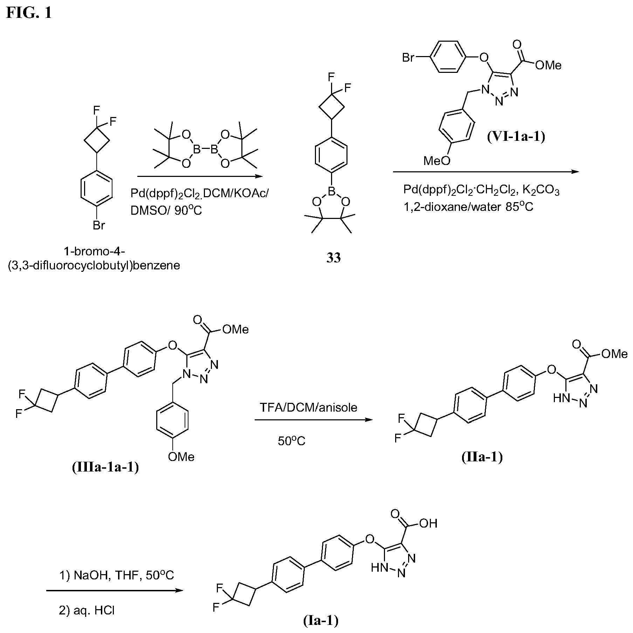

WO 2019/165159 describes compounds and compositions for modulating glycolate oxidase. WO 2019/133770 describes compounds, methods of making such compounds, pharmaceutical compositions and medicaments containing such compounds, and methods of using such compounds to treat or prevent diseases or disorders associated with the enzyme glycolate oxidase (GO). As described in PCT/US2019/040690 (WO 2020/010309, referred herein as the '690 application), a compound of formula (Ia-1): 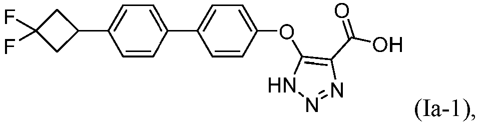 was synthesized according to the scheme as shown in FIG. 1. A compound of formula (IIIa-1a-1) was obtained by a Suzuki coupling reaction, which required 3.36 equivalents of Compound (33) (2-(4-(3,3-difluorocyclobutyl)phenyl)-4,4,5,5-tetramethyl-1,3,2-dioxaborolane), relative to a compound of formula (VI-1a-1). Compound (33) was prepared from 1-bromo-4-(3,3-difluorocyclobutyl)benzene in a yield of about 59% and required further purification. With respect to the Suzuki coupling reaction, the compound of formula (IIIa-1a-1) was isolated in a yield of about 51%. By using the Suzuki reaction as described in the '690 application, the compound of formula (IIIa-1a-1) was isolated in an overall yield of only about 30% in two steps from the compound of formula (VI-1a-1).

Despite the above described process, there remains a need for the development of more efficient and improved processes for preparing of 1,2,3-triazole-4-carboxylic acid related compounds, in particular cycloalkyl diphenyl 1,2,3-triazole-4-carboxylic acid compounds.

### BRIEF SUMMARY

In one aspect, the present disclosure provides a process for preparing a compound represented by formula (II): 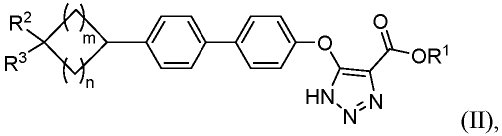 a tautomer thereof, or a salt thereof. The process includes:
a) contacting a compound of formula (IV): 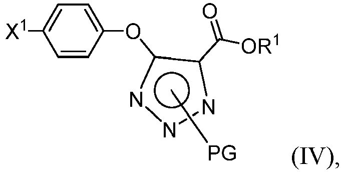 or a salt thereof, with a compound of formula (V): 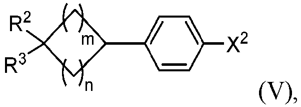 a first transition-metal catalyst, and a first base in a first solvent to form a compound of formula (III): 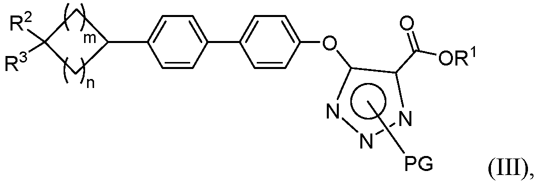 or a salt thereof; and
b) removing the PG group of the compound of formula (III) or the salt thereof to provide the compound of formula (II), the tautomer thereof, or the salt thereof,
wherein:
subscripts m and n are each independently 1 or 2;
R¹ is C₁₋₆ alkyl;
R² and R³ are each independently H or halogen;
X¹ represents a boron-containing group;
X² is halogen or a sulfonate; and 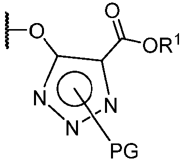 in formula (IV) or (III) is represented by the formula: 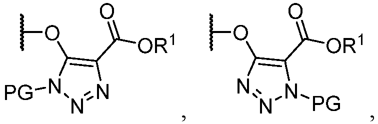
or a mixture thereof; and
PG is an amine-protecting group.

In another aspect, the present disclosure provides a process for preparing a compound represented by the formula (Ia-1): 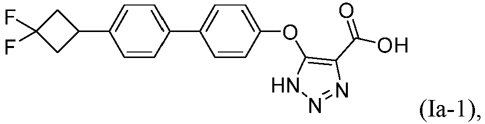 a tautomer thereof, or a salt thereof. The process includes:
a1) converting a compound of formula (VI-1a-1): 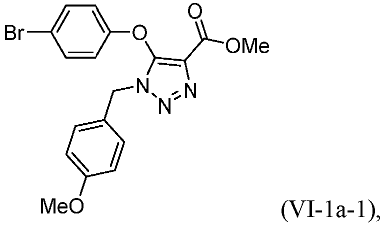 or a salt thereof, with bis(pinacolato)diboron, Pd(dppf)Cl₂·CH₂Cl₂, and potassium acetate in 1,4-dioxane to a compound of formula (IV-1a-2): 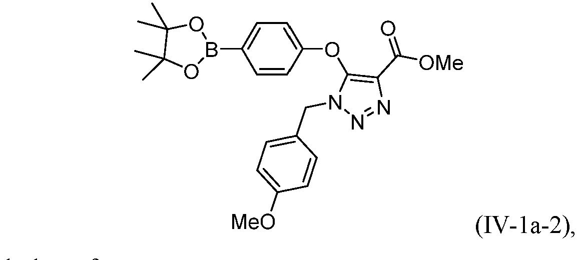 or a salt thereof;
a) contacting the compound of formula (IV-1a-2) or the salt thereof with 1-bromo-4-(3,3-difluorocyclobutyl)benzene, Pd(dppf)Cl₂·CH₂Cl₂, and potassium carbonate in a mixture of 1,4-dioxane and water to form a compound of formula (IIIa-1a-1): 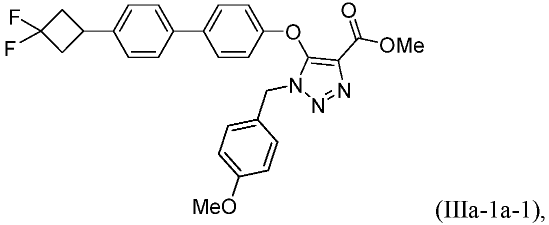 or a salt thereof;
b) treating the compound of formula (IIIa-1a-1) or the salt thereof, with trifluoroacetic acid and anisole in dichloromethane to provide a compound of formula (IIa-1): 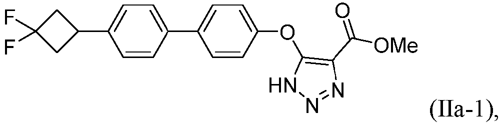 a tautomer thereof, or a salt thereof;
c) saponifying the compound of formula (IIa-1), the tautomer thereof, or the salt thereof with an aqueous solution of sodium hydroxide in tetrahydrofuran; and
d) acidifying with an aqueous solution of HCl to provide the compound of formula (Ia-1), the tautomer thereof, or the salt thereof.

Described herein but not encompassed by the wording of the claims are compounds represented by formula (X): 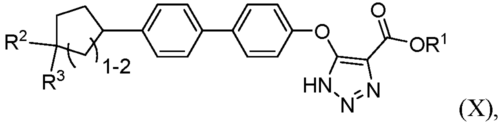 a tautomer thereof, or a pharmaceutically acceptable salt thereof, wherein R¹ is H or C₁₋₆ alkyl; and R² and R³ are each independently H or halogen.

Also described herein but not encompassed by the wording of the claims are pharmaceutical compositions including a compound of formula (X), a tautomer thereof, or a pharmaceutically acceptable salt thereof, and a pharmaceutically acceptable excipient.

Also described herein but not encompassed by the wording of the claims are methods for inhibiting glycolate oxidase with a compound of formula (X) in a subject for the treatment of primary hyperoxaluria, type I (PH1), and kidney and/or bladder stones.

### BRIEF DESCRIPTION OF THE DRAWINGS

FIG. 1 shows the synthesis scheme for preparing a compound of formula (Ia-1) as described in PCT/US2019/040690.
FIG. 2 shows a synthesis scheme for preparing a compound of formula (I).
FIG. 3 shows a synthesis scheme for preparing a compound of formula (Ia-1).
FIG. 4 shows a synthesis scheme for preparing a compound of formula (VI-1a-1).
FIG. 5 shows a synthesis scheme for preparing 1-bromo-4-(3,3-difluorocyclobutyl)benzene.
FIG. 6 shows a synthesis scheme for preparing compounds of formulae (Ib-1) and (IIb-1).
FIG. 7 shows a synthesis scheme for preparing compounds of formulae (Ic-1) and (IIc-1).
FIG. 8 shows a synthesis scheme for preparing an intermediate used in preparation of compounds of formulae (Ib-1), (IIb-1), (Ic-1), and (IIc-1).
FIG. 9 shows the catalytic reactions used for assaying glycolate oxidase activity.

### DETAILED DESCRIPTION

### I. GENERAL

The present disclosure provides processes for preparing 1,2,3-triazole-4-carboxylic acid related compounds of formula (I) and (II) via a Suzuki coupling reaction. The Suzuki coupling reaction is achieved by coupling a compound of formula (IV), a boron-containing derivative of 1,2,3-triazole-4-carboxylate, with a cycloalkyl phenyl halide or sulfonate of formula (V). The Suzuki coupling reaction provides an excellent yield of about 61% in two steps starting from a compound of formula (VI), which is an amine-protected 5-(4-halo-phenoxy)-1H-1,2,3-triazole-4-carboxylate.

Also described but not encompassed by the wording of the claims are effective therapeutic approaches for inhibiting biosynthetic formation of oxalate and for treating primary hyperoxaluria, type I (PH1) and other conditions related to deposition of calcium oxalate. Novel cycloalkyl triazole compounds which are useful as glycolate oxidase inhibitors are described, as well as methods for making and using the cycloalkyl triazole compounds.

### II. DEFINITIONS

"Alkyl" refers to a straight or branched, saturated, aliphatic radical having the number of carbon atoms indicated (i.e., C₁₋₆ means one to six carbons). Alkyl can include any number of carbons, such as C₁₋₂, C₁₋₃, C₁₋₄, C₁₋₅, C₁₋₆, C₁₋₇, C₁₋₈, C₁₋₉, C₁₋₁₀, C₂₋₃, C₂₋₄, C₂₋₅, C₂₋₆, C₃₋₄, C₃₋₅, C₃₋₆, C₄₋₅, C₄₋₆ and C₅₋₆. For example, C₁₋₆ alkyl includes, but is not limited to, methyl, ethyl, propyl, isopropyl, butyl, isobutyl, sec-butyl, tert-butyl, pentyl, isopentyl, hexyl, etc. Alkyl can also refer to alkyl groups having up to 20 carbon atoms, such as, but not limited to, heptyl, octyl, nonyl, decyl, etc.

"Alkylene" refers to a straight or branched, saturated, aliphatic radical having the number of carbon atoms indicated (i.e., C₁₋₆ means one to six carbons), and linking at least two other groups, *i.e.,* a divalent hydrocarbon radical. The two moieties linked to the alkylene can be linked to the same atom or different atoms of the alkylene group. For instance, a straight chain alkylene can be the bivalent radical of -(CH₂)ₙ-, where n is 1, 2, 3, 4, 5 or 6. Representative alkylene groups include, but are not limited to, methylene, ethylene, propylene, isopropylene, butylene, isobutylene, sec-butylene, pentylene and hexylene.

"Alkoxy" refers to an alkyl group having an oxygen atom that connects the alkyl group to the point of attachment: alkyl-O-. Alkoxy groups can have any suitable number of carbon atoms, such as C₁-C₆. Alkoxy groups include, for example, methoxy, ethoxy, propoxy, iso-propoxy, butoxy, 2-butoxy, iso-butoxy, sec-butoxy, tert-butoxy, pentoxy, hexoxy, etc.

"Aryl" refers to an aromatic ring system having any suitable number of ring atoms and any suitable number of rings. Aryl groups can include any suitable number of ring atoms, such as, 6, 7, 8, 9, 10, 11, 12, 13, 14, 15 or 16 ring atoms, as well as from 6 to 10, 6 to 12, or 6 to 14 ring members. Aryl groups can be monocyclic, fused to form bicyclic or tricyclic groups, or linked by a bond to form a biaryl group. Representative aryl groups include phenyl, naphthyl and biphenyl. Other aryl groups include benzyl, having a methylene linking group. Some aryl groups have from 6 to 12 ring members, such as phenyl, naphthyl or biphenyl. Other aryl groups have from 6 to 10 ring members, such as phenyl or naphthyl. Some other aryl groups have 6 ring members, such as phenyl.

"Aryloxy" refers to an aryl group having an oxygen atom that connects the aryl group to the point of attachment: aryl-O-. Aryloxy groups useful in the present disclosure include C₆₋₁₀ aryloxy, wherein the aryl group has from 6 to 10 ring members.

"Halogen" refers to fluorine, chlorine, bromine and iodine.

"Carboxylate" refers to a mono-carboxylate group having formula RC(O)O⁻ where the R group can be alkyl, aryl, or arylalkyl; or a di-carboxylate group R(C(O)O⁻)₂ where the R group can be alkylene, alkylene-O-alkylene, alkylene-NH-alkylene, or alkylene-N(alkyl)-alkylene. Carboxylates useful in the present disclosure include, but are not limited to, acetate, malonate, 2,2'-oxydiacetate, iminodiacetate, and 2,2'-(methylazanediyl)acetate.

"Sulfonate" refers to an -SO₃R group where R group can be halo (e.g., -F), alkyl (e.g., methyl or ethyl), haloalkyl (e.g., trifluoromethyl), aryl (e.g., phenyl, tosyl, p-fluorophenyl, or p-nitrophenyl), heteroaryl (e.g., imidazolyl). Exemplary sulfonate groups include, but are not limited to, fluorosulfonate, methanesulfonate (OMs), trifluoromethanesulfonate (OTf), p-toluenesulfonate (OTs), p-fluorobenzenesulfonate, p-nitrophenylsulfonate (nosylate), and imidazole-1-sulfonate (imidazylate).

"OMs" refers to methanesulfonate; "-OTs" refers to p-toluenesulfonate; and "-OTf" refers to trifluoromethanesulfonate.

"Bidentate" refers to an alkoxy group, an aryloxy group, or a carboxylate group having at least two oxygen atoms (-O-), two of which can form both bonds to the same third atom such as boron (B), provided that the two oxygen atoms are not a part of a carbonyl group (-C=O). Bidentate groups useful in the present disclosure include bidentate C₂₋₈ alkoxy groups, bidentate C₆₋₁₀ aryloxy groups, and bidentate carboxylate groups.

"Tridentate" refers to an alkoxy group having at least three carbon atoms and three oxygen atoms (-O-), wherein three of oxygen atoms can form bonds to the same fourth atom such as boron (B). Tridentate groups useful in the present disclosure include tridentate C₃₋₁₀ alkoxy groups.

"Catalyst" refers to a substance that increases the rate of a chemical reaction by reducing the activation energy, but which is left unchanged by the reaction.

"Metal" refers to elements of the periodic table that are metallic and that can be neutral, or positively charged as a result of having more or fewer electrons in the valence shell than is present for the neutral metallic element. Metals useful in the present disclosure include the alkali metals and transition metals. Alkali metals in the present disclosure include alkali metal cations. Alkali metal cations useful in the present disclosure include Li⁺, Na⁺, K⁺, and Cs⁺. Transition metals useful in the present disclosure include Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg and Ac.

"Transition-metal catalyst" refers to a compound that is composed of a transition metal as defined above that can be neutral or positively charged.

Bases useful in the present disclosure include organic bases and inorganic bases. Exemplary organic bases include amines, alkali carboxylates, and alkali alkoxides, as defined herein. Exemplary inorganic bases include alkali bicarbonates, alkali carbonates, alkali phosphates tribasic, and alkali hydroxides, as defined herein. Amines useful in the present disclosure as bases include tertiary amines and aromatic amine bases, as defined herein.

"Amine" refers to a compound having formula N(R)₃ where the R groups can be hydrogen, alkyl, aryl, or heteroalkyl, among others. The R groups can be the same or different. For example, the amines can be primary amine (two R is each hydrogen), secondary amine (one R is hydrogen), and tertiary amine (each R is other than hydrogen). In some embodiments, the secondary amine is a cyclic amine where two R groups bond to the nitrogen atom form a 5-6 membered heterocyclic ring. Non-limiting examples of cyclic amines include pyrrolidine, piperidine, and morpholine.

"Tertiary amine" refers to a compound having formula N(R)₃ wherein the R groups can be alkyl, aryl, heteroalkyl, heteroaryl, among others, or two R groups together form a*N*-linked heterocycloalkyl. The R groups can be the same or different. Non-limiting examples of tertiary amines include triethylamine, tri-*n*-butylamine, *N,N*-diisopropylethylamine, *N*-methylpyrrolidine, *N*-methylmorpholine, dimethylaniline, diethylaniline, 1,8-bis(dimethylamino)naphthalene, quinuclidine, and 1,4-diazabicylo[2.2.2]-octane (DABCO).

"Aromatic amine base" refers to a *N*-containing 5- to 10-membered heteroaryl compound or a tertiary amine having formula N(R)₃ wherein at least one R group is an aryl or heteroaryl. Aromatic amine bases useful in the present application include, but are not limited to, pyridine, lutidines (e.g., 2,6-lutidine, 3,5-lutidine, and 2,3-lutidine), collidines (e.g., 2,3,4-collidine, 2,3,5-collidine, 2,3,6-collidine, 2,4,5-collidine, 2,4,6-collidine, and 3,4,5-collidine), 4-dimethylaminopyridine, imidazole, 1-methylimidazole, dimethylaniline, and diethylaniline.

"Alkali carboxylate" refers to a class of chemical compounds which are composed of an alkali metal cation and the carboxylate anion (RC(O)O⁻) where the R group can be alkyl or aryl. Alkali carboxylates useful in the present include, but are not limited to, lithium acetate (LiOC(O)CH₃), sodium acetate (NaOC(O)CH₃), potassium acetate (KOC(O)CH₃), cesium acetate (CsOC(O)CH₃), and potassium trimethylacetate (KOC(O)C(CH₃)₃).

"Alkali alkoxide" refers to a class of chemical compounds which are composed of an alkali metal cation and the alkoxide anion (RO⁻), wherein R is C₁₋₄ alkyl. Alkali alkoxides useful in the present disclosure include, but are not limited to, sodium methoxide, sodium isopropoxide, sodium tert-butoxide, potassium tert-butoxide, and potassium isopropoxide.

"Alkali bicarbonate" refers to a class of chemical compounds which are composed of an alkali metal cation and the carbonate anion (HCO₃⁻). Alkali carbonates useful in the present disclosure include lithium bicarbonate (LiHCO₃), sodium bicarbonate (NaHCO₃), potassium bicarbonate (KHCO₃), and cesium bicarbonate (CsHCO₃).

"Alkali carbonate" refers to a class of chemical compounds which are composed of an alkali metal cation and the carbonate anion (CO₃²⁻). Alkali carbonates useful in the present disclosure include lithium carbonate (Li₂CO₃), sodium carbonate (Na₂CO₃), potassium carbonate (K₂CO₃), and cesium carbonate (Cs₂CO₃).

"Alkali phosphate tribasic" refers to a class of chemical compounds which are composed of an alkali metal cation and the phosphate anion (PO₄³⁻). Alkali phosphates tribasic useful in the present disclosure include sodium phosphate tribasic (Na₃PO₄) and potassium phosphate tribasic (K₃PO₄).

"Alkali hydroxide" refers a class of chemical compounds which are composed of an alkali metal cation and the hydroxide anion (OH⁻). Alkali hydroxides useful in the present disclosure include LiOH, NaOH, KOH, and CsOH.

"Acid" refers to a compound that is capable of donating a proton (H⁺) under the Bronsted-Lowry definition, or is an electron pair acceptor under the Lewis definition. Acids useful in the present disclosure include, but are not limited to, fluorinated carboxylic acids (trifluoroacetic acid), sulfonic acids and mineral acids, as defined herein. Mineral acids are inorganic acids such as hydrogen halides (hydrofluoric acid, hydrochloric acid, hydrobromic acid, etc.), as well as sulfuric acid, nitric acid, and phosphoric acid. Sulfonic acids include methanesulfonic acid, benzenesulfonic acid, p-toluenesulfonic acid, triflouromethanesulfonic acid, among others.

"A protecting group" refers to a compound that renders a functional group unreactive to a particular set of reaction conditions, but that is then removable in a later synthetic step so as to restore the functional group to its original state. Such protecting groups are well known to one of ordinary skill in the art and include compounds that are disclosed in "Protective Groups in Organic Synthesis", 4th edition, T. W. Greene and P. G. M. Wuts, John Wiley & Sons, New York, 2006.

"An amine protecting group" refers a protecting group that is used to protect one of nitrogen atoms in 1,2,3-triazole group. The amine protecting groups useful in the present disclosure include, but are not limited to, a benzyl (Bn) group, 2-(trimethylsilyl)ethoxymethyl, p-methoxybenzyl (PMB), 2,4-dimethoxybenzyl group (DMB), 1-(2,4-dimethoxyphenyl)ethyl, 3,4-dimethoxybenzyl (DMPB), p-methoxyphenyl (PMP), Tosyl (Ts) group, and other sulfonamides (Nosyl & Nps) groups.

"Deprotecting" refers to remove the protecting group as defined above with one or more chemicals or agents so that the functional group is restored to its original state.

"Transferring agent" refers to a chemical agent added to a reaction mixture during the deprotecting step in order to efficiently remove the amine protecting group, such as p-methoxybenzyl (PMB), 2,4-dimethoxybenzyl group (DMB), 1-(2,4-dimethoxyphenyl)ethyl, or 3,4-dimethoxybenzyl (DMPB). Transferring agents useful in the present disclosure include, but are not limited to, anisole or the like. It is believed that anisole is acting as a transfer reagent wherein the PMB protecting group or the like is transferred from an amine to the para position of anisole.

"Contacting" refers to the process of bringing into contact at least two distinct species such that they can react. It should be appreciated, however, the resulting reaction product can be produced directly from a reaction between the added reagents or from an intermediate from one or more of the added reagents which can be produced in the reaction mixture.

"Solvent" refers to a substance, such as a liquid, capable of dissolving a solute. Solvents can be polar or non-polar, protic or aprotic. Polar solvents typically have a dielectric constant greater than about 5 or a dipole moment above about 1.0, and non-polar solvents have a dielectric constant below about 5 or a dipole moment below about 1.0. Protic solvents are characterized by having a proton available for removal, such as by having a hydroxy or carboxy group. Aprotic solvents lack such a group. Representative polar protic solvents include alcohols (methanol, ethanol, propanol, isopropanol, etc.), acids (formic acid, acetic acid, etc.) and water. Representative polar aprotic solvents include dichloromethane, chloroform, tetrahydrofuran, methyltetrahydrofuran, diethyl ether, 1,4-dioxane, acetone, ethyl acetate, dimethylformamide, acetonitrile, dimethyl sulfoxide, and N-methylpyrrolidone. Representative non-polar solvents include alkanes (pentanes, hexanes, etc.), cycloalkanes (cyclopentane, cyclohexane, etc.), benzene, and toluene. Other solvents are useful in the present disclosure.

Solvents can also be grouped based on their chemical structures, for example, ethers (e.g., diethyl ether, methyl *tert*-butyl ether, tetrahydrofuran, 2-methyltetrahydrofuran, 1,4-dioxane, etc.), ketones (e.g., acetone, methyl isobutyl ketone, etc.), esters (ethyl acetate, butyl acetate, isobutyl acetate, etc.), aromatic solvents (e.g., benzene, toluene, xylenes, etc.), chlorinated solvents (e.g., dichloromethane, 1,2-dichloroethane, etc.), hydrocarbons (n-heptane, hexanes, cyclohexane, methylcyclohexane, etc.), alcohols (methanol, ethanol, propanol, isopropanol, etc.), or acids (e.g., formic acid, acetic acid, etc.).

"Solvate" refers to a compound provided herein or a salt thereof, that further includes a stoichiometric or non-stoichiometric amount of solvent bound by non-covalent intermolecular forces. Where the solvent is water, the solvate is a hydrate.

"Hydrate" refers to a compound that is complexed to a water molecule. The compounds of the present disclosure can be complexed with ½ water molecule or from 1 to 10 water molecules.

"Tautomer" refers to one of two or more structural isomers which exist in equilibrium and which are readily converted from one form to another.

"Salt" refers to acid or base salts of the compounds used in the methods of the present disclosure. Pharmaceutically acceptable salts includes salts of the compounds which are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amine, or magnesium salt, or a similar salt. It is understood that the pharmaceutically acceptable salts are non-toxic. Additional information on suitable pharmaceutically acceptable salts can be found in Remington's Pharmaceutical Sciences, 17th ed., Mack Publishing Company, Easton, Pa., 1985.

"About" means a range of values including the specified value, which a person of ordinary skill in the art would consider reasonably similar to the specified value. In some embodiments, the term "about" means within a standard deviation using measurements generally acceptable in the art. In some embodiments, "about" means a range extending to +/- 10% of the specified value. In some embodiments, "about" means the specified value.

"Composition" as used herein is intended to encompass a product comprising the specified ingredients in the specified amounts, as well as any product, which results, directly or indirectly, from combination of the specified ingredients in the specified amounts. By "pharmaceutically acceptable" it is meant the carrier, diluent or excipient must be compatible with the other ingredients of the formulation and not deleterious to the recipient thereof.

"Pharmaceutically acceptable excipient" refers to a substance that aids the administration of an active agent to and absorption by a subject. Pharmaceutical excipients useful in the present disclosure include, but are not limited to, binders, fillers, disintegrants, lubricants, coatings, sweeteners, flavors and colors. Other pharmaceutical excipients can be useful in the present disclosure.

"Inhibition", "inhibits", and "inhibitor" refer to a compound that prohibits or a method of prohibiting, a specific action or function. Inhibition may be partial or complete inhibition. Inhibition may be prophylactic or preventative.

"Administering" refers to oral administration, administration as a suppository, topical contact, parenteral administration, intravenous administration, intraperitoneal administration, intramuscular administration, intralesional administration, intranasal administration, subcutaneous administration, intrathecal administration, the implantation of a slow-release device e.g., a mini-osmotic pump, or administration via any other useful mechanism to the subject.

"Treat", "treating", and "treatment" refer to any indicia of success in the treatment or amelioration of an injury, pathology or condition, including any objective or subjective parameter such as abatement; remission; diminishing of symptoms or making the injury, pathology or condition more tolerable to the patient; slowing in the rate of degeneration or decline; making the final point of degeneration less debilitating; improving a patient's physical or mental well-being. The treatment or amelioration of symptoms can be based on objective or subjective parameters; including the results of a physical examination, neuropsychiatric exams, and/or a psychiatric evaluation.

"Patient" or "subject" refers to a living organism suffering from or prone to a disease or condition that can be treated by administration of a pharmaceutical composition as provided herein. Non-limiting examples include humans, other mammals, bovines, rats, mice, dogs, monkeys, goat, sheep, cows, deer, and other non-mammalian animals. In some embodiments, the patient is human. As described herein, the patient may have been diagnosed with a disorder or condition such as a kidney stone disease and/or primary hyperoxaluria. As described herein, the patient may be suspected of having a disorder or condition such as a kidney stone disease and/or primary hyperoxaluria. As described herein, the patient may have previously undergone treatment for a disorder or condition such as a kidney stone disease and/or primary hyperoxaluria. As described herein, the patient may be undergoing treatment or assessment for a disorder or condition such as a kidney stone disease and/or primary hyperoxaluria.

"Therapeutically effective amount" refers to an amount of a compound or of a pharmaceutical composition useful for treating or ameliorating an identified disease or condition, or for exhibiting a detectable therapeutic or inhibitory effect. The exact amounts will depend on the purpose of the treatment, and will be ascertainable by one skilled in the art using known techniques (*see, e.g.,* Lieberman, Pharmaceutical Dosage Forms (vols. 1-3, 1992); Lloyd, The Art, Science and Technology of Pharmaceutical Compounding (1999); Pickar, Dosage Calculations (1999); and Remington: The Science and Practice of Pharmacy, 20th Edition, 2003, Gennaro, Ed., Lippincott, Williams & Wilkins).

"Primary hyperoxaluria, type I" and "PH1" are interchangeable and refer to a condition caused by the deficiency of alanine:glyoxylate aminotransferase (AGT), a liver enzyme. This deficiency causes impaired glyoxylate metabolism in the liver and an ultimate increase in oxalate synthesis, contributing to the formation of calcium oxalate kidney stones.

"Kidney stone" and/or "bladder stone" refer to a small, solid particle that occur in the kidneys, renal pelvis, ureter, urinary bladder, and/or urethra. Commonly, kidney and/or bladder stones contain or consist of calcium salt particles including, but not limited to, calcium oxalate particles and calcium phosphate particles (*e.g.*, apatite particles or brushite particles). Kidney and/or bladder stones can also contain or consist of uric acid, struvite (*i.e.,* NH₄MgPO₄·6H₂O particles), and cystine (*i.e.,* particles containing oxidized cysteine disulfide dimer). Kidney and/or bladder stones typically range in size from less than a millimeter in their largest dimension to 5 or more centimeters in their largest dimension. Kidney stones often form in the kidney or renal pelvis and, when they are small enough (e.g., less than 5 mm), they can pass through the ureter, bladder, and urethra to be eliminated from the body via urination. Kidney and/or bladder stones often cause severe pain in the side and back, below the ribs, and severe pain in the lower abdomen and groin. Other symptoms of kidney and/or bladder stones include, but are not limited to, pain upon urination, abnormally colored urine (*e.g.,* pink, red, or brown), cloudy urine, foul-smelling urine, nausea and vomiting, a persistent need to urinate, low urine volume, fever, and chills. The presence of kidney and/or bladder stones in the urinary system can be confirmed using imaging techniques such as abdominal X-ray, CT scan, and ultrasound.

"Glycolate oxidase" and "GO" are used interchangeably to refer to the liver peroxisomal enzyme glycolate oxidase 1 (GO1), also known as hydroxyacid oxidase 1 (HAO1). The human enzyme is cataloged under NCBI Accession No. NP_060015.1 and UniProtKB Reference No. Q9UJM8. The mouse enzyme is cataloged under GenBank Accession No. EDL28373.1 and UniProtKB Reference No. Q9WU19. The enzyme catalyzes the conversion of glycolic acid to glyoxylic acid, an oxalic acid precursor.

### III. PROCESSES FOR PREPARING COMPOUNDS

In one aspect, the present disclosure provides a process for preparing a compound represented by formula (II): 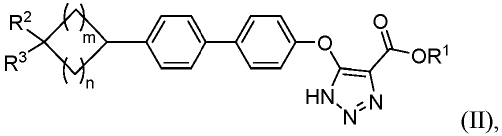 a tautomer thereof, or a salt thereof. The process includes:
a) contacting a compound of formula (IV): 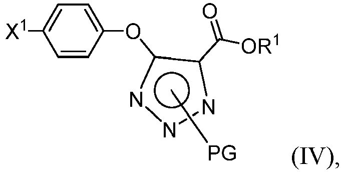 or a salt thereof, with a compound of formula (V): 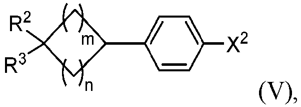 a first transition-metal catalyst, and a first base in a first solvent to form a compound of formula (III): 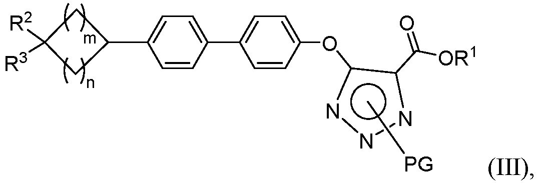 or a salt thereof; and
b) removing the PG group of the compound of formula (III) or the salt thereof to provide the compound of formula (II), the tautomer thereof, or the salt thereof,
wherein:
subscripts m and n are each independently 1 or 2;
R¹ is C₁₋₆ alkyl;
R² and R³ are each independently H or halogen;
X¹ represents a boron-containing group;
X² is halogen or a sulfonate; and 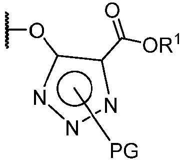 in formula (IV) or (III) is represented by the formula: 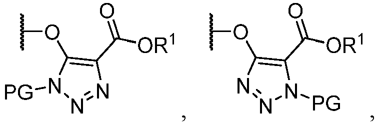
or a mixture thereof; and
PG is an amine-protecting group.

With reference to formula (IV) or (III), the 1,2,3-triazole moiety is protected with an amine-protecting group (PG). Suitable amine-protecting group include, but are not limited to, a benzyl (Bn) group, 2-(trimethylsilyl)ethoxymethyl (SEM), p-methoxybenzyl (PMB), 2,4-dimethoxybenzyl group (DMB), 3,4-dimethoxybenzyl (DMPM), 1-(2,4-dimethoxyphenyl)ethyl, p-methoxyphenyl (PMP), p-toluenesulfonyl (Ts), and p-nitrophenylsulfonyl (a Nosyl group). In some embodiments, the amine-protecting group is 2-(trimethylsilyl)ethoxymethyl (SEM), p-methoxybenzyl (PMB), 2,4-dimethoxybenzyl group (DMB), 3,4-dimethoxybenzyl (DMPM), or 1-(2,4-dimethoxyphenyl)ethyl. In some embodiments, the amine-protecting group is p-methoxybenzyl (PMB).

In some embodiments, when the amine-protecting group is benzyl (Bn), p-methoxybenzyl (PMB), 2,4-dimethoxybenzyl group (DMB), 3,4-dimethoxybenzyl (DMPM), or 1-(2,4-dimethoxyphenyl)ethyl, the compound of formula (IV) is represented by the formula (IV-1): 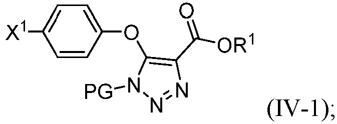 and
the compound of formula (III) is represented by the formula (III-1): 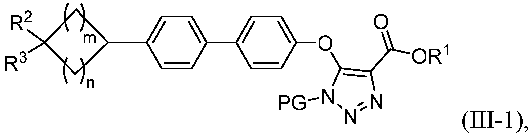
wherein subscripts m and n, R¹, R², R³, and X¹ are as defined and described herein.

In some embodiments, when the amine-protecting group is p-methoxybenzyl (PMB), the compound of formula (IV) is represented by the formula (IV-1a): 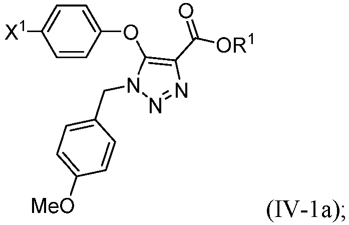 and
the compound of formula (III) is represented by the formula (III-1a): 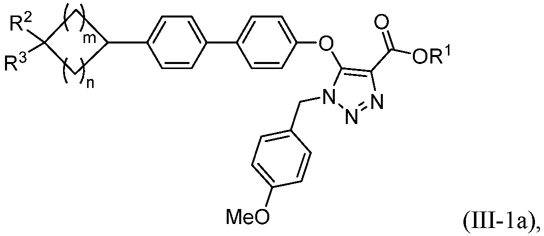
wherein subscripts m and n, R¹, R², R³, and X¹ are as defined and described herein.

In some embodiments, when the amine-protecting group is other than benzyl (Bn), p-methoxybenzyl (PMB), 2,4-dimethoxybenzyl group (DMB), 3,4-dimethoxybenzyl (DMPM), or 1-(2,4-dimethoxyphenyl)ethyl, the compound of formula (IV) is represented by the formula (IV-1) or (IV-2): 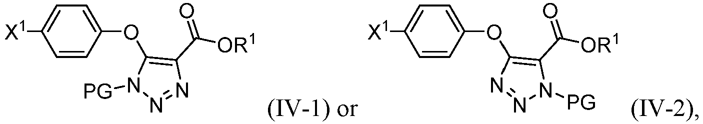 or a mixture thereof; and the compound of formula (III) is represented by the formula (III-1) or (III-2): 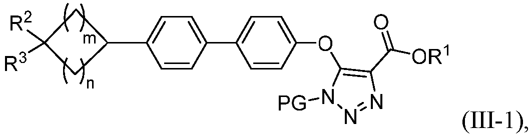 or 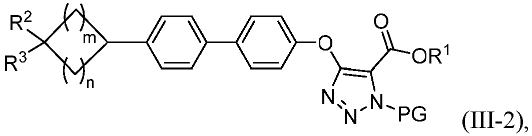 or a mixture thereof, wherein subscripts m and n, R¹, R², R³, and X¹ are as defined and described herein.

In some embodiments, when the amine-protecting group is 2-(trimethylsilyl)ethoxymethyl (SEM), the compound of formula (IV) is represented by the formula (IV-1b) or (IV-2b): 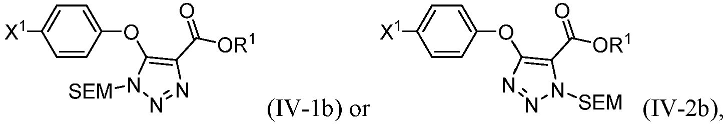 or a mixture thereof; and the compound of formula (III) is represented by the formula (III-1b) or (III-2b): 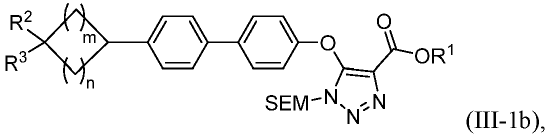 or 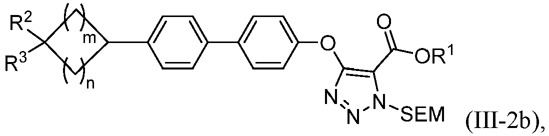 or a mixture thereof, wherein subscripts m and n, R¹, R², R³, and X¹ are as defined and described herein.

With reference to any one of formulae (IV), (IV-1), (IV-2), (IV-1a), (IV-1b) and (IVb-2b), in some embodiments, X¹ is represented by the formula:
1) -BY₂, wherein Y is -OH, C₁₋₆ alkyl, C₁₋₆ alkoxy, C₆₋₁₀ aryloxy, or a carboxylate group;
2) -BY, wherein Y is a bidentate C₂₋₈ alkoxy group, a bidentate C₆₋₁₀ aryloxy group, or
   a bidentate carboxylate group;
3) a 9-borabicyclo[3,3,1]nonane (9-BBN) group;
4) -BY₃M, wherein Y is F or C₁₋₆ alkoxy and M is an alkaline metal ion, an ammonium ion, or a phosphonium ion; or
5) -BYM, wherein Y is a tridentate C₃₋₁₀ alkoxy group and M is an alkaline metal ion, an ammonium ion, or a phosphonium ion.

In some embodiments, X¹ is -BY₂, wherein Y is -OH, C₁₋₆ alkyl, C₁₋₆ alkoxy, C₆₋₁₀ aryloxy, or a carboxylate group. In some embodiments, X¹ is -B(OH)₂, -B(OEt)₂, or - B(O*i*Pr)₂.

In some embodiments, X¹ is -BY, wherein Y is a bidentate C₂₋₈ alkoxy group, a bidentate C₆₋₁₀ aryloxy group, or a bidentate carboxylate group. In some embodiments, X¹ is selected from the group consisting of:

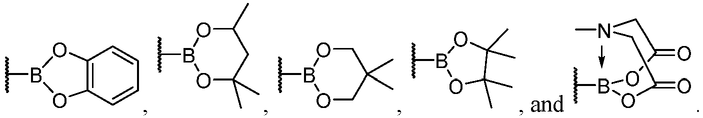

In some embodiments, X¹ is a 9-borabicyclo[3,3,1]nonane (9-BBN) group represented by the formula:

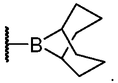

In some embodiments, X¹ is -BY₃M, wherein Y is F or C₁₋₆ alkoxy; and M is an alkaline metal ion, an ammonium ion, or a phosphonium ion. In some embodiments, X¹ is -BF₃M, -B(O*i*Pr)₃M, or -B(O*i*Pr)₃M, wherein M is Li⁺, Na⁺, or K⁺. In some embodiments, X¹ is -BF₃K, -B(O*i*Pr)₃K, or -B(O*i*Pr)₃Li.

In some embodiments, X¹ is -BYM, wherein Y is a tridentate C₃₋₁₀ alkoxy group; and M is an alkaline metal ion, an ammonium ion, or a phosphonium ion. In some embodiments, X¹ is represented by the formula: 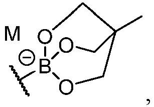 wherein M is Li⁺, Na⁺, or K⁺. In some embodiments, M is K⁺.

In some embodiments, X¹ is represented by the formula:

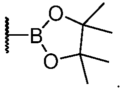

In some embodiments, the compound of formula (IV) is represented by formula (IV-1a-1): 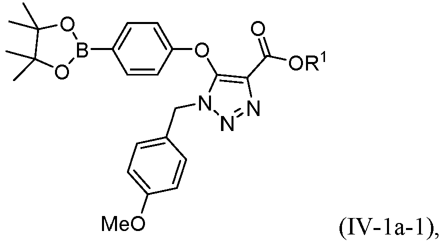 wherein R¹ is as defined and described herein.

With respect to formula (V), in some embodiments, X² is halogen. In some embodiments, X² is Cl, Br, or I. In some embodiments, X² is Br. In some embodiments, X² is a sulfonate. In some embodiments, X² is fluorosulfonate, methanesulfonate (OMs), p-toluenesulfonate (OTs), trifluoromethanesulfonate (OTf), p-fluorobenzenesulfonate, p-nitrophenylsulfonate (nosylate), or imidazole-1-sulfonate (imidazylate). In some embodiments, X² is methanesulfonate (OMs), p-toluenesulfonate (OTs), or trifluoromethanesulfonate (OTf).

In some embodiments, the compound of formula (V) is represented by formula (V-1): 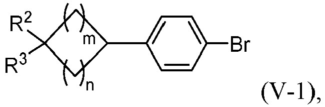 wherein subscripts m and n, R², and R³ are as defined and described herein.

With respect to step a), the first transition-metal catalyst can be a compound that includes one or more transition metals or transition metal cations. Suitable transition metals include, but are not limited to, Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg and Ac. Suitable transition metal cations include, but are not limited to, Cd²⁺, Co²⁺, Co⁺, Cr²⁺, Cr⁺, Cu⁺ (i.e., Cu(I)), Cu²⁺ (i.e., Cu(II)), Fe²⁺, Fe⁺, Mn²⁺, Mn⁺, Ni²⁺, Ni⁺, Pd²⁺ (i.e., Pd(II)), and Zn²⁺. In some embodiments, the first transition-metal catalyst is a first palladium catalyst, a ruthenium catalyst, a rhodium catalyst, a cobalt catalyst, a nickel catalyst, an iron catalyst, a copper catalyst, or a combination thereof. In some embodiments, the first transition-metal catalyst is a first palladium catalyst.

In some embodiments, the first palladium catalyst is Pd(acac)₂, [Pd(allyl)Cl]₂, Pd(CH₃CN)₂Cl₂, Pd(dba)₂, Pd(CH₃COO)₂, Pd₂(dba)₃, Pd₂(dba)₃·CHCl₃, Pd(PPh₃)₄, Pd(OAc)₂, Pd(PCy₃)₂Cl₂, Pd(PPh₃)₂Cl₂, Pd[P(o-tol)₃]₂Cl₂, Pd(amphos)Cl₂, Pd(dppf)Cl₂, Pd(dppf)Cl₂·CH₂Cl₂, Pd(dtbpf)Cl₂, Pd(CH₃CN)₄(BF₄)₂, PdCl₂, XPhos-Pd-G3, Pd-PEPPSI^{™}-IPr, Pd-PEPPSI^{™}-SIPr, or Pd-PEPPSI^{™}-IPent. In some embodiments, the first palladium catalyst is [Pd(OAc)₂]₃, Na₂PdCl₄, Pd(CH₃CN)₄(CF₃SO₃)₂, bis(benzonitrile)palladium(II) dichloride, palladium(II)(π-cinnamyl) chloride dimer, [di-tert-butyl(chloro)-phosphine]-palladium(II) dichloride dimer, dihydrogen di-µ-chlorotetrakis(di-tert-butylphosphinito)-dipalladate, di-µ-chlorobis(2'-amino-1,1'-biphenyl-2-yl-C,N)-dipalladium(II), chloro[(tri-tertbutylphosphine)-2-(2-aminobiphenyl)]-palladium(II), Najera catalyst I, Najera catalyst II, di-µ-chlorobis[2-[(dimethylamino)-methyl]-phenyl-C,N]dipalladium(II), di-µ-chlorobis[2-[(dimethylamino)-methyl]-4,6-dimethoxyphenyl-C,N]-dipalladium(II), 1,2-bis(diphenylphosphino)-ethane]palladium(II) dichloride, [1,3-bis(diphenylphosphino)-propane]palladium(II) dichloride, [1,4-bis(diphenylphosphino)-butane]palladium(II) dichloride, bis(methyldiphenylphosphine) palladium(II) dichloride, benzylbis(triphenylphosphine)palladium(II) chloride, bis(triphenylphosphine)-palladium(II) diacetate, dichloro(1,5-cyclooctadiene)palladium(II), dichloro[9,9-dimethyl-4,5-bis(diphenylphosphino)xanthene]-palladium(II), SingaCycle^{™}-A1, SingaCycle^{™}-A2, SingaCycle^{™}-A3, SingaCycle^{™}-A4, bis(tri-tert-butylphosphine)palladium(0), bis[di-tert-butyl(4-dimethylaminophenyl)phosphine]-palladium(0), bis[1,2-bis(diphenylphosphino)ethane]-palladium(0), poly(methylphenyl)silane supported palladium/alumina hybrid catalyst, polydimethylsilane supported palladium/alumina hybrid catalyst, or poly[N-isopropylacrylamide-co-4-(diphenylphosphino)-styrene] palladium(II) dichloride (ratio, acrylamide:phosphine=20:2). In some embodiments, the first palladium catalyst is Pd(dppf)Cl₂, Pd(dppf)Cl₂·CH₂Cl₂, or Pd(dtbpf)Cl₂. In some embodiments, the first palladium catalyst is Pd(dppf)Cl₂·CH₂Cl₂.

In some embodiments, the first transition-metal catalyst is in a substoichiometric amount. In some embodiments, the first palladium catalyst is in a substoichiometric amount. In some embodiments, Pd(dppf)Cl₂·CH₂Cl₂ is in a substoichiometric amount. In some embodiments, the first transition-metal catalyst is in an amount of from 0.01 to 0.5 equivalent, from 0.02 to 0.2 equivalent, or from 0.05 to 0.1 equivalent, relative to the compound of formula (IV) or any one of its related formulae. In some embodiments, the first palladium catalyst is in an amount of from 0.01 to 0.5 equivalent, from 0.02 to 0.2 equivalent, or from 0.05 to 0.1 equivalent, relative to the compound of formula (IV) or any one of its related formulae. In some embodiments, the first palladium catalyst is in an amount of from 0.05 to 0.1 equivalent relative to the compound of formula (IV) or any one of its related formulae. In some embodiments, Pd(dppf)Cl₂·CH₂Cl₂ is in an amount of from 0.05 to 0.1 equivalent relative to the compound of formula (IV) or any one of its related formulae.

With respect to step a), the first base can be an alkali carbonate, an alkali bicarbonate, an alkali phosphate tribasic, an alkali hydroxide, an alkali carboxylate, an amine, an alkali alkoxide, or a combination thereof. Suitable alkali carbonates include lithium carbonate, sodium carbonate, potassium carbonate, and cesium carbonate. Suitable alkali bicarbonates include lithium bicarbonate, sodium bicarbonate, potassium bicarbonate, and cesium bicarbonate. Suitable alkali phosphates tribasic include sodium phosphate tribasic and potassium phosphate tribasic. Suitable alkali hydroxides include lithium hydroxide, sodium hydroxide, potassium hydroxide, and cesium hydroxide. Suitable alkali carboxylates include lithium acetate, sodium acetate, potassium acetate, cesium acetate, and potassium trimethylacetate. Suitable amines include primary amines, secondary amines, tertiary amines, and aromatic amine bases. Suitable alkali alkoxides include sodium methoxide or sodium ethoxide. In some embodiments, the first base is sodium carbonate, potassium carbonate, cesium carbonate, sodium bicarbonate, potassium bicarbonate, cesium bicarbonate, sodium phosphate tribasic, potassium phosphate tribasic, cesium phosphate tribasic, sodium hydroxide, potassium hydroxide, cesium hydroxide, sodium acetate, potassium acetate, cesium acetate, 1,8-bis(dimethylamino)-naphthalene, *tert-*butylamine, diisopropylamine, N,N-diisopropylethylamine, 1-methylimidazole, sodium methoxide, or a combination thereof. In some embodiments, the first base is sodium carbonate, potassium carbonate, cesium carbonate, sodium phosphate tribasic, potassium phosphate tribasic, sodium acetate, potassium acetate, cesium acetate, or a combination thereof. In some embodiments, the first base is potassium carbonate, potassium phosphate tribasic, or potassium acetate. In some embodiments, the first base includes potassium carbonate. In some embodiments, the first base is potassium carbonate.

In some embodiments, the first base is present in an amount of from 10 to 1 equivalents, from 1 to 5 equivalents, from 2 to 5 equivalents, from 2 to 4 equivalents, from 3 to 4 equivalents, or about 3.5 equivalents, relative to the compound of formula (IV) or any one of its related formulae. In some embodiments, the first base is present in an amount of from 2 to 5 equivalents, from 2 to 4 equivalents, from 3 to 4 equivalents, or about 3.5 equivalents, relative to the compound of formula (IV) or any one of its related formulae. In some embodiments, the first base is present in an amount of from 3 to 4 equivalents or about 3.5 equivalents, relative to the compound of formula (IV) or any one of its related formulae. In some embodiments, potassium carbonate is present in an amount of from 3 to 4 equivalents or about 3.5 equivalents relative to the compound of formula (IV) or any one of its related formulae.

With respect to step a), in some embodiments, the compound of formula (V) is in an amount of from 1.0 to 2.0 equivalents, from 1.1 to 2.0 equivalents, from 1.1 to 1.5 equivalents, or about 1.2 equivalents, relative to the compound of formula (IV) or any one of its related formulae. In some embodiments, the compound of formula (V) is in an amount of about 1.2 equivalents relative to the compound of formula (IV) or any one of its related formulae. In some embodiments, the compound of formula (V-1) is in an amount of about 1.2 equivalents relative to the compound of formula (IV-1a-1).

The first solvent can be any suitable polar or non-polar, protic or aprotic solvent. In some embodiments, the first solvent is water, C₁₋₄ alcohol, benzene, toluene, dioxane, tetrahydrofuran (THF), 2-methyl-tetrahydrofuran (MeTHF), acetonitrile (ACN), N-methylpyrrolidone (NMP), N,N-dimethylformamide (DMF), N,N-dimethylacetamide (DMAC), dimethoxyethane (DME), ethylene glycol, or a combination thereof. In some embodiments, the first solvent includes dioxane and water. In some embodiments, the first solvent includes 1,4-dioxane and water. In some embodiments, the first solvent has a 1,4-dioxane : water ratio of from 20:1 to 1:1, from 20:1 to 5:1, from 15:1 to 5:1, or about 10:1 by volume. In some embodiments, the first solvent has a 1,4-dioxane : water ratio of about 10:1 by volume.

In general, the Suzuki reaction of step (a) can be performed at an ambient to an elevated temperature. For example, the reaction mixture of step a) can be at a temperature of from 30°C to 110°C or heated to reflux. In some embodiments, step a) is conducted at a temperature of from 60°C to 110°C, from 70°C to 110°C, from 70°C to 100°C, from 70°C to 90°C, or about 80°C. In some embodiments, step a) is conducted at a temperature of from 70°C to 90°C. In some embodiments, step a) is conducted at a temperature of about 80°C.

With respect to step b), the compound of formula (III) can be deprotected by various methods to provide the compound of formula (II). When PG is p-methoxybenzyl (PMB), the compound of any one of formula (III), (III-1), and (III-1a) can be deprotected by various methods, for example, under acidic, reductive (hydrogenolysis), or oxidative conditions to provide the compound of the compound of formula (II).

In some embodiments, the PG group is removed by treating with a first acid in a second solvent. In some embodiments, the p-methoxybenzyl (PMB) group is removed by treating with a first acid in a second solvent. In some embodiments, the first acid is trifluoroacetic acid, trichloroacetic acid, hydrochloric acid, sulfuric acid, phosphoric acid, or a combination thereof. In some embodiments, the first acid includes trifluoroacetic acid. In some embodiments, the first acid is trifluoroacetic acid.

The second solvent can be any suitable polar or non-polar, protic or aprotic solvent. In some embodiments, the second solvent is water, C₁₋₄ alcohol, ethyl acetate, isopropyl acetate, butyl acetate, isobutyl acetate, tetrahydrofuran, 2-methyltetrahydrofuran, dioxane, benzene, toluene, xylenes, chlorobenzene, dichloromethane, 1,2-dichloroethane, or a combination thereof. In some embodiments, the second solvent is dichloromethane or 1,2-dichloroethane. In some embodiments, the second solvent includes dichloromethane. In some embodiments, the second solvent is dichloromethane.

When the PG group is removed under the acidic conditions, in some embodiments, the deprotection reaction mixture further includes a transferring agent. When PG is p-methoxybenzyl (PMB), in some embodiments, the transferring agent is anisole.

In some embodiments, the p-methoxybenzyl (PMB) group is removed by treating with trifluoroacetic acid and anisole in dichloromethane.

In general, step b) under the acidic conditions can be performed at an ambient to an elevated temperature. For example, the reaction mixture of step b) can be at a temperature of from 30°C to 60°C or heated to reflux. In some embodiments, step b) is conducted at a temperature of about 50°C.

In some embodiments, the p-methoxybenzyl (PMB) group is removed by hydrogenolysis. In some embodiments, the p-methoxybenzyl (PMB) group is removed under oxidative conditions by treating 2,3-dichloro-5,6-dicyano-1,4-benzoquinone (DDQ).

In some embodiments, the process further includes:
c) contacting the compound of formula (II), the tautomer thereof, or the salt thereof with a second base in a third solvent; and
d) acidifying with a second acid to provide a compound of formula (I): 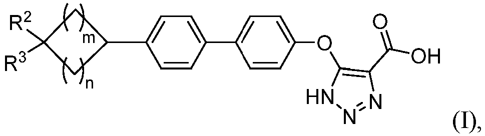 a tautomer thereof, or a salt thereof.

With respect to step c), the second base can be an alkali carbonate, an alkali hydroxide, an alkali alkoxide, or a combination thereof. Suitable alkali carbonates include lithium carbonate, sodium carbonate, potassium carbonate, and cesium carbonate. Suitable alkali hydroxides include lithium hydroxide, sodium hydroxide, potassium hydroxide, and cesium hydroxide. Suitable alkali alkoxides include sodium methoxide, sodium *tert*-butoxide, and potassium *tert*-butoxide. In some embodiments, the second base is lithium carbonate, sodium carbonate, potassium carbonate, cesium carbonate, lithium hydroxide, sodium hydroxide, potassium hydroxide, cesium hydroxide, sodium methoxide, sodium *tert*-butoxide, potassium *tert*-butoxide, or a combination thereof. In some embodiments, the second base is lithium hydroxide, sodium hydroxide, or potassium hydroxide. In some embodiments, the second base is sodium hydroxide. In some embodiments, the second base is an aqueous solution of sodium hydroxide.

The third solvent can be any suitable polar or non-polar, protic, or aprotic solvent. In some embodiments, the first solvent is water, C₁₋₄ alcohol, dioxane, tetrahydrofuran (THF), 2-methyl-tetrahydrofuran (MeTHF), acetonitrile (ACN), N-methylpyrrolidone (NMP), N,N-dimethylformamide (DMF), dimethoxyethane (DME), or a combination thereof. In some embodiments, the third solvent includes tetrahydrofuran (THF) and water.

In general, step c) can be performed at an ambient to an elevated temperature. For example, the reaction mixture of step c) can be at a temperature of from 30°C to 100°C or heated to reflux. In some embodiments, step c) is conducted at a temperature of from 30°C to 80°C, from 40°C to 70°C, from 40°C to 60°C, or from 50°C to 60°C. In some embodiments, step c) is conducted at a temperature of from 50°C to 60°C. In some embodiments, step c) is conducted at a temperature of about 55°C.

With respect to step d), in some embodiments, the second acid is hydrochloric acid, sulfuric acid, phosphoric acid, or a combination thereof. In some embodiments, the second acid is hydrochloric acid. In some embodiments, the second acid is an aqueous solution of hydrochloric acid.

In some embodiments, the step d) is conducted in an aqueous solution. In some embodiments, the step d) is conducted by treating an aqueous extract of step c) with an aqueous solution of hydrochloric acid. In some embodiments, the step d) is conducted by treating an aqueous extract of step c) with an aqueous solution of hydrochloric acid to have a mixture with a pH value of from 2 to 3.

In general, step d) can be performed at an ambient to an elevated temperature. For example, the reaction mixture of step d) can be at a temperature of from 20°C to 50°C. In some embodiments, step d) is conducted at a temperature of from 20°C to 30°C, or at room temperature. In some embodiments, step d) is conducted at room temperature.

With reference to any one of formulae (I), (II), (III), (V), and their related formulae, in some embodiments, subscripts m and n are each 1. In some embodiments, one of subscripts m and n is 2 and the other is 1. In some embodiments, subscripts m and n are each 2.

In some embodiments, the compound of formula (I) is represented by any one of formulae (Ia), (Ib), and (Ic): 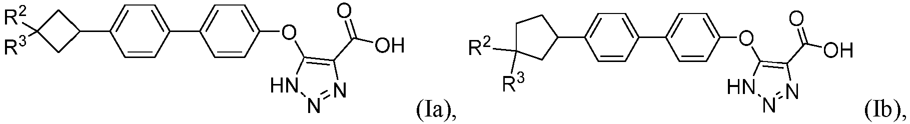 and 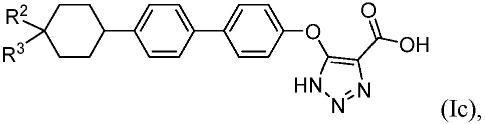 wherein R² and R³ are as defined and described herein.

In some embodiments, the compound of formula (II) is represented by any one of formulae (IIa), (IIb), and (IIc): 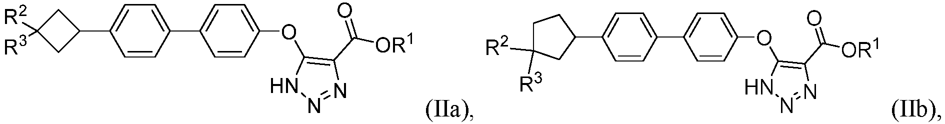 and 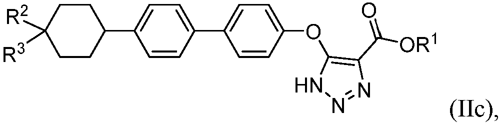 wherein R¹, R², and R³ are as defined and described herein.

In some embodiments, the compound of formula (III) is represented by any one of formulae (IIIa-1a), (IIIb-1a), and (IIIc-1a): 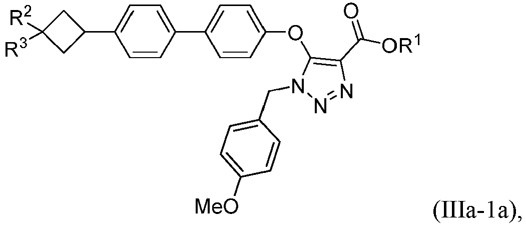 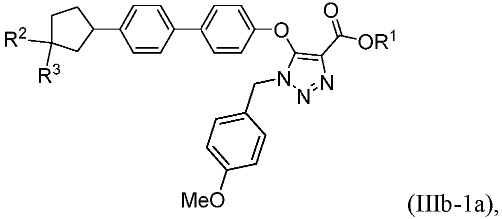 and 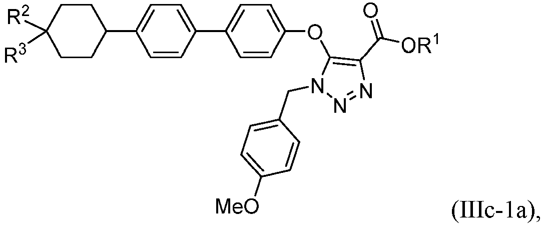 wherein R¹, R², and R³ are as defined and described herein.

In some embodiments, the compound of formula (V) is represented by any one of formulae (Va-1), (Vb-1), and (Vc-1): 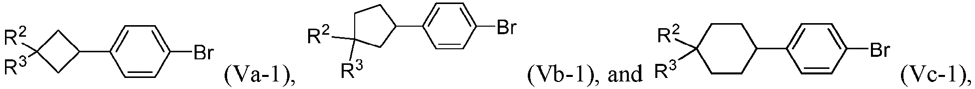 wherein R² and R³ are as defined and described herein.

With reference to any one of formulae (II), (III), (IV), and their related formulae, in some embodiments, R¹ is methyl, ethyl, n-propyl, isopropyl, n-butyl, isobutyl, sec-butyl, tert-butyl, n-pentyl, isopentyl, or n-hexyl. In some embodiments, R¹ is methyl.

With reference to any one of formulae (I), (II), (III), (V), and their related formulae, in some embodiments, R² and R³ are each independently halogen. In some embodiments, R² and R³ are each F.

In some embodiments, the compound of formula (I) is represented by any one of formulae (Ia-1), (Ib-1), and (Ic-1): 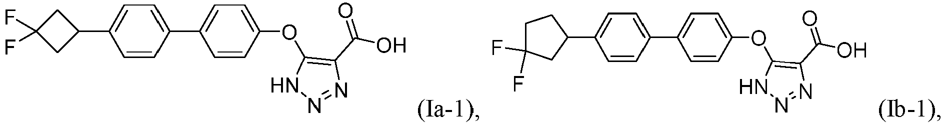 and

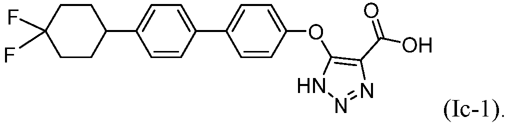

In some embodiments, the compound of formula (II) is represented by any one of formulae (IIa-1), (IIb-1), and (IIc-1): 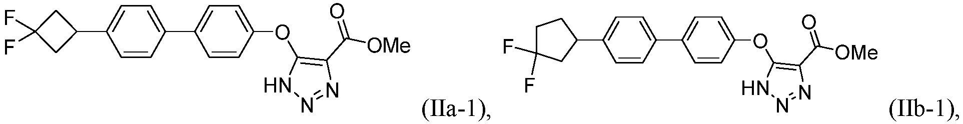 and

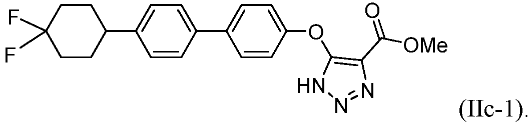

In some embodiments, the compound of formula (III) is represented by any one of formulae (IIIa-1a-1), (IIIb-1a-1), and (IIIc-1a-1): 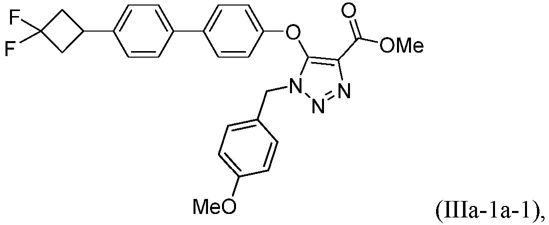 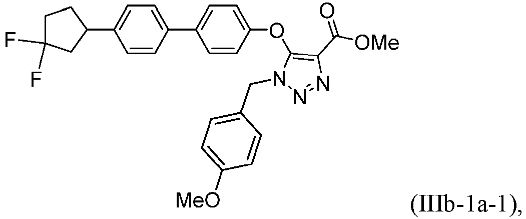 and

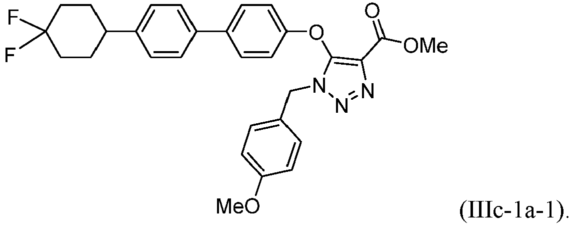

In some embodiments, the compound of any one of formula (IV), (IV-1), (IV-1a), and (IV-1a-1) is represented by formula (IVa-1a-2):

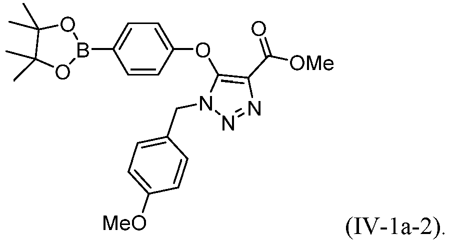

In some embodiments, the compound of formula (V) is represented by any one of formulae (Va-1-1), (Vb-1-1), and (Vc-1-1): 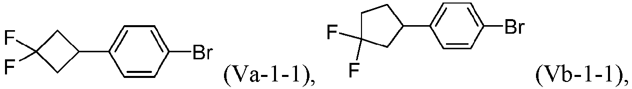 and

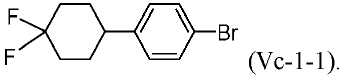

In some embodiments, the process further includes prior to step a):
a1) contacting a compound of formula (VI): 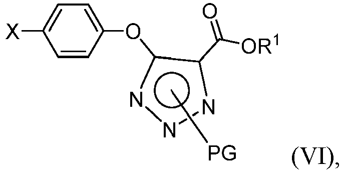 or a salt thereof, with a boron reagent, a second palladium catalyst, and a third base in a fourth solvent to form the compound of formula (IV) or the salt thereof, wherein X is Cl, Br, or I.

With respect to step a1), the boron reagent can be any agent capable of converting the compound of formula (VI) to the corresponding compound of formula (IV): 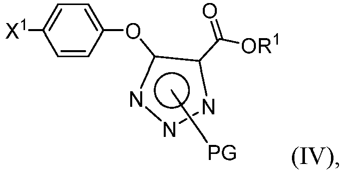 wherein X¹ represents a boron-containing group, which is defined and described herein. In some embodiments, the boron reagent is tetrahydroxydiboron, bis(catecholato)diboron, bis(hexylene glycolato)diboron, bis(neopentyl glycolato)diboron, or bis(pinacolato)diboron. In some embodiments, the boron reagent is bis(pinacolato)diboron.

The second palladium catalyst can be the same as the first palladium catalyst as described above. In some embodiments, second palladium catalyst is the same as the first palladium catalyst. In some embodiments, the second palladium catalyst is Pd(dppf)Cl₂, Pd(dppf)Cl₂·CH₂Cl₂, or Pd(dtbpf)Cl₂. In some embodiments, the second palladium catalyst is Pd(dppf)Cl₂·CH₂Cl₂.

In some embodiments, the second palladium catalyst is in a substoichiometric amount. In some embodiments, Pd(dppf)Cl₂·CH₂Cl₂ is in a substoichiometric amount. In some embodiments, the second palladium catalyst is in an amount of from 0.01 to 0.5 equivalent, from 0.02 to 0.2 equivalent, from 0.02 to 0.1 equivalent, or from 0.03 to 0.05 equivalent, relative to the compound of formula (VI). In some embodiments, the second palladium catalyst is in an amount of from 0.03 to 0.05 equivalent relative to the compound of formula (VI). In some embodiments, Pd(dppf)Cl₂·CH₂Cl₂ is in an amount of from 0.03 to 0.05 equivalent relative to the compound of formula (VI).

With respect to step a1), the third base can be an alkali carbonate, an alkali bicarbonate, an alkali phosphate tribasic, an alkali hydroxide, an alkali carboxylate, an amine, an alkali alkoxide, or a combination thereof, each of which is defined and described above. In some embodiments, the third base is sodium carbonate, potassium carbonate, cesium carbonate, sodium bicarbonate, potassium bicarbonate, cesium bicarbonate, sodium phosphate tribasic, potassium phosphate tribasic, cesium phosphate tribasic, sodium hydroxide, potassium hydroxide, cesium hydroxide, sodium acetate, potassium acetate, cesium acetate, 1,8-bis(dimethylamino)-naphthalene, *tert*-butylamine, diisopropylamine, N,N-diisopropylethylamine, 1-methylimidazole, sodium methoxide, or a combination thereof. In some embodiments, the third base is sodium carbonate, potassium carbonate, cesium carbonate, sodium phosphate tribasic, potassium phosphate tribasic, sodium acetate, potassium acetate, cesium acetate, or a combination thereof. In some embodiments, the third base is potassium carbonate, potassium phosphate tribasic, or potassium acetate. In some embodiments, the third base includes potassium acetate. In some embodiments, the third base is potassium acetate.

In some embodiments, the third base is present in an amount of from 1 to 10 equivalents, from 1 to 5 equivalents, from 2 to 5 equivalents, from 2 to 4 equivalents, or about 3.0 equivalents, relative to the compound of formula (VI). In some embodiments, the third base is present in an amount of from 2 to 5 equivalents, from 2 to 4 equivalents, or about 3.0 equivalents, relative to the compound of formula (VI). In some embodiments, the third base is present in an amount of about 3.0 equivalents, relative to the compound of formula (VI). In some embodiments, potassium acetate is present in an amount of about 3.0 equivalents relative to the compound of formula (VI).

The fourth solvent can be any suitable polar or non-polar, protic or aprotic solvent. In some embodiments, the fourth solvent is water, C₁₋₄ alcohol, benzene, toluene, dioxane, tetrahydrofuran (THF), 2-methyl-tetrahydrofuran (MeTHF), acetonitrile (ACN), N-methylpyrrolidone (NMP), N,N-dimethylformamide (DMF), N,N-dimethylacetamide (DMAC), dimethoxyethane (DME), ethylene glycol, or a combination thereof. In some embodiments, the fourth solvent includes dioxane. In some embodiments, the fourth solvent includes 1,4-dioxane. In some embodiments, the fourth solvent is 1,4-dioxane.

In some embodiments, when the amine-protecting group (i.e., PG) is p-methoxybenzyl (PMB), the compound of formula (VI) is represented by the formula (VI-1a): 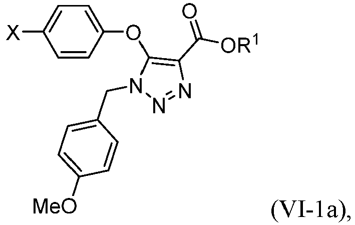 wherein R¹ and X are as defined and described herein.

In some embodiments of formula (VI) or (VI-1a), X is Br.

In some embodiments of formula (VI) or (VI-1a), R¹ is methyl, ethyl, n-propyl, isopropyl, n-butyl, isobutyl, sec-butyl, tert-butyl, n-pentyl, isopentyl, or n-hexyl. In some embodiments, R¹ is methyl.

In some embodiments, the compound of formula (VI) or (VI-1a) is represented by the formula (VI-1a-1):

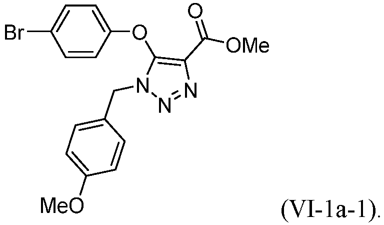

In some embodiments, the compound of formula (VI) or (VI-1a) is represented by the formula (VI-1a-1): 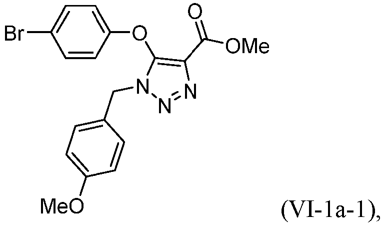 and
the compound of formula (IV) is represented by formula (IV-1a-2):

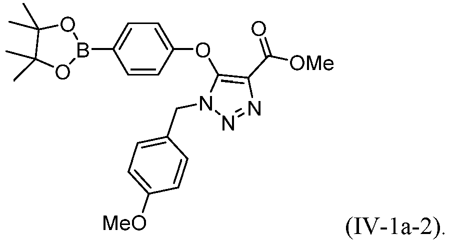

In general, step a1) can be performed at an ambient to an elevated temperature. For example, the reaction mixture of step a1) can be at a temperature of from 30°C to 110°C or heated to reflux. In some embodiments, step a1) is conducted at a temperature of from 60°C to 110°C, from 70°C to 110°C, from 70°C to 100°C, from 70°C to 90°C, or about 80°C. In some embodiments, step a1) is conducted at a temperature of from 70°C to 90°C. In some embodiments, step a1) is conducted at a temperature of about 80°C.

In some embodiments, the compound of formula (IV) produced by step a1) is used directly in the next step (i.e., step a)) without further purification.

In some embodiments, the present disclosure provides a process for preparing a compound represented by the formula (I): 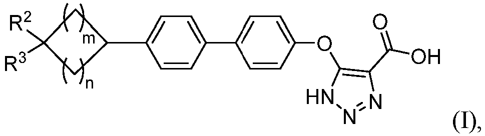 a tautomer thereof, or a salt thereof. The process includes:
a1) converting a compound of formula (VI-1a): 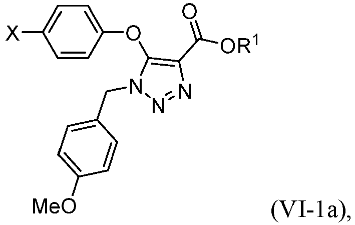 or a salt thereof, with bis(pinacolato)diboron, a second palladium catalyst, and a third base in a fourth solvent to a compound of formula (IV-1a-1): 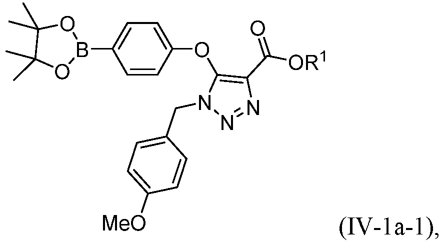 or a salt thereof;
a) contacting the compound of formula (IV-1a-1) or the salt thereof with a compound of formula (V-1): 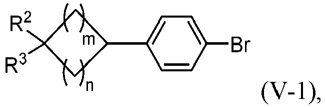 a first palladium catalyst, and a first base in a first solvent to form a compound of formula (III-1a): 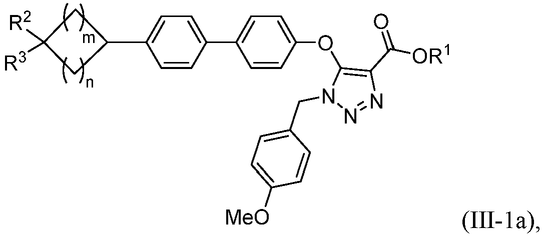 or a salt thereof;
b) treating the compound of formula (III-1a) or the salt thereof, with trifluoroacetic acid and anisole in dichloromethane to provide a compound of formula (II): 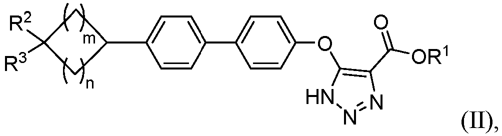 a tautomer thereof, or a salt thereof;
c) saponifying the compound of formula (II), the tautomer thereof, or the salt thereof with a second base in a third solvent; and
d) acidifying with a second acid to provide the compound of formula (I), the tautomer thereof, or the salt thereof,
wherein X is Cl, Br, or I;
subscripts m and n are each independently 1 or 2;
R¹ is C₁₋₃ alkyl; and
R² and R³ are each F.

In some embodiments, the process further includes:
e) converting the compound of formula (I), the tautomer thereof, or the salt thereof, to a mono-sodium salt of the compound of formula (I) represented by the formula: 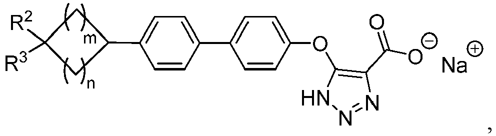 a di-sodium salt of the compound of formula (I) represented by the formula: 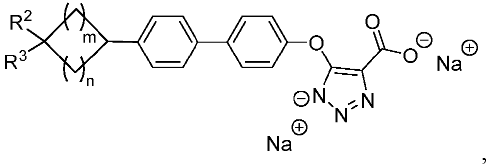 or a mixture thereof, or a tautomer thereof, wherein subscripts m and n are each independently 1 or 2; and R² and R³ are each F.

In some embodiments of formula (VI-1a), X is Br.

In some embodiments, the second palladium catalyst of step a1) and the first palladium of step a) are each independently Pd(dppf)Cl₂, Pd(dppf)Cl₂·CH₂Cl₂, or Pd(dtbpf)Cl₂. In some embodiments, the second palladium catalyst of step a1) and the first palladium of step a) are each Pd(dppf)Cl₂·CH₂Cl₂.

In some embodiments, the third base of step a1) and the first base of step a) are each independently sodium carbonate, potassium carbonate, cesium carbonate, sodium phosphate tribasic, potassium phosphate tribasic, sodium acetate, potassium acetate, or cesium acetate. In some embodiments, the third base of step a1) and the first base of step a) are each independently potassium carbonate or potassium acetate. In some embodiments, the third base of step a1) is potassium acetate; and the first base of step a) is potassium carbonate.

In some embodiments, the fourth solvent of step a1) and the first solvent of step a) are each independently water, dioxane, tetrahydrofuran (THF), 2-methyl-tetrahydrofuran (MeTHF), or a combination thereof. In some embodiments, the fourth solvent of step a1) and the first solvent of step a) are each independently water, 1,4-dioxane, or a combination thereof. In some embodiments, the fourth solvent of step a1) is 1,4-dioxane; and the first solvent of step a) is a mixture of 1,4-dioxane and water.

In some embodiments, the second base of step c) is lithium hydroxide, sodium hydroxide, or potassium hydroxide. In some embodiments, the second base of step c) is sodium hydroxide. In some embodiments, the second base of step c) is an aqueous solution of sodium hydroxide.

In some embodiments, the third solvent of step c) is water, dioxane, tetrahydrofuran (THF), 2-methyl-tetrahydrofuran (MeTHF), or a combination thereof. In some embodiments, the third solvent of step c) is a mixture water and tetrahydrofuran (THF).

In some embodiments, the second acid is hydrochloric acid. In some embodiments, the second acid is an aqueous solution of hydrochloric acid.

With reference to any one of formulae (I), (II), (III-1a), (V-1), in some embodiments, subscripts m and n are each 1. In some embodiments, one of subscripts m and n is 2 and the other is 1. In some embodiments, subscripts m and n are each 2.

With reference to any one of formulae (II), (III-1a), (IV-1a-1), and (VI-1a), in some embodiments, R¹ is methyl or ethyl. In some embodiments, R¹ is methyl.

All other reaction conditions of steps a1) and a) to d) are described above.

In some embodiments, step e) is conducted by treating the compound of formula (I) or a tautomer thereof with an aqueous solution of sodium hydroxide in water. In some embodiments, sodium hydroxide is in an amount of less than 1.0 equivalent relative to the compound of formula (I), on a salt-free and anhydrous basis. In some embodiments, sodium hydroxide is in an amount of from 0.80 to 0.95 or from 0.85 to 0.95 equivalent relative to the compound of formula (I), on a salt-free and anhydrous basis. In some embodiments, sodium hydroxide is in an amount of about 0.88 equivalent relative to the compound of formula (I), on a salt-free and anhydrous basis. In some embodiments, a reaction mixture of step e) has a pH value of about 9.5.

In some embodiments, step e) is conducted by:
i) treating the compound of formula (I) or a tautomer thereof with an aqueous solution of sodium hydroxide in water;
ii) forming a slurry having a pH value of about 9.5; and
iii) lyophilizing the slurry to provide the mono-sodium salt of the compound of formula (I) represented by the formula: 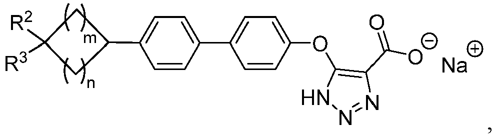 or a tautomer thereof,
wherein sodium hydroxide is in an amount of less than 1.0 equivalent relative to the compound of formula (I), on a salt-free and anhydrous basis; subscripts m and n are each independently 1 or 2; and R² and R³ are each F. In some embodiments, sodium hydroxide is in an amount of about 0.88 equivalent relative to the compound of formula (I), on a salt-free and anhydrous basis.

In another aspect, the present disclosure provides a process for preparing a compound represented by the formula (Ia-1): 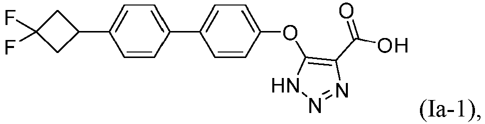 a tautomer thereof, or a salt thereof. The process includes:
a1) converting a compound of formula (VI-1a-1): 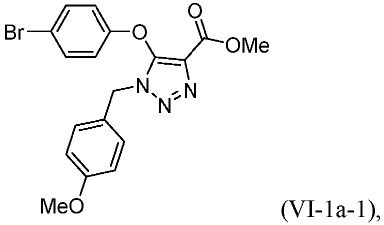 or a salt thereof, with bis(pinacolato)diboron, Pd(dppf)Cl₂·CH₂Cl₂, and potassium acetate in 1,4-dioxane to a compound of formula (IV-1a-2): 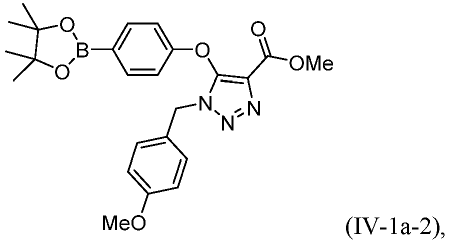 or a salt thereof;
a) contacting the compound of formula (IV-1a-2) or the salt thereof with 1-bromo-4-(3,3-difluorocyclobutyl)benzene, Pd(dppf)Cl₂·CH₂Cl₂, and potassium carbonate in a mixture of 1,4-dioxane and water to form a compound of formula (IIIa-1a-1): 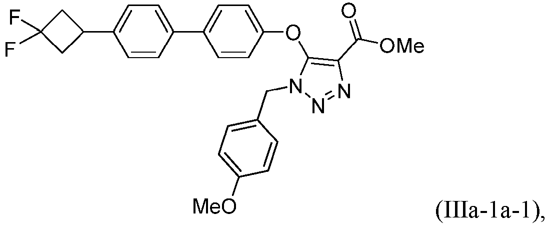 or a salt thereof;
b) treating the compound of formula (IIIa-1a-1) or the salt thereof, with trifluoroacetic acid and anisole in dichloromethane to provide a compound of formula (IIa-1): 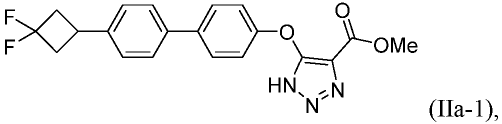 a tautomer thereof, or a salt thereof;
c) saponifying the compound of formula (IIa-1), the tautomer thereof, or the salt thereof with an aqueous solution of sodium hydroxide in tetrahydrofuran; and
d) acidifying with an aqueous solution of HCl to provide the compound of formula (Ia-1), the tautomer thereof, or the salt thereof.

In some embodiments, step a1) is conducted at a temperature of about 80°C; step a) is conducted at a temperature of about 80°C; step b) is conducted at a temperature of about 50°C; and step c) is conducted at a temperature of about 55°C; and step d) is conducted at a temperature of about 20-25°C.

In some embodiments of step a1), Pd(dppf)Cl₂·CH₂Cl₂ is in an amount of from 0.02 to 0.5 equivalent relative to the compound of formula (VI-1a-1). In some embodiments of step a1), Pd(dppf)Cl₂·CH₂Cl₂ is in an amount of about 0.04 equivalent relative to the compound of formula (VI-1a-1). In some embodiments of step a1), bis(pinacolato)diboron is in amount of about 1.5 equivalents relative to the compound of formula (VI-1a-1). In some embodiments of step a1), potassium acetate is in an amount of about 3.0 equivalents relative to the compound of formula (VI-1a-1).

In some embodiments of step a), 1-bromo-4-(3,3-difluorocyclobutyl)benzene is in an amount of from 1.0 to 1.5 equivalents relative to the compound of formula (IV-1a-2). In some embodiments of step a), 1-bromo-4-(3,3-difluorocyclobutyl)benzene is in an amount of about 1.2 equivalents relative to the compound of formula (IV-1a-2). In some embodiments of step a), Pd(dppf)Cl₂·CH₂Cl₂ is in an amount of from 0.05 to 0.1 equivalent relative to the compound of formula (IV-1a-2). In some embodiments of step a), Pd(dppf)Cl₂·CH₂Cl₂ is in an amount of about 0.07 equivalent relative to the compound of formula (IV-1a-2). In some embodiments of step a), potassium carbonate is in an amount of from 2.0 to 4.0 equivalents relative to the compound of formula (IV-1a-2). In some embodiments of step a), potassium carbonate is in an amount of about 3.5 equivalents relative to the compound of formula (IV-1a-2).

In some embodiments, the compound of formula (IV-1a-2) produced by step a1) is directly used in the next step (i.e., step a)) without further purification.

In some embodiments, the compound of formula (IIIa-1a-1) is isolated in a combined yield of at least 50% from both steps a1) and a). In some embodiments, the compound of formula (IIIa-1a-1) is isolated in a combined yield of from 50% to 80% or from 60% to 70% from both steps a1) and a). In some embodiments, the compound of formula (IIIa-1a-1) is isolated in a combined yield of about 60% from both steps a1) and a).

In some embodiments, the compound of formula (IIa-1) produced by step b) is directly used in next step (i.e., step c)) without further purification.

In some embodiments, step d) is conducted by acidifying an aqueous extract of a reaction mixture of step c).

In some embodiments, the process further includes:
e) converting the compound of formula (Ia-1), the tautomer thereof, or the salt thereof, to a mono-sodium salt of the compound of formula (Ia-1) represented by the formula: 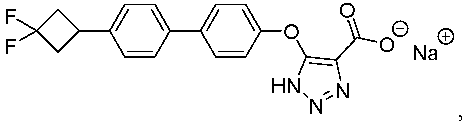 a di-sodium salt of the compound of formula (Ia-1) represented by the formula: 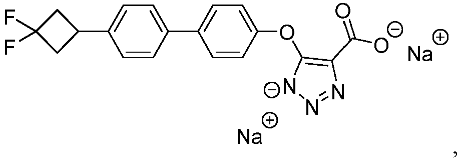 or a mixture thereof, or a tautomer thereof.

In some embodiments, step e) is conducted by treating the compound of formula (Ia-1) or a tautomer thereof with an aqueous solution of sodium hydroxide in water. In some embodiments, sodium hydroxide is in an amount of less than 1.0 equivalent relative to the compound of formula (Ia-1), on a salt-free and anhydrous basis. In some embodiments, sodium hydroxide is in an amount of from 0.80 to 0.95 or from 0.85 to 0.95 equivalent relative to the compound of formula (Ia-1), on a salt-free and anhydrous basis. In some embodiments, sodium hydroxide is in an amount of about 0.88 equivalent relative to the compound of formula (Ia-1), on a salt-free and anhydrous basis. In some embodiments, a reaction mixture of step e) has a pH value of about 9.5.

In some embodiments, step e) is conducted by:
i) treating the compound of formula (Ia-1) or a tautomer thereof with an aqueous solution of sodium hydroxide in water;
ii) forming a slurry having a pH value of about 9.5; and
iii) lyophilizing the slurry to provide the mono-sodium salt of the compound of formula (Ia-1) represented by the formula: 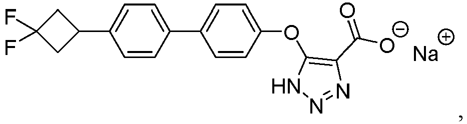 or a tautomer thereof,
wherein sodium hydroxide is in an amount of less than 1.0 equivalent relative to the compound of formula (Ia-1), on a salt-free and anhydrous basis; subscripts m and n are each independently 1 or 2; and R² and R³ are each F. In some embodiments, sodium hydroxide is in an amount of about 0.88 equivalent relative to the compound of formula (Ia-1), on a salt-free and anhydrous basis.

Certain compounds of the present disclosure can exist in unsolvated forms as well as solvated forms, including hydrated forms. In general, the solvated forms are equivalent to unsolvated forms and are intended to be encompassed within the scope of the present disclosure.

Certain compounds of this disclosure may exist in tautomeric forms, all such tautomeric forms of the compounds being within the scope of the present disclosure. Tautomer refers to one of two or more structural isomers which exist in equilibrium and which are readily converted from one isomeric form to another. For example, 1,2,3-triazole of the following formula can exit in equilibrium:

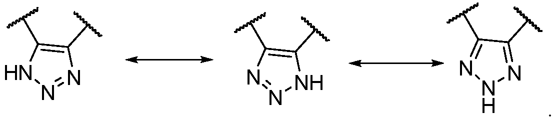

In some embodiments of formula (I), compounds of the following formulae can exist in equilibrium:

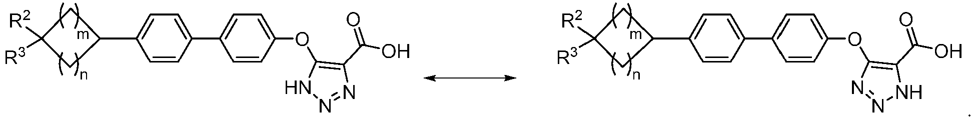

In some embodiments of formula (II), compounds of the following formulae can exist in equilibrium:

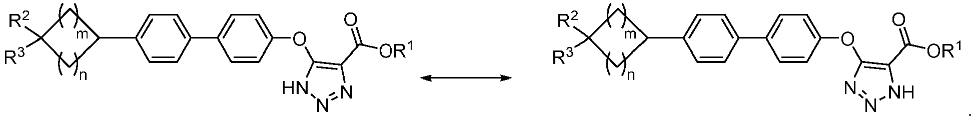

In some embodiments, the compound of the present disclosure may exist as a monobasic addition salt, wherein the carboxylic acid or 1,2,3-triazole moiety is deprotonated and forms a salt with a base. In some embodiments, the compound of the present disclosure may exist as a dibasic addition salt, wherein the carboxylic acid and 1,2,3-triazole moiety are both deprotonated and form a dibasic salt with a base. In some embodiments, compounds of formula (I) in sodium salts can have a mono-sodium salt having the formula: 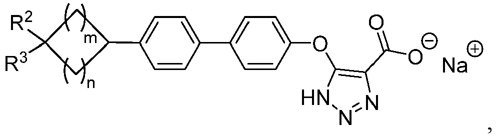
a di-sodium salt having the formula: 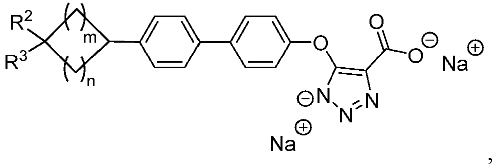 or
a mixture thereof, wherein subscripts m and n, R², and R³ are as defined and described herein. In some embodiments, compounds of formula (II) in sodium salts can have the formula: 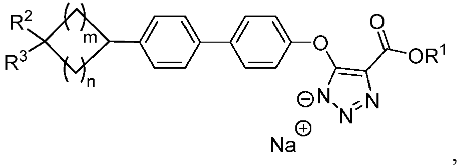
wherein subscripts m and n, R¹, R², and R³ are as defined and described herein.

### IV. COMPOUNDS

Also described herein but not encompassed by the wording of the claims is a compound represented by formula (X): 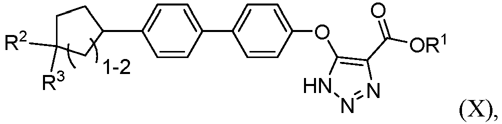 a tautomer thereof, or a pharmaceutically acceptable salt thereof, wherein R¹ is H or C₁₋₆ alkyl; and R² and R³ are each independently H or halogen.

As described herein, the compound of formula (X) may be represented by formula (Xa):

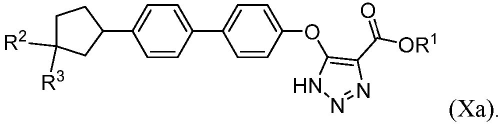

As described herein, the compound of formula (X) may be represented by formula (Xb):

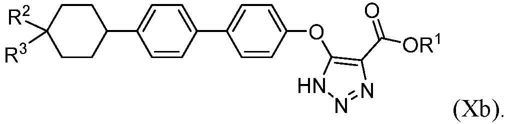

With reference to any one of formulae (X), (Xa), and (Xb), as described herein, R¹ may be H. As described herein, R¹ may be C₁₋₆ alkyl. As described herein, R¹ may be methyl, ethyl, propyl, isopropyl, butyl, isobutyl, sec-butyl, tert-butyl, pentyl, isopentyl, or hexyl. As described herein, R¹ may be methyl. As described herein, R¹ may be ethyl.

With reference to any one of formulae (X), (Xa), and (Xb), as described herein, R² and R³ may each be independently halogen. As described herein, R² and R³ may each be independently F, Cl, Br, or I. As described herein, R² and R³ may each be F.

Exemplified compounds of formula (X) are listed in Table 1.

**Table 1: Compounds**

| **Formula** | **Structures** |
|---|---|
| **Ib-1** | 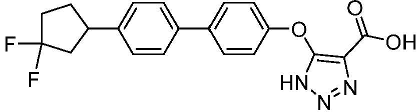 |
| **IIb-1** | 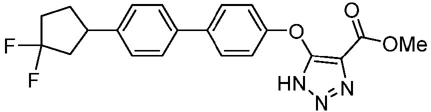 |
| **Ic-1** | 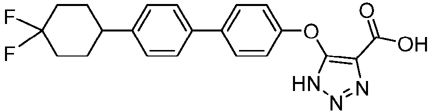 |
| **IIc-1** | 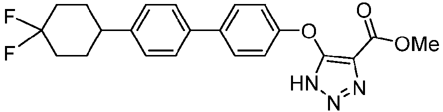 |

As described herein, the compound of formula (X) may be represented by the formula (Ib-1):

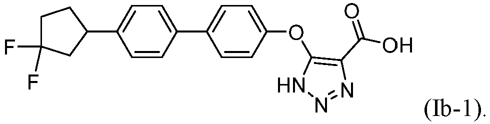

As described herein, the compound of formula (X) may be represented by the formula (Ic-1):

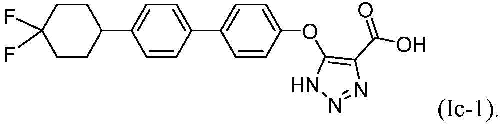

The compounds described herein may exist as salts.. When compounds contain relatively acidic functionalities (e.g., 1,2,3-triazole-4-carboxylic acid or 1,2,3-triazole), the present disclosure includes base addition salts such as sodium, potassium, calcium, ammonium, organic amino, or magnesium salt, or a similar salt. When R¹ is C₁₋₆ alkyl, the present disclosure may include acid addition salts, such as mineral acid (hydrochloric acid, hydrobromic acid, phosphoric acid, and the like) salts, and organic acid (acetic acid, propionic acid, glutamic acid, citric acid and the like) salts.

Pharmaceutically acceptable salts includes salts of the active compounds which are prepared with relatively nontoxic acids or bases, depending on the particular substituents found on the compounds described herein. When compounds of the present disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. It is understood that the pharmaceutically acceptable salts are non-toxic. Additional information on suitable pharmaceutically acceptable salts can be found in Remington's Pharmaceutical Sciences, 17th ed., Mack Publishing Company, Easton, Pa., 1985.

, The compound may exist as a monobasic addition salt, wherein the carboxylic acid or 1,2,3-triazole moiety is deprotonated and forms a salt with a base. The compound may exist as a dibasic addition salt, wherein the carboxylic acid and 1,2,3-triazole moiety are both deprotonated and form a dibasic salt with a base. As described herien, compounds of formula (X) in sodium salts can have any one of following formulae: 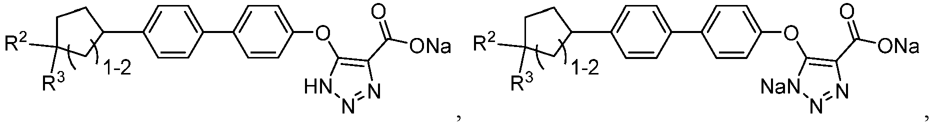 and 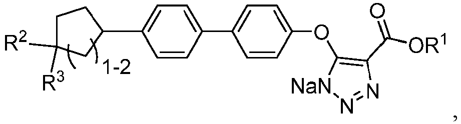 wherein R¹, R², and R³ are as defined and described herein.

The neutral forms of the compounds are preferably regenerated by contacting the salt with a base or acid and isolating the parent compound. The parent form of the compound differs from the various salt forms in certain physical properties, such as solubility in polar solvents.

Certain compounds can exist in unsolvated forms as well as solvated forms, including hydrated forms. In general, the solvated forms are equivalent to unsolvated forms and are encompassed within the scope of the present disclosure. Certain compounds may exist in multiple crystalline or amorphous forms. In general, all physical forms are equivalent for the uses contemplated by the present disclosure and are intended to be within the scope of the present disclosure.

Certain compounds possess asymmetric carbon atoms (optical centers); the enantiomers, racemates, diastereomers, tautomers, geometric isomers, stereoisometric forms that may be defined, in terms of absolute stereochemistry, as (R)-or (S)-, and individual isomers are encompassed within the scope of the present disclosure. The compounds of the present disclosure do not include those which are known in art to be too unstable to synthesize and/or isolate. The present disclosure is meant to include compounds in racemic and optically pure forms. Optically active (R)- and (S)-isomers may be prepared using chiral synthons or chiral reagents, or resolved using various techniques.

Isomers include compounds having the same number and kind of atoms, and hence the same molecular weight, but differing in respect to the structural arrangement or configuration of the atoms.

Certain compounds of this disclosure may exist in tautomeric forms, all such tautomeric forms of the compounds being within the scope of the present disclosure. Tautomer refers to one of two or more structural isomers which exist in equilibrium and which are readily converted from one isomeric form to another. For example, 1,2,3-triazole of the following formula can exit in equilibrium:

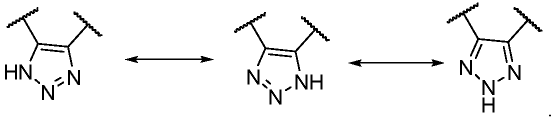

For compounds of formula (X) as herein described, which as noted above are not encompassed by the wording of the claims, compounds of the following formulae can exist in equilibrium:

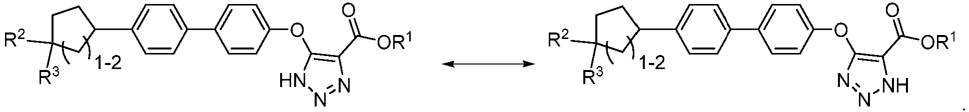

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the present disclosure.

Unless otherwise stated, the compounds of the present disclosure may also contain unnatural proportions of atomic isotopes at one or more of the atoms that constitute such compounds. For example, the compounds of the present disclosure may be labeled with radioactive or stable isotopes, such as for example deuterium (²H), tritium (³H), iodine-125 (¹²⁵I), fluorine-18 (¹⁸F), nitrogen-15 (¹⁵N), oxygen-17 (¹⁷O), oxygen-18 (¹⁸O), carbon-13 (¹³C), or carbon-14 (¹⁴C). All isotopic variations of the compounds of the present disclosure, whether radioactive or not, are encompassed within the scope of the present disclosure.

### V. COMPOSITION

The compositions as described herein but not encompassed by the wording of the claims can be prepared in a wide variety of oral, parenteral and topical dosage forms. Oral preparations include tablets, pills, powder, dragees, capsules, liquids, lozenges, cachets, gels, syrups, slurries, suspensions, etc., suitable for ingestion by the patient. The compositions as described herein can also be administered by injection, that is, intravenously, intramuscularly, intracutaneously, subcutaneously, intraduodenally, or intraperitoneally. Also, the compositions described herein can be administered by inhalation, for example, intranasally. Additionally, the compositions as described herein can be administered transdermally. The compositions as described herein can also be administered by intraocular, intravaginal, and intrarectal routes including suppositories, insufflation, powders and aerosol formulations (for examples of steroid inhalants, see Rohatagi, J. Clin. Pharmacol. 35:1187-1193, 1995; Tjwa, Ann. Allergy Asthma Immunol. 75:107-111, 1995). Accordingly, described herein but not encompassed by the woding of the claims are pharmaceutical compositions including one or more pharmaceutically acceptable carriers and/or excipients and either a compound provided herein (e.g., a compound of formula (X)), or a pharmaceutically acceptable salt of a compound provided herein (e.g., a compound of formula (X)).

For preparing pharmaceutical compositions from the compounds as herein described, pharmaceutically acceptable carriers can be either solid or liquid. Solid form preparations include powders, tablets, pills, capsules, cachets, suppositories, and dispersible granules. A solid carrier can be one or more substances, which may also act as diluents, flavoring agents, binders, preservatives, tablet disintegrating agents, or an encapsulating material. Details on techniques for formulation and administration are well described in the scientific and patent literature, see, e.g., the latest edition of Remington's Pharmaceutical Sciences, Maack Publishing Co, Easton PA ("Remington's").

In powders, the carrier is a finely divided solid, which is in a mixture with the finely divided active component. In tablets, the active component is mixed with the carrier having the necessary binding properties in suitable proportions and compacted in a particular shape and size.

The powders, capsules and tablets preferably contain from about 5% to about 70% of the active compound, such as from about 10% to about 70% of the active compound. Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other excipients, is surrounded by a carrier, which is thus in association with it. Similarly, cachets and lozenges are included. Tablets, powders, capsules, pills, cachets, and lozenges can be used as solid dosage forms suitable for oral administration.

Suitable solid excipients include, but are not limited to, magnesium carbonate; magnesium stearate; talc; pectin; dextrin; starch; tragacanth; a low melting wax; cocoa butter; carbohydrates; sugars including, but not limited to, lactose, sucrose, mannitol, or sorbitol, starch from corn, wheat, rice, potato, or other plants; cellulose such as methyl cellulose, hydroxypropylmethyl-cellulose, or sodium carboxymethylcellulose; and gums including arabic and tragacanth; as well as proteins including, but not limited to, gelatin and collagen. Disintegrating or solubilizing agents may be added, such as the cross-linked polyvinyl pyrrolidone, agar, alginic acid, or a salt thereof, such as sodium alginate.

Dragee cores are provided with suitable coatings such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound (i.e., dosage). Pharmaceutical preparations as described herein but not encompassed by the wording of the claims can also be used orally using, for example, push-fit capsules made of gelatin, as well as soft, sealed capsules made of gelatin and a coating such as glycerol or sorbitol. Push-fit capsules can contain a compound provided herein (e.g., a compound of formula (X)) mixed with a filler or binders such as lactose or starches, lubricants such as talc or magnesium stearate, and, optionally, stabilizers. In soft capsules, the compound provided herein (e.g., a compound of formula (X)) may be dissolved or suspended in suitable liquids, such as fatty oils, liquid paraffin, or liquid polyethylene glycol with or without stabilizers.

For preparing suppositories, a low melting wax, such as a mixture of fatty acid glycerides or cocoa butter, is first melted and the compound described herein (e.g., a compound of formula (X)) are dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the compound provided herein (e.g., a compound of formula (X)) in water and adding optional suitable colorants, flavors, stabilizers, and thickening agents. Aqueous suspensions suitable for oral use can be made by dispersing the finely divided active component in water with viscous material, such as natural or synthetic gums, resins, methylcellulose, sodium carboxymethylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Also included are solid form preparations, which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

Oil suspensions can be formulated by suspending the compound provided herein (e.g., a compound of formula (X)) in a vegetable oil, such as arachis oil, olive oil, sesame oil or coconut oil, or in a mineral oil such as liquid paraffin; or a mixture of these. The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto, J. Pharmacol. Exp. Ther. 281:93-102, 1997. The pharmaceutical formulations as herein described can also be in the form of oil-in-water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent.

The compositions as herein described can be delivered by any suitable means, including oral, parenteral and topical methods. Transdermal administration methods, by a topical route, can be formulated as applicator sticks, solutions, suspensions, emulsions, gels, creams, ointments, pastes, jellies, paints, powders, and aerosols.

The compositions as herein described can also be delivered as microspheres for slow release in the body. For example, microspheres can be formulated for administration via intradermal injection of drug-containing microspheres, which slowly release subcutaneously (see Rao, J. Biomater Sci. Polym. Ed. 7:623-645, 1995; as biodegradable and injectable gel formulations (see, e.g., Gao Pharm. Res. 12:857-863, 1995); or, as microspheres for oral administration (see, e.g., Eyles, J. Pharm. Pharmacol. 49:669-674, 1997). Both transdermal and intradermal routes afford constant delivery for weeks or months.

The compositions as herein described can be formulated for parenteral administration, such as intravenous (IV) administration or administration into a body cavity or lumen of an organ. The formulations for administration will commonly comprise a solution of the compositions as herein described dissolved in a pharmaceutically acceptable carrier. Among the acceptable vehicles and solvents that can be employed are water and Ringer's solution, an isotonic sodium chloride. In addition, sterile fixed oils can be employed as a solvent or suspending medium. For this purpose any bland fixed oil can be employed including synthetic mono- or diglycerides. In addition, fatty acids such as oleic acid can likewise be used in the preparation of injectables. These solutions are sterile and generally free of undesirable matter. These formulations may be sterilized by various sterilization techniques. The formulations may contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of the compositions as herein described in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated using those suitable dispersing or wetting agents and suspending agents. The sterile injectable preparation can also be a sterile injectable solution or suspension in a nontoxic parenterally-acceptable diluent or solvent, such as a solution of 1,3-butanediol.

The formulations of the compositions as herein described can be delivered by the use of liposomes. Without wishing to be bound by theory, such liposomes may fuse with the cellular membrane; or be endocytosed, i.e., by employing ligands attached to the liposome or directly attach to the oligonucleotide, that bind to surface membrane protein receptors of the cell resulting in endocytosis. By using liposomes, particularly where the liposome surface carries ligands specific for target cells, or are otherwise preferentially directed to a specific organ, one can focus the delivery of the compositions as herein described into the target cells in vivo. (See, e.g., Al-Muhammed, J. Microencapsul. 13:293-306, 1996; Chonn, Curr. Opin. Biotechnol. 6:698-708, 1995; Ostro, Am. J. Hosp. Pharm. 46:1576-1587, 1989).

Lipid-based drug delivery systems include lipid solutions, lipid emulsions, lipid dispersions, self-emulsifying drug delivery systems (SEDDS) and self-microemulsifying drug delivery systems (SMEDDS). In particular, SEDDS and SMEDDS are isotropic mixtures of lipids, surfactants and co-surfactants that can disperse spontaneously in aqueous media and form fine emulsions (SEDDS) or microemulsions (SMEDDS). Lipids useful in the formulations as herein described include any natural or synthetic lipids including, but not limited to, sesame seed oil, olive oil, castor oil, peanut oil, fatty acid esters, glycerol esters, Labrafil^{®}, Labrasol^{®}, Cremophor^{®}, Solutol^{®}, Tween^{®}, Capryol^{®}, Capmul^{®}, Captex^{®}, and Peceol^{®}.

The pharmaceutical formulations of the compounds of formula (X) as herein descibred but not encompassed by the wording of the claims can be provided as a salt and can be formed with many acids, including but not limited to hydrochloric, sulfuric, acetic, lactic, tartaric, malic, succinic, etc. Salts tend to be more soluble in aqueous or other protonic solvents that are the corresponding free base forms. In other cases, the preparation may be a lyophilized powder in 1 mM-50 mM histidine, 0.1%-2% sucrose, 2%-7% mannitol at a pH range of 4.5 to 5.5, that is combined with buffer prior to use.

The pharmaceutical formulations of the compounds of formula (X) as herein described but not encompassed by the wording of the claims can be provided as a salt and can be formed with bases, namely cationic salts such as alkali and alkaline earth metal salts, such as sodium, lithium, potassium, calcium, magnesium, as well as ammonium salts, such as ammonium, trimethyl-ammonium, diethylammonium, and tris-(hydroxymethyl)-methyl-ammonium salts.

### VI. METHODS

Compounds of formula (I), (II), or (X) and compounds as listed in Table 1 are useful as glycolate oxidase inhibitors, and methods for inhibiting glycolate oxidase are also described herein but not encompassed by the wording of the claims. The methods include contacting glycolate oxidase with a compound according to any one of formulae (I), (II), (X), (Xa), and (Xb) as described above, a pharmaceutically acceptable salt thereof, or a pharmaceutical composition containing the compound or salt to a subject (e.g., patient) in need thereof. Inhibiting glycolate oxidase generally includes contacting the glycolate oxidase with an amount of the compound sufficient to reduce the activity of the glycolate oxidase as compared to the glycolate oxidase activity in the absence of the compound. For example, contacting glycolate oxidase with a compound according to any one of formulae (I), (II), (X), (Xa), and (Xb) can result in from about 1% to about 99% glycolate oxidase inhibition (*i.e.,* the activity of the inhibited glycolate oxidase ranges from 99% to 1% of the glycolate oxidase activity in the absence of the compound). The level of glycolate oxidase inhibition can range from about 1% to about 10%, or from about 10% to about 20%, or from about 20% to about 30%, or from about 30% to about 40%, or from about 40% to about 50%, or from about 50% to about 60%, or from about 60% to about 70%, or from about 70% to about 80%, or from about 80% to about 90%, or from about 90% to about 99%. The level of glycolate oxidase inhibition can range from about 5% to about 95%, or from about 10% to about 90%, or from about 20% to about 80%, or from about 30% to about 70%, or from about 40% to about 60%. As described herein, contacting glycolate oxidase with a compound as described herein will result in complete (*i*.*e*., 100%) glycolate oxidase inhibition. As described herein, the compound of formula (I), (II), or (X) reduces glyoxylate level. As described herein, the compound of formula (X) reduces glyoxylate level.

The methods for inhibiting glycolate oxidase as herein described but not encompassed by the wording of the claims include contacting glycolate oxidase with a compound represented by the formula: 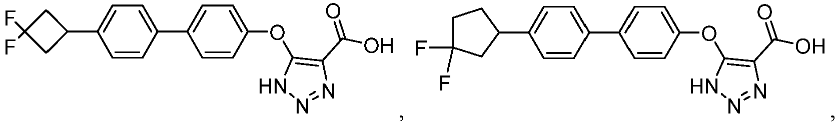 or 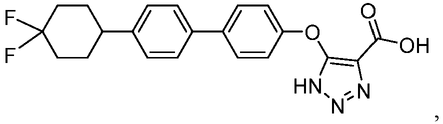 a tautomer thereof, or a pharmaceutically acceptable salt thereof.

The methods for inhibiting glycolate oxidase as herein described include contacting glycolate oxidase with a compound represented by the formula: 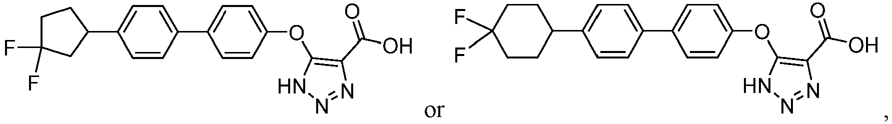 a tautomer thereof, or a pharmaceutically acceptable salt thereof.

In primary hyperoxaluria type I (PH1), mutation of alanine-glyoxylate transaminate (AGT) disrupts the glyoxylate detoxification pathway. Mutation of AGT prevents AGT from converting glyoxylate to pyruvate, and the resulting build-up of glyoxylate results in higher levels of oxalate and oxalate-containing kidney stones. Glycolate oxidase (GO) is a peroxisomal hepatic enzyme which catalyzes the oxidation of glycolate to glyoxylate, the AGT substrate. As such, GO plays a pivotal role in glyoxylate production while AGT plays a pivotal role in glyoxylate detoxification. Described herein are compounds and methods for treating PH1 by targeting GO, the source of the AGT substrate. GO inhibitors as described herein can reduce glyoxylate levels in PH1 patients, thus compensating for the inability of mutant AGT-located downstream of GO in the glyoxylate detoxification pathway-to metabolize glyoxylate and preventing the harmful build-up of oxalate. As described herein, the compound of formula (I) reduces glyoxylate level in PH1 patients or the subject having PH1.

Also described herein are methods for treating primary hyperoxaluria (PH1). PH1 has a prevalence of 1-3 per million individuals and an incidence of 1-9: 100,000 live births per year in Europe [Salido, *supra*]. PH1 is caused by mutations of the gene encoding peroxisomal enzyme AGT, which fails to detoxify glyoxylate and leads to a marked increase in oxalate synthesis by the liver. In PH1, excreted urinary oxalate (UOx) is elevated leading to the production of insoluble calcium oxalate (CaOx) crystals which tend to precipitate primarily in the kidney, forming kidney stones and diffuse nephrocalcinosis [Kaufman, *supra*]. This impairs renal function which progresses to end-stage renal disease (ESRD). Once renal function declines to a glomerular filtration rate (GFR) below 30 ml/min/1.73m², the amount of oxalate produced by the liver can no longer be cleared by the kidneys, leading to systemic deposition of CaOx (oxalosis). In people with PH1, the accumulated oxalate is deposited in the kidneys and urinary tract. It combines with calcium, forming the main component of kidney and bladder stones. First symptoms of PH1 include hematuria, abdominal pain, passage of a stone, or repeated urinary tract infections. The initial diagnosis is based on clinical and sonographic findings, and UOx assessment. AGT activity assessment in a liver biopsy and/or DNA analysis is required to confirm a PH1 diagnosis and to initiate conservative treatment (high fluid intake, pyridoxine, CaOx crystallization inhibitors), aimed at maintaining renal function. The most effective treatment for PH1 is liver transplantation (LTx), alone (pre-emptive) or combined with kidney transplantation [Cochat, et al. (2012) Nephrol Dial Transplant. 27: 1729].

As described herein, the methods for treating PH1 include administering a compound according to any one of formulae (I), (II), (X), (Xa), and (Xb) as described above, a pharmaceutically acceptable salt thereof; or a pharmaceutical composition containing the compound or salt to a subject in need thereof. As described herein, the subject may have kidney and/or bladder stones. As described herein, the compound of formula (X) may reduce glyoxylate level in the subject. As described herein, the compound of formula (X) may reduce an accumulation of oxalate in kidney and/or urinary tract.

The methods for treating PH1 as herein described include administering a compound represented by the formula: 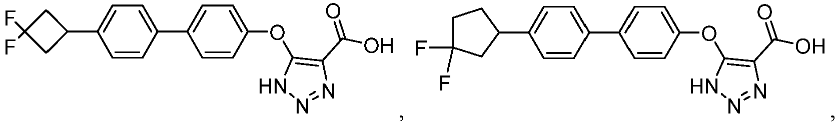 or 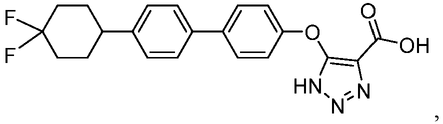 a tautomer thereof, or a pharmaceutically acceptable salt thereof.

The methods for treating PH1 as herein described include administering a compound represented by the formula: 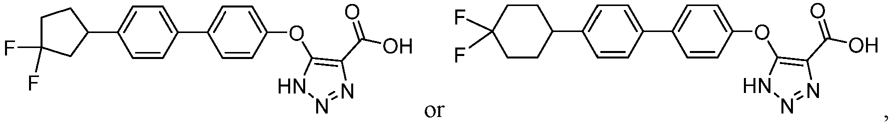 a tautomer thereof, or a pharmaceutically acceptable salt thereof.

Methods for treating kidney and/or bladder stones are also described herein but not encompassed by the wording of the claims. The methods include administering a compound according to any one of formulae (I), (II), (X), (Xa), and (Xb) as described above, a pharmaceutically acceptable salt thereof, or a pharmaceutical composition containing the compound or salt to a subject in need thereof. As described herein, the compound of formula (X) may reduce an accumulation of oxalate in kidney and/or urinary tract.

The methods for treating kidney and/or bladder stonesas herein describedinclude administering a compound represented by the formula: 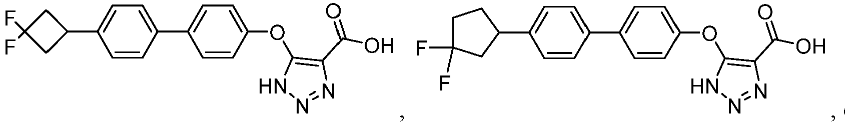 or 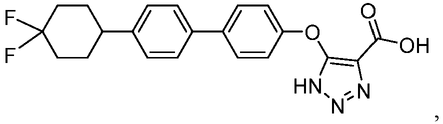 a tautomer thereof, or a pharmaceutically acceptable salt thereof.

The methods for treating kidney and/or bladder stonesas herein described include administering a compound represented by the formula: 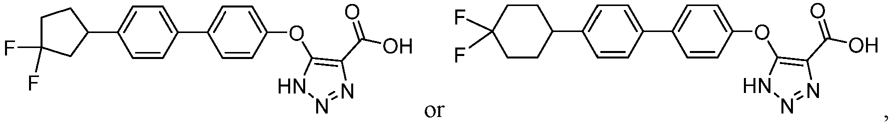 a tautomer thereof, or a pharmaceutically acceptable salt thereof.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the present compounds are within the scope of the present disclosure.

### VII. EXAMPLES

The following abbreviations are used in the examples below:
- aq: aqueous
- bs: broad singlet
- CD₃OD: methanol-d₄
- CDCl₃: chloroform-d
- conc.: concentrated
- DAST: diethylaminosulfur trifluoride
- DCE: 1,2-dichloroethane
- DCM: dichloromethane
- DIPEA: diisopropylethyl amine
- DMA: dimethylacetamide
- DMF: dimethylformamide
- DMSO: dimethylsulfoxide
- Eq.: equivalent
- Et₂O: diethylether
- EtOAc: ethylacetate
- h: hour(s)
- Hex: hexanes
- HPLC: high performance liquid chromatography
- LRMS: low resolution mass spec
- M: molar
- MeOH: methanol
- MTBE: Methyl tert-butyl ether
- min: minute(s)
- NaCl: sodium chloride
- Na₂SO₄: sodium sulfate
- PMB: paramethoxybenzyl
- RBF: round bottom flask
- rt: room temperature
- t_{R}: retention time
- satd.: saturated
- SiO₂: silica gel
- TFA: trifluoroacetic acid
- Tf₂O: trifluoromethanesulfonic anhydride
- THF: tetrahydrofuran
- TLC: thin layer chromatography
- XtalFluor-E: *N,N*-diethyl-*S,S*-difluorosulfiliminium tetrafluoroborate.

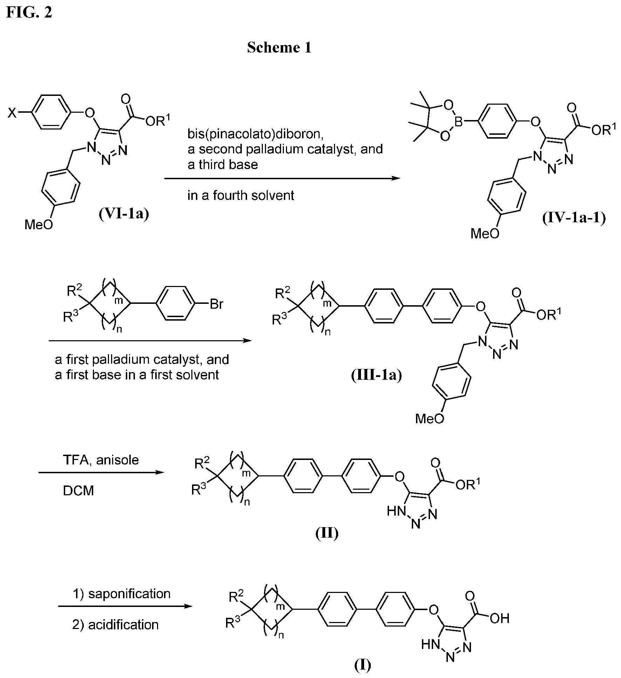

The compounds of formulae (I) and (II) can be synthesized via Suzuki reaction of the compound of formula (IV-1a-1) and the compound of formula (V-1) according to Scheme 1 as shown in FIG. 2, in which subscripts m and n, R¹, R², R³, and X are as defined and described herein; and the first and second palladium catalysts, the first and third bases, and the first and fourth solvents are defined and described herein.

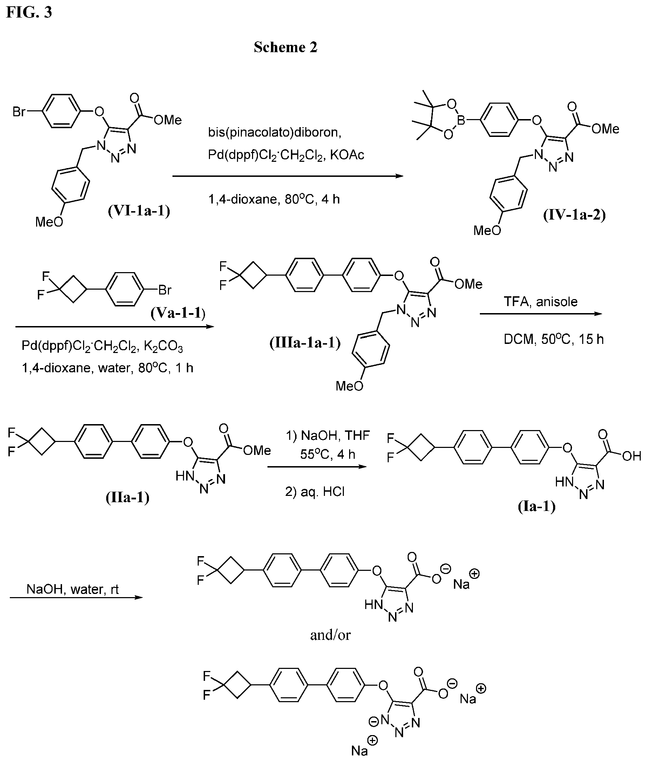

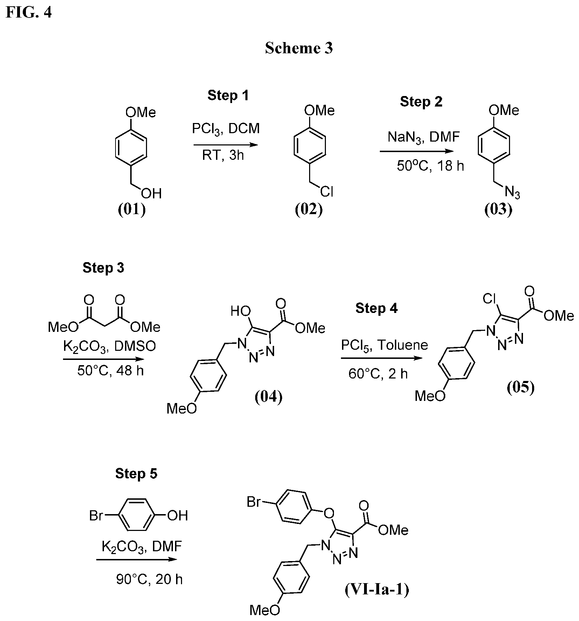

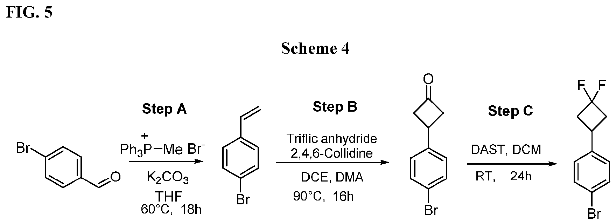

In particular, the compound of formula (Ia-1) was prepared according to Scheme 2 as shown in FIG. 3. The intermediate, the compound of formula (VI-1a-1), was prepared according to Scheme 3 as shown in FIG. 4. 1-Bromo-4-(3,3-difluorocyclobutyl)benzene was prepared according to Scheme 4 as shown in FIG. 5.

### Example 1: Methyl 5-(4-bromophenoxy)-1-(4-methoxybenzyl)-1H-1,2,3-triazole-4-carboxylate (VI-1a-1)

### Step-1: 1-(chloromethyl)-4-methoxybenzene (02)

### Step 1

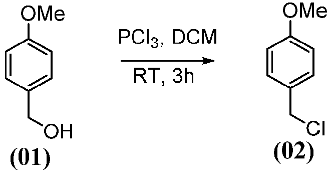

PCl₃ (2.73 kg, 19.9 mol, 1.1 equiv.) was added to a stirred solution ofp-methoxybenzyl alcohol (2.5 kg, 18.09 mol, 1.0 equiv.) in DCM (12.5 L, 5.0 Vol) at 0°C. The reaction mixture was stirred for 3.0 h at RT. Reaction was monitored by TLC (mobile phase: 60% EtOAc in *n-*heptane). The reaction mixture was poured into chilled aqueous ammonia (10.0 L, 4.0 vol). Organic layer was collected and the aqueous layer was further extracted with DCM (2 X 6.0 L). The combined organic extract was washed with brine (100 L, 4.0 vol), dried over sodium sulphate. After filtration, the filtrate was concentrated under *vacuum* at 40°C to provide crude p-methoxybenzyl chloride (02) (2.45 kg, 86.48%) as a yellow oil, which was used in step 2 without further purification. ¹H NMR (300 MHz, Chloroform-d) δ 7.17 (d, *J* = 8.7 Hz, 2H), 6.75 (d, *J* = 8.7 Hz, 2H), 4.42 (s, 2H), 3.65 (s, 3H).

### Step 2: 1-(azidomethyl)-4-methoxybenzene (03)

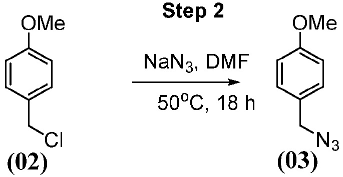

Sodium azide (1.19 kg, 18.3 mol, 1.2 equiv.) was added to a stirred solution ofp-methoxybenzyl chloride (02) (2.4 kg, 15.32 mol, 1.0 equiv.) in DMF (9.6 L, 4.0 Vol) at RT. The reaction mixture was stirred for 18.0 h at 50°C. The progress of the reaction was monitored by TLC (mobile phase: 100 % n-heptane). The reaction mixture was quenched with water (24.0 L, 10.0 Vol) and extracted with EtOAc (2 x 20.0 L). The combined organic extract was washed with water (2 x 20.0 L) and brine (3 x 20.0 L). After drying over sodium sulphate, it was filtered and the solvent was evaporated under *vacuum* at 50°C to provide the crude 1-(azidomethyl)-4-methoxybenzene (03) (2.12 kg) as a brown oil. The crude product was used in step 3 without further purification. ¹H NMR (300 MHz, Chloroform-*d*) δ 7.27 (d, *J* = 8.6 Hz, 2H), 6.94 (d, *J* = 8.6 Hz, 2H), 4.29 (s, 2H), 3.84 (s, 3H).

### Step 3: Methyl 5-hydroxy-1-(4-methoxybenzyl)-1H-1,2,3-triazole-4-carboxylate (04)

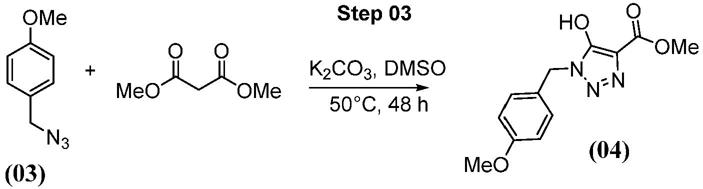

Dimethylmalonate (2.32 kg, 17.55 mol, 1.37 equiv.) and K₂CO₃ (7.11 kg, 51.44 mol, 4.0 equiv.) were added to a stirred solution of azide 03 (2.1 kg, 12.87 mol, 1.0 equiv.) in DMSO (13.65 L, 6.5 vol) at RT. The reaction mixture was stirred for 48.0 h at 50°C. The progress of the reaction was monitored by TLC (mobile phase: 40 % ethyl acetate in n-heptane). The reaction mixture was cooled to RT, diluted with water (21.0 L, 10 vol) then washed with MTBE (2 X 10.0 L). MTBE extracts were discarded. The aqueous layer was quenched with 4 N HCl (21.0 L, 10.0 Vol) below 10°C and extracted with EtOAc (2 x 20.0 L). The combined ethyl acetate extracts were washed with brine (3 x 20.0 L), dried over sodium sulphate. After filtration the filtrate was concentrated under *vacuum* at 50°C to obtain crude methyl 5-hydroxy-1-(4-methoxybenzyl)-1H-1,2,3-triazole-4-carboxylate (04) as a yellow oil (1.6 kg), which was used as such in the next step.

### Step 4: Methyl 5-chloro-1-(4-methoxybenzyl)-1H-1,2,3-triazole-4-carboxylate (05)

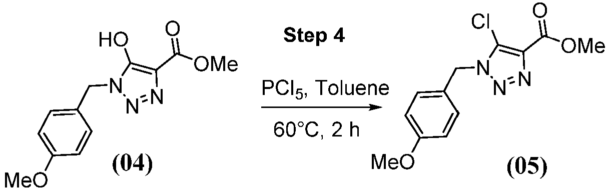

PCl₅ (1.51 kg, 7.25 mol, 1.2 equiv.) was added to a stirred solution of 04 (1.6 kg, 6.08 mol, 1.0 equiv.) in toluene (10.0 L, 6.25 vol) at RT. The reaction mixture was stirred for 2.0 h at 60°C. The progress of the reaction was monitored by TLC (mobile phase 40 % ethyl acetate in n-heptane). The reaction mixture was then poured into chilled aqueous ammonia (16.0 L, 10.0 vol) and ice (10.0 kg). The reaction mixture was extracted with ethyl acetate (2 x 20.0 L). The combined organic extract was washed with brine (16.0 L, 10.0 vol). After drying over sodium sulphate and filtration, the solvent was evaporated under *vacuum* at 50°C to provide crude methyl 5-chloro-1-(4-methoxybenzyl)-1H-1,2,3-triazole-4-carboxylate (05) as a brown semi solid (1.32 kg). The crude product 05 was used as such without further purification in step 5. ¹H NMR (300 MHz, Chloroform-*d*) δ 7.2-7.5 (m, 2H), 6.9-7.1 (m, 2H), 5.45 (s, 2H), 3.9 (s, 3H), 3.8 (s, 3H).

### Step 5: Methyl 5-(4-bromophenoxy)-1-(4-methoxybenzyl)-1H-1,2,3-triazole-4-carboxylate (VI-1a-1)

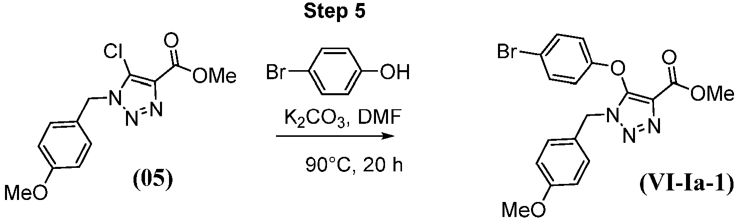

4-Bromophenol (0.798 kg, 4.615 mol, 1.0 equiv.) and K₂CO₃ (2.551 kg, 18.44 mol, 4.0 equiv.) were added to a stirred solution of crude 05 (1.3 kg, 4.615 mol, 1.0 equiv.) in DMF (7.8 L, 6.0 vol) at RT. The reaction mixture was stirred at 90°C for 20 h. The progress of the reaction was monitored by TLC (mobile phase 30 % ethyl acetate in n-heptane). The reaction mixture was cooled to RT, quenched with water (20.0 L, 15.3 vol) and then extracted with EtOAc (2 X 15.0 L). The combined organic extract was washed with water (2 x 10.0 L) and brine (3 X 10.0 L). After drying over sodium sulphate, it was then concentrated under *vacuum* at 50°C to obtain crude product of formula (VI-Ia-1). Crude product of formula (VI-Ia-1) was purified by column chromatography on silica gel (5.0 kg, mesh size 230-400) eluting with 0-30% EtOAc in n-heptane to provide pure compound of formula (VI-Ia-1) (650 g, 33.67%) as a light yellow solid. ¹H NMR (300 MHz, Chloroform-*d*) δ 7.35 (d, *J* = 9.0 Hz, 2H), 7.21 - 7.13 (m, 2H), 6.77 (d, *J* = 8.6 Hz, 2H), 6.61 (d, *J* = 9.0 Hz, 2H), 5.36 (s, 2H), 3.76 (s, 3H), 3.75 (s, 3H).

### Example 2: 1-Bromo-4-(3,3-difluorocyclobutyl)benzene

### Step A: 4-bromostyrene

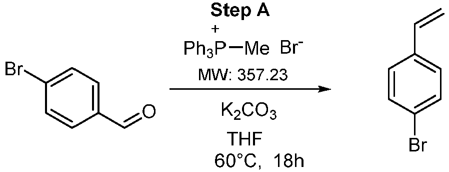

Methyltriphenylphosphoniumbromide (7.53 kg, 21.07 mol, 1.3 equiv.) and K₂CO₃ (13.44 kg, 97.27 mol, 6.0 equiv.) were added to a stirred solution ofp-bromobenzaldehyde (3.0 kg, 16.2 mol, 1.0 equiv.) in THF (45.0 L, 15.0 vol) at RT under a nitrogen atmosphere. The reaction mixture was stirred for 18 h at 60°C. The progress of the reaction was monitored by TLC (mobile phase 25 % EtOAc in n-hexane). The reaction mixture was cooled to RT, quenched with water (40.0 L, 13.33 vol) and extracted with MTBE (2 X 25.0 L). The organic layer was washed with 0.5 N HCl solutions (10 vol). Organic layer was collected, dried over sodium sulphate, filtered and the filtrate was evaporated to obtain 4-bromostyrene (2.33 kg, 78.57%) as a light-yellow liquid. ¹H NMR (300 MHz, Chloroform-d) δ 7.29 (d, *J* = 8.5 Hz, 2H), 7.09 (d, *J* = 8.5 Hz, 2H), 6.49 (dd, *J* = 17.6, 10.9 Hz, 1H), 5.58 (dd, *J* = 17.6, 0.8 Hz, 1H), 5.12 (dd, *J =* 10.9, 0.8 Hz, 1H).

### Step B: 3-(4-Bromophenyl) cyclobutan-1-one

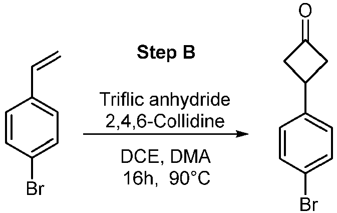

To a solution of dimethylacetamide (DMAc) (1.34 L, 14.45 mol, 1.1 equiv.) in 1,2-Dichloroethane (4.8 L, 2.0 vol) at -15°C was added a solution of Tf₂O (2.42 L, 14.42 mol, 1.1 equiv.) in 1,2-dichloroethane (0.72 L, 0.3 vol) and stirred at -15°C for 30 min. A solution of 4-bromostyrene (2.4 kg, 13.11 mol, 1.0 equiv.) in 1,2-dichloroethane (1.68 L, 0.7 vol) was added followed by the slow addition of a solution of 2,4,6-collidine (1.75 kg, 14.45 mol, 1.1 equiv.) in 1,2-dichloroethane (2.4 L, 1.0 vol). The reaction mixture was heated at 90°C for 16.0 h and then was allowed to cool to RT. The progress of the reaction was monitored by TLC (mobile phase 30% EtOAc in n-hexane). The reaction mixture was quenched with water (24.0 L, 10.0 vol) and the product was extracted with DCM (3 x 15.0 L). The combined organic extract was washed with brine (3 x 15.0 L) and concentrated. The crude product was purified by column chromatography on silica gel (7.0 kg, mesh size 230-400) eluting with 0-5 % ethyl acetate in *n*-heptane). The oily product was triturated with n-hexane at RT which gave solid product. The solid was collected by filtration to provide 3-(4-bromophenyl) cyclobutan-1-one (0.750 kg, 25.42 %) as an off white solid. ¹H NMR (300 MHz, Chloroform-*d*) δ 7.41 (d, *J =* 8.5 Hz, 2H), 7.16 - 7.07 (m, 2H), 3.64 - 3.51 (m, 1H), 3.50 - 3.38 (m, 2H), 3.22 - 3.07 (m, 2H).

### Step C: 1-Bromo-4-(3,3-difluorocyclobutyl)benzene

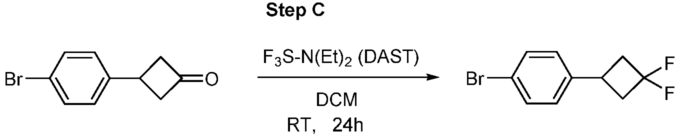

(Diethylamino) sulfur trifluoride (DAST) (1.074 kg, 6.66 mol, 2.5 equiv.) was added to a stirred solution of 3-(4-bromophenyl) cyclobutan-1-one (0.6 kg, 2.666 mol, 1.0 equiv.) in DCM (12.0 L, 20.0 vol) at -30°C. The reaction mixture was stirred for 24 h at RT *(26°C to 28°C*). The progress of the reaction was monitored by TLC (mobile phase 20% EtOAc in n-heptane). The reaction mixture was poured into 10% NaHCO₃ solution (12.0 L, 20.0 vol) and ice (5.0 kg) and extracted with DCM (2 x 6.0 L). The organic layers were collected and the solvent was evaporated to afford the crude product which was purified by column chromatography on silica gel (4.5 kg, mesh size 230-400) (mobile phase 0-5 % ethyl acetate and n-heptane) to obtain pure 1-bromo-4-(3,3-difluorocyclobutyl)benzene (362.0 g, 54.95%) as a yellow liquid. ¹H NMR (300 MHz, Chloroform-*d*) δ 7.36 - 7.28 (m, 2H), 7.02 - 6.95 (m, 2H), 3.29 - 3.14 (m, 1H), 2.97 - 2.78 (m, 2H), 2.62 - 2.40 (m, 2H).

### Example 3: Methyl 1-(4-methoxybenzyl)-5-(4-(4,4,5,5-tetramethyl-1,3,2-dioxaborolan-2-yl)phenoxy)-1H-1,2,3-triazole-4-carboxylate (IV-1a-2)

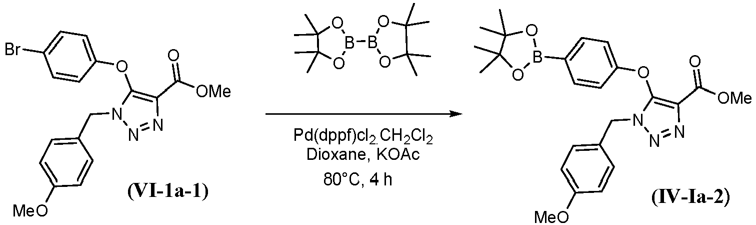

Pd(dppf)Cl₂.CH₂Cl₂ (65.85 g, 76.5 mmol, 0.04 equiv.), potassium acetate (563.14 g, 5.738 mol, 3.0 equiv.), bis(pinacolato)diboron (728.58 g, 2.869 mol, 1.5 equiv.) were added to a stirred solution of the compound of formula (VI-1a-1) (800.0 g, 1.912 mol, 1.0 equiv.) in 1,4-dioxane (12.0 L, 15.0 vol.) at room temperature. The reaction mixture was degassed and stirred at 80°C for 4.0 h. The progress of the reaction was monitored by TLC (mobile phase: 50 % ethyl acetate in n-heptane). The reaction mixture was cooled to room temperature then filtered through celite (1.0 kg) and the celite was washed with ethyl acetate (2.4 L, 3.0 vol). The filtrate was diluted with water (8.0 L, 10.0 vol) and extracted with EtOAc (2 x 6.0 L). The organic layer was washed with brine (8.0 L, 10.0 vol), after drying over sodium sulphate, the solvent was evaporated under reduced pressure at 50°C. The residue was triturated with n-heptane (4 x 3.0 L) to remove excess of bis(pinacolato)diboron and n-heptane was decanted. The residual amount of heptane was removed under vacuum to obtain compound of formula (IV-1a-2) (952.0 g) as reddish oily syrup. Crude material of formula (IV-1a-2) was used as such in the following step without further purification. ¹H NMR (300 MHz, DMSO-*d*₆) δ 7.66 - 7.58 (m, 2H), 7.22 - 7.11 (m, 2H), 6.94 - 6.79 (m, 4H), 5.42 (s, 2H), 3.70 (s, 3H), 3.60 (s, 3H), 1.29 (s, 12H).

### Example 4: Methyl 5-((4'-(3,3-difluorocyclobutyl)-[1,1'-biphenyl]-4-yl)oxy)-1-(4-methoxybenzyl)-1H-1,2,3-triazole-4-carboxylate (IIIa-1a-1)

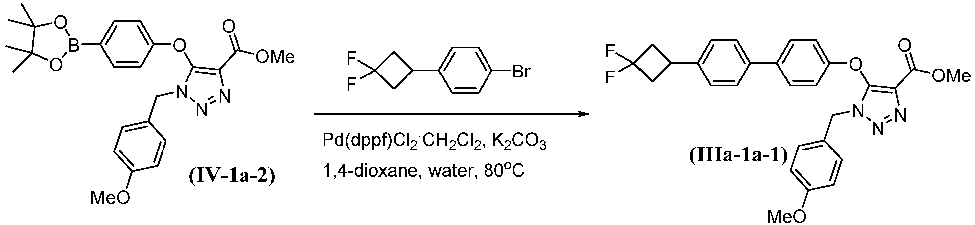

Pd(dppf)Cl₂·CH₂Cl₂ (123.02 g, 142.91 mmol, 0.07 eq.), K₂CO₃ (987.54 g, 7145.74 mmol, 3.5 eq.) in water (950.0 mL) were added to a stirred solution of the compound of formula (IV-1a-2) (950.0 g, 2041.64 mmol, 1.0 eq.) and 1-bromo-4-(3,3-difluorocyclobutyl)benzene (605.34 g, 2449.97 mmol, 1.2 eq.) in 1,4-dioxane (9.5 L) at room temperature. The reaction mixture was degassed and stirred at 80°C for 1 hour. The progress of the reaction was monitored by HPLC. The reaction mixture was cooled to room temperature then filtered through celite bed (1.0 kg) and the celite was washed with ethyl acetate (2.4 L, 3.0 vol). The filtrate was concentrated to provide crude product of formula (IIIa-1a-1). The crude product was then purified by column chromatography on silica gel (mobile phase 0-10 % ethyl acetate in DCM). The fractions containing the product were combined and the solvent was evaporated to provide the compound of formula (IIIa-1a-1) (600.0 g, 61.16 % for both steps as described in Examples 3-4) as a light brown solid. ¹H NMR (300 MHz, Chloroform-*d*) δ 7.57 - 7.41 (m, 4H), 7.37 - 7.18 (m, 4H), 6.88 - 6.76 (m, 4H), 5.39 (s, 2H), 3.77 (s, 3H), 3.75 (s, 3H), 3.56 - 3.35 (m, 1H), 3.16 - 2.95 (m, 2H), 2.86 - 2.58 (m, 2H).

### Example 5: Methyl 5-((4'-(3,3-difluorocyclobutyl)-[1,1'-biphenyl]-4-yl)oxy)-1H-1,2,3-triazole-4-carboxylate (IIa-1)

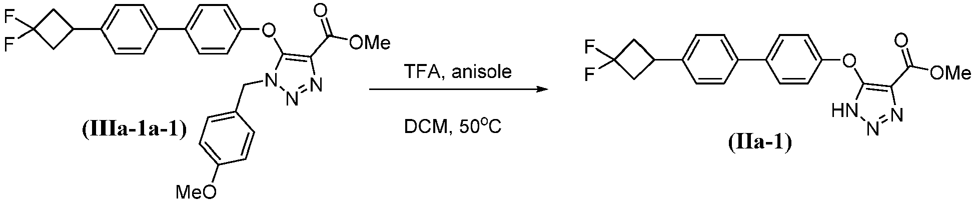

Anisole (385.01 g, 3.560 mol, 3.0 equiv.) and TFA (6.0 L, 10.0 vol) were added to a stirred solution of the compound of formula (IIIa-1a-1) (600.0 g, 1.186 mol, 1.0 equiv.) in DCM (6.0 L, 10.0 vol.) at room temperature. The reaction mixture was stirred for 15.0 h at 50°C. The progress of the reaction was monitored by TLC (mobile phase 50 % ethyl acetate in n-heptane). The solution was concentrated and the residue was treated with n-heptane (10.0 vol). The resulting solid was filtered and the wet solid was suspended in toluene (1.8 L, 3.0 vol). The resulting solid was collected by filtration to provide crude product of formula (IIa-1) as a light gray solid (362.2 g, 79.18 %), which was used in the next step without further purification. ¹H NMR (300 MHz, Chloroform-d) δ 7.63 - 7.49 (m, 4H), 7.33 - 7.27 (m, 4H), 3.96 (s, 3H), 3.51 - 3.34 (m, 1H), 3.15 - 2.93 (m, 2H), 2.84 - 2.56 (m, 2H).

### Example 6: 5-((4'-(3,3-Difluorocyclobutyl)-[1,1'-biphenyl]-4-yl)oxy)-1H-1,2,3-triazole-4-carboxylic acid (Ia-1)

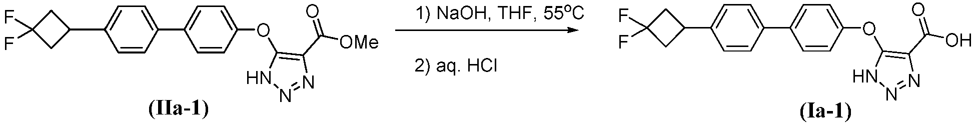

A solution of 1.0 M NaOH (115.83 g, 2.895.75 mmol, 3.1 eq.) in water (2.9 L) was added to a stirred solution of crude compound of formula (IIa-1) (360.0 g, 934.16 mmol, 1.0 eq.) in THF (1.8 L) at room temperature. The reaction mixture was stirred for 4.0 hours at 55°C. The progress of the reaction was monitored by TLC (mobile phase 80 % ethyl acetate in n-heptane). The reaction mixture was cooled to room temperature and washed with DCM (2 x 3.0 L). DCM layer was discarded and the aqueous layer was acidified with 1N HCl (3.0 L) (to pH 2 to 3) at room temperature. The precipitated solid was collected by filtration and wet cake was washed with water (3.6 L). Wet material (650.0 g) was dried at 45°C for 40 h to obtain the acid of formula (Ia-1) as an off white solid (320.0 g). ¹H NMR (300 MHz, DMSO-*d*₆) δ 7.72 - 7.56 (m, 4H), 7.39 (d, *J =* 8.1 Hz, 2H), 7.23 - 7.08 (m, 2H), 3.53 - 3.38 (m, 1H), 3.11 - 2.92 (m, 2H), 2.85 - 2.57 (m, 2H); MS (ES⁻): 370.4 (M-1); HPLC (area purity): 97.67%; and water content: 4.85%.

### Example 7: Sodium Salts of 5-((4'-(3,3-Difluorocyclobutyl)-[1,1'-biphenyl]-4-yl)oxy)-1H-1,2,3-triazole-4-carboxylic acid

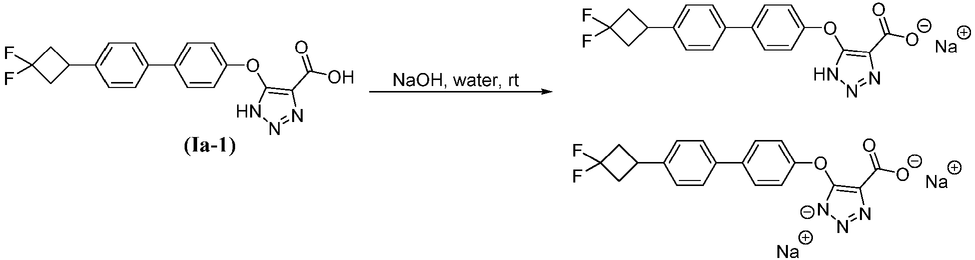

A solution of 1.0 M NaOH (1.12 g, 28.14 mmol, 0.95 eq.) in water (30.0 mL, 2.7 Vol.) was added to a stirred solution of *wet* compound of formula (Ia-1) (11.0 g, 29.62 mmol, 1.0 eq.) in water (55.0 mL) at room temperature. The reaction mixture was stirred for 1.0 hours (to pH 9.5). The reaction mixture was filtered to remove un-dissolved solid (2.5 g). The filtrate was lyophilized by freeze drying for 42.0 hours to obtain sodium salts (5.8 g) as an off white solid.

### Example 8: Mono-sodium salt of 5-((4'-(3,3-Difluorocyclobutyl)-[1,1'-biphenyl]-4-yl)oxy)-1H-1,2,3-triazole-4-carboxylic acid

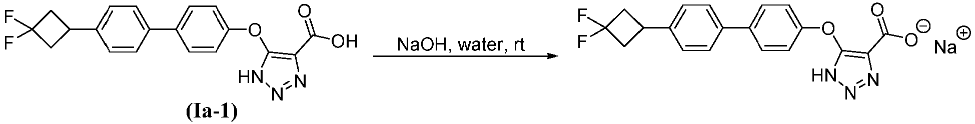

To a mechanically stirred (798 rpm) suspension of 5-((4'-(3,3-difluorocyclobutyl)-[1,1'-biphenyl]-4-yl)oxy)-1H-1,2,3-triazole-4-carboxylic acid (290.0 g, 1.00 eq, 746 mmol) (4.5% water content, which was adjusted accordingly for calculation) in water (870 mL) at room temperature was added 1N NaOH (609 mL) over a period of 10 min (pH = 9.69). The suspension was stirred for 2.5 h and pH was then measured to be 9.04. Additional 50 mL of 1N NaOH was added and pH then became 9.65. After stirring for additional 30 min, pH was 9.54 (a total of 0.883 eq. of 1 NaOH). The slurry was lyophilized to dryness to afford sodium 5-((4'-(3,3-difluorocyclobutyl)-[1,1'-biphenyl]-4-yl)oxy)-1H-1,2,3-triazole-4-carboxylate (283.5 g, 96.6 %) as an off-white solid. ¹H NMR (300 MHz, DMSO-*d*₆) δ 7.59 (dd, *J =* 8.6, 3.1 Hz, 4H), 7.38 (d, *J* = 8.1 Hz, 2H), 7.06 - 6.95 (m, 2H), 3.50 - 3.36 (m, 1H), 3.12 - 2.93 (m, 2H), 2.82 - 2.59 (m, 2H); MS (ES⁻): 370.2 (M-1); HPLC (area purity): 96.56%.

Elemental analysis of the product: carbon (C) found 57.26%; hydrogen (H) found 3.82%; nitrogen (N) found 10.87%; and sodium (Na) found 5.04%. The elemental analysis result was consistent with the mono-sodium salt of the compound of formula (Ia-1) represented by the formula:

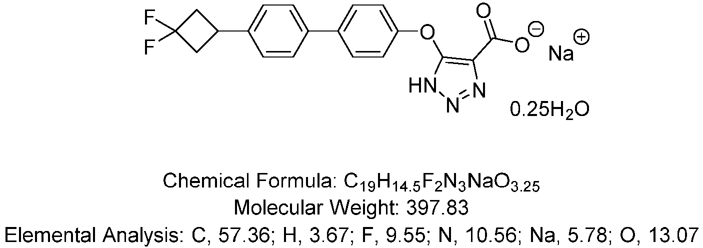

### Example 9: 5-((4'-(3,3-difluorocyclopentyl)-[1,1'-biphenyl]-4-yl)oxy)-1H-1,2,3-triazole-4-carboxylic acid (Ib-1)

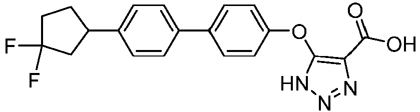

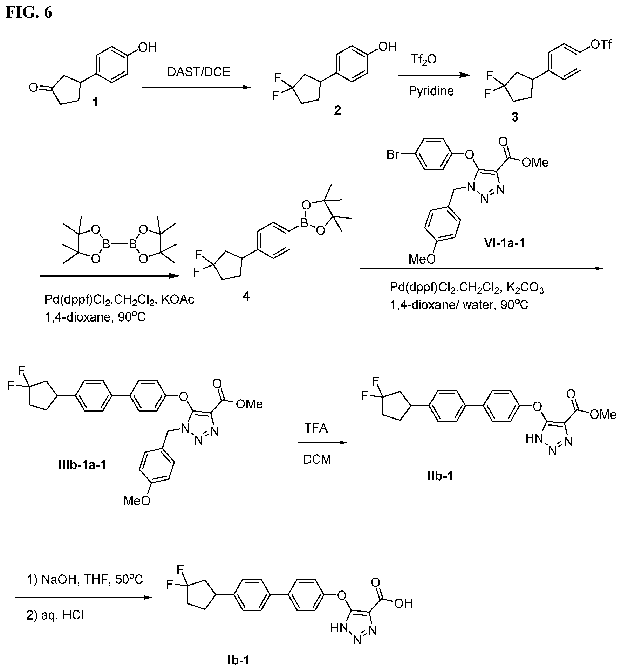

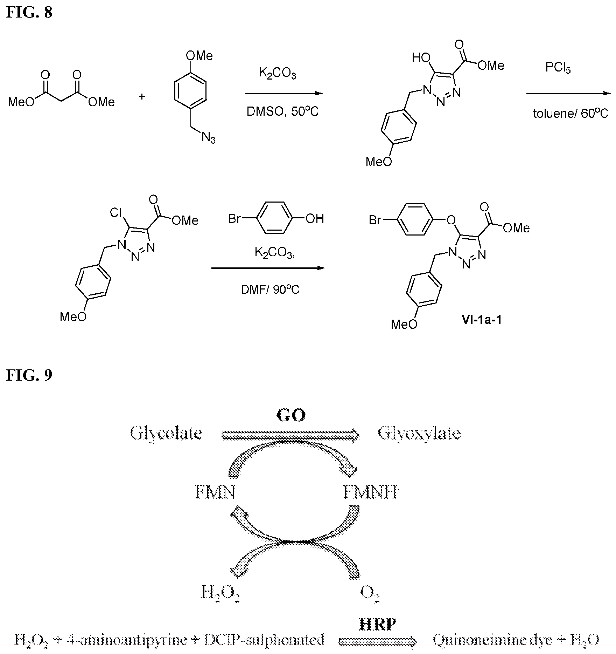

The compound of formula (Ib-1) was synthesized according to synthesis Scheme as shown in FIG. 6. In FIG. 6, compounds of formulae (Ib-1) and (IIb-1) can exist in tautomeric forms, as described herein. Compound 5 was prepared according to Scheme as shown in FIG. 8. The structure of compound 5 is determined according to J. Chem. Soc., Perkin Trans. 1, 1982, 627-630 and Humphrey J. Heterocyclic Chem 1991 301-304.

### Step 1: 4-(3,3-Difluorocyclopentyl)phenol (2)

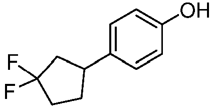

To a solution of 3-(4-hydroxyphenyl)cyclopentanone (**1**) (2.00 g, 11.3 mmol) in DCE (20 mL) was added DAST. After a period of four days the reaction mixture was diluted with DCM (20 mL) and added to a saturated solution of sodium bicarbonate. The organic phase was collected, dried over sodium sulfate, filtered and evaporated. The mixture was purified on combiflash using a silica gel (40 g) column with 0% to 25 % ethyl acetate-hexanes to afford the title compound (2) (1.20 g) (54 %). ¹H NMR (400 MHz, CDCl₃) δ 7.10 (d, *J* = 8.6 Hz, 2H), 6.79 (d, *J =* 8.5 Hz, 2H), 4.71 (s, 1H), 3.35 - 3.14 (m, 1H), 2.52 (m, 1H), 2.30 (m, 1H), 2.14 (m, 3H), 1.84 (m, 1H).

### Step 2: 4-(3,3-Difluorocyclopentyl)phenyl Trifluoromethanesulfonate (3)

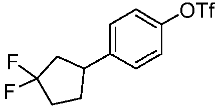

To a solution of 4-(3,3-difluorocyclopentyl)phenol (**2**) (0.200 g, 1.01 mmol) in DCM (4.0 mL), under nitrogen, was added pyridine (0.097 mL, 1.21 mmol, 1.20 eq). The reaction mixture was cooled at 0°C, then trifluoromethanesulfonic anhydride (Tf₂O) (0.255 mL, 1.51 mmol, 1.50 eq) was added. The reaction mixture was slowly brought to room temperature. After a period of two hours, the mixture was diluted with Et₂O and quenched with 1.0 M aqueous HCl. The organic layer was washed with saturated NaHCO₃, brine, dried over sodium sulfate, filtered and evaporated. The residue was purified on a silica gel (24 g) cartridge using 0% to 15 % EtOAc-hexanes to afford the title compound (0.240 g, 0.727 mmol) (72.0%) as a colorless oil. ¹H NMR (300 MHz, CDCl₃) δ 7.43 - 7.14 (m, 4H), 3.60 - 3.23 (m, 1H), 2.83 - 2.47 (m, 1H), 2.42 - 2.00 (m, 4H), 2.01 - 1.75 (m, 1H).

### Step 3: 2-(4-(3,3-Difluorocyclopentyl)phenyl)-4,4,5,5-tetramethyl-1,3,2-dioxaborolane (4)

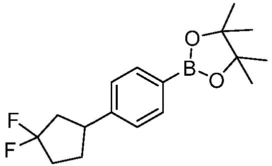

To a solution of 4-(3,3-difluorocyclopentyl)phenyl trifluoromethanesulfonate (**3**) (0.231 g, 0.70 mmol), bis(pinacolato)diboron (0.266 g, 1.05 mmol, 1.50 eq), in 1,4-dioxane (3.2 mL) was added potassium acetate (0.206 g, 2.10 mmol, 3.00 eq). The mixture was purged with argon gas for 5 min and was added Pd(dppf)Cl₂.CH₂Cl₂ (0.042 g, 0.049 mmol, 0.07 eq). The reaction mixture was heated at 90°C for 18 hrs. The mixture was cooled to room temperature, diluted with water (5 mL), extracted with EtOAc (2 X 20 mL). The combined organics were washed with water (200 mL), brine, dried and evaporated. The residue was purified on a silica gel (24 g) cartridge using 0% to 30% EtOAc-hexane to afford the title compound (0.240 g, 0.475 mmol) (67.8%) as a white solid. ¹H NMR (300 MHz, CDCl₃) δ 7.79 (d, *J =* 8.0 Hz, 2H), 7.27 (t, *J* = 8.0 Hz, 2H), 3.42 - 3.25 (m, 1H), 2.75 - 2.46 (m, 1H), 2.49 - 2.09 (m, 4H), 2.02 - 1.76 (m, 1H), 1.36 (s, 12H).

### Step 4: Methyl 5-((4'-(3,3-difluorocyclopentyl)-[1,1'-biphenyl]-4-yl)oxy)-1-(4-methoxybenzyl)-1H-1,2,3-triazole-4-carboxylate (IIIb-1a-1)

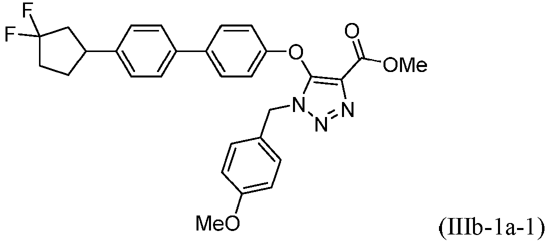

To a solution of methyl 5-(4-bromophenoxy)-1-(4-methoxybenzyl)-1H-1,2,3-triazole-4-carboxylate (**VI-1a-1**) (0.270 g, 0.646 mmol) and 2-(4-(3,3-difluorocyclopentyl)phenyl)-4,4,5,5-tetramethyl-1,3,2-dioxaborolane (**4**) (0.238 g, 0.775 mmol, 1.20 eq) in 1,4-dioxane (2.8 mL) were added potassium carbonate (0.312 g, 2.26 mmol, 3.50 eq) and water. After degassing the mixture with nitrogen, Pd(dppf)Cl₂.CH₂Cl₂ (0.033 g, 0.039 mmol, 0.060 eq) was added. The reaction mixture was heated at 85°C for 4 hrs. The mixture was filtered through a celite pad and washed with DCM. Water was added to filtrate and the two layers were separated. The organic layer was washed with brine, dried over sodium sulfate, filtered and concentrated. The residue was purified on a silica gel (24 g) column using 0% to 100% EtOAc-hexanes to afford the title compound, correspondingly (0.240 g, 0.462 mmol) (68 %) as a white solid. ¹H NMR (300 MHz, CDCl₃) δ 7.55 - 7.44 (m, 4H), 7.39 - 7.24 (m, 2H), 7.28 - 7.13 (m, 2H), 6.94 - 6.69 (m, 4H), 5.39 (s, 2H), 3.77 (s, 3H), 3.76 (s, 3H), 3.43 - 3.24 (m, 1H), 2.84 - 2.50 (m, 1H), 2.53 - 2.11 (m, 4H), 2.01 - 1.80 (m, 1H).

### Step 5: Methyl 5-((4'-(3,3-difluorocyclopentyl)-[1,1'-biphenyl]-4-yl)oxy)-1H-1,2,3-triazole-4-carboxylate (Ilb-1)

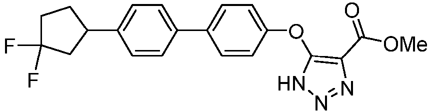

To a suspension of methyl 5-((4'-(3,3-difluorocyclopentyl)-[1,1'-biphenyl]-4-yl)oxy)-1-(4-methoxybenzyl)-1H-1,2,3-triazole-4-carboxylate (**IIIb-1a-1**) (0.213 g, 0.411 mmol) in DCM (2.0 mL) were added anisole (0.223 mL, 2.05 mmol, 5.00 eq) and TFA (5.0 mL, 65 mmol, 158 eq). The reaction mixture was stirred at 50°C for 4 hrs. The reaction mixture was then evaporated to dryness under reduced pressure to provide a solid. Hexane was added to the solid and triturated. The hexane was decanted followed by stirring overnight with EtOAc-Hexane to afford after filtration the title compound (0.106 g, 0.265 mmol) (64.6%) as a beige solid. ¹H NMR (300 MHz, CDCl₃) δ 7.57 (m, 4H), 7.37 - 7.23 (m, 4H), 3.98 (s, 3H), 3.50-3.24(m, 1H), 2.75 - 2.49 (m, 1H), 2.50 - 2.10 (m, 4H), 2.02-1.82 (m, 1H).

### Step 6: 5-((4'-(3,3-Difluorocyclopentyl)-[1,1'-biphenyl]-4-yl)oxy)-1H-1,2,3-triazole-4-carboxylic acid (Ib-1)

To a solution of methyl 5-((4'-(3,3-difluorocyclopentyl)-[1,1'-biphenyl]-4-yl)oxy)-1H-1,2,3-triazole-4-carboxylate **(IIb-1)** (0.106 g, 0.265 mmol, 1.00 eq) in THF (1.0 mL) was added a solution of 1 M NaOH, (0.796 mL, 0.796 mmol, 3.00 eq). The reaction mixture was stirred at 50°C for 6 hrs. The reaction mixture was acidified with a solution of 1 M HCl and the resulting solid was filtered and washed with. The solid was transfer to a flask and was dissolved in 10% water-EtOH (20 mL) heated to dissolve completely then water was added slowly until a white solid was crashed out. The solid was filtered and dried to provide the title compound (0.050 g, 0.130 mmol) (48.9%) as an off-white solid. ¹H NMR (300 MHz, CD₄OD) δ 7.60 (m, 4H), 7.36 (d, *J* = 8.1 Hz, 2H), 7.20 (d, *J* = 8.1 Hz, 2H), 3.59 - 3.36 (m, 1H), 2.70 - 2.44 (m, 1H), 2.42 - 2.08 (m, 4H), 2.03 - 1.77 (m, 1H); LRMS (ES-): 384.43 (M-H)⁻; and HPLC: t_{R} = 6.911, 97.7% purity).

### Example 10: 5-((4'-(4,4-difluorocyclohexyl)-[1,1'-biphenyl]-4-yl)oxy)-1H-1,2,3-triazole-4-carboxylic acid (Ic-1)

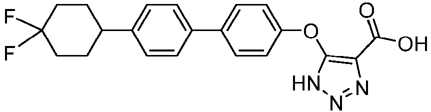

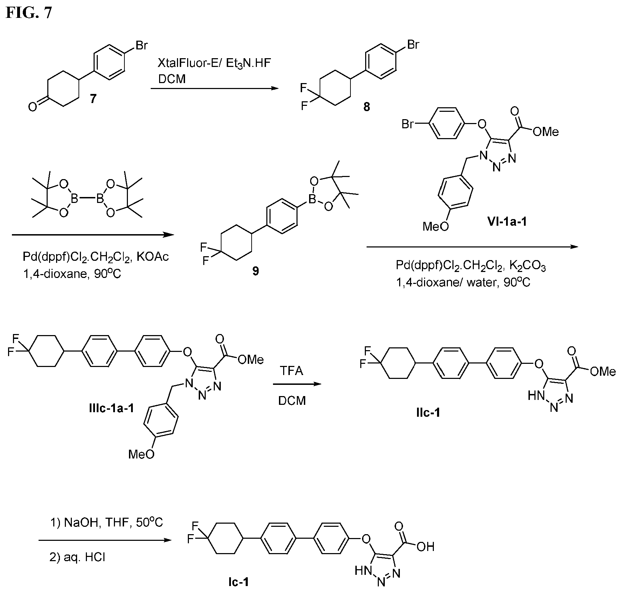

The compound of formula (Ic-1) was synthesized according to synthesis Scheme as shown in FIG. 7. In FIG. 7, compounds of formulae (Ic-1) and (IIc-1) can exist in tautomeric forms, as described herein. Compound 5 was prepared according to Scheme as shown in FIG. 8. The structure of compound 5 is determined according to J. Chem. Soc., Perkin Trans. 1, 1982, 627-630 and Humphrey J. Heterocyclic Chem 1991 301-304.

### Step 1: 1-Bromo-4-(4,4-difluorocyclohexyl)benzene (Vc-1-1)

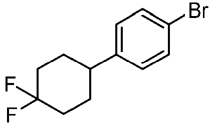

To a solution of triethylamine trihydrofluoride (1.30 mL, 7.90 mmol, 2.00 eq) and trimethylamine (0.549 mL, 3.95 mmol, 1.00 eq) in DCM (6.0 mL) at 0°C was added XtalFluor-E (*N,N*-Diethyl-*S,S*-difluorosulfiliminium tetrafluoroborate) (1.35 g, 5.92 mmol, 1.50 eq) and 4-(4-bromophenyl)cyclohexan-1-one (7) (1.00 g, 3.95 mmol, 1.00 eq) in DCM (3.0 mL). The mixture stirred at room temperature for 24 h. The reaction mixture was diluted with DCM (20 mL), then it was added to saturated aqueous sodium bicarbonate solution (50 mL) and resulting mixture was extracted with DCM (2 x 20 mL). The organic phases were combined and dried over Na₂SO₄ and filtered, solvent was evaporated and the resulting crude mixture was purified by silica gel column chromatography (40 g) using EtOAc/ hexanes (0 to 10%) to provide 1-bromo-4-(4,4-difluorocyclohexyl)benzene (1.03 g, 3.74 mmol) ( 94.8%) as a colorless oil (transparent solid) which became white solid on standing. ¹H NMR (300 MHz, CDCl₃) δ 7.48 - 7.39 (m, 2H), 7.14 - 7.06 (m, 2H), 2.64 - 2.50 (m, 1H), 2.30 - 2.14 (m, 2H), 2.02 - 1.68 (m, 6H).

### Step 2: 2-(4-(4,4-Difluorocyclohexyl)phenyl)-4,4,5,5-tetramethyl-1,3,2-dioxaborolane (9)

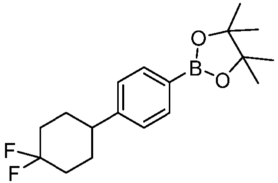

To a solution of 1-bromo-4-(4,4-difluorocyclohexyl)benzene (**Vc-1-1**) (1.12 g, 4.07 mmol), bis(pinacolato)diboron (1.54 g, 6.10 mmol, 1.50 eq), in 1,4-dioxane (19.0 mL) was added potassium acetate (1.20 g, 12.20 mmol, 3.00 eq). The mixture was purged with argon gas for 5 min, to this was added Pd(dppf)Cl₂.CH₂Cl₂ (0.245 g, 0.285 mmol, 0.070 eq). The reaction mixture was heated at 90°C for 4 hours. The mixture was cooled to room temperature, filtered through celite, washed with DCM. Water (50 mL) was added to the filtrate and extracted with DCM (2 x 25 mL). The combine organic layers were dried, concentrated to give crude residue which was purified on silica gel column chromatography (40 g) using EtOAc in hexanes (0-10%) to afford the title product (0.600 g, 1.86 mmol)( 45.8%) as a white solid. ¹H NMR (300 MHz, CDCl₃) δ 7.76 (d, *J* = 8.0 Hz, 2H), 7.23 (d, *J* = 8.2 Hz, 2H), 2.67 - 2.55 (m, 1H), 2.28 - 2.16 (m, 2H), 2.00 - 1.74 (m, 6H), 1.34 (s, 12H).

### Step 3: Methyl 5-((4'-(4,4-difluorocyclohexyl)-[1,1'-biphenyl]-4-yl)oxy)-1-(4-methoxybenzyl)-1H-1,2,3-triazole-4-carboxylate (IIIc-1a-1)

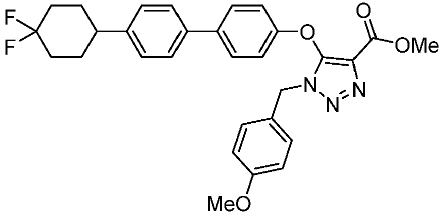

To a solution of methyl 5-(4-bromophenoxy)-1-(4-methoxybenzyl)-1H-1,2,3-triazole-4-carboxylate (**VI-1a-1**) (0.600 g, 1.43 mmol) and 2-(4-(4,4-difluorocyclohexyl)phenyl)-4,4,5,5-tetramethyl-1,3,2-dioxaborolane (**9**) (0.655 g, 1.75 mmol, 1.22 eq) in p-dioxane(6.3 mL) was added potassium carbonate (0.693 g, 5.02 mmol, 3.50 eq) and water (0.629 mL). After degassing the reaction mixture for 5 minutes, Pd(dppf)Cl₂.CH₂Cl₂ (0.074 g, 0.086 mmol, 0.060 eq) was added. The mixture was stirred at 90°C for 6 hrs. The reaction mixture was cooled to rt and filtered through celite, washed with DCM. Water (50 mL) was added to the mixture and extracted with DCM (2 x 25 mL). The combined organic layers were dried, concentrated under reduced pressure to give crude residue, which was purified on silica gel column chromatography (40 g) using EtOAc in DCM (0-10%) as eluent to give the title compound as a solid (0.620 g, 1.16 mmol)( 81 %). ¹H NMR (300 MHz, CDCl3) δ 7.51 - 7.45 (m, 4H), 7.30 (d, J = 8.2 Hz, 2H), 7.25 - 7.21 (m, 2H), 6.86 - 6.78 (m, 4H), 5.39 (s, 2H), 3.77 (s, 3H), 3.76 (s, 3H), 2.77 - 2.57 (m, 1H), 2.36 - 2.16 (m, 2H), 2.06 - 1.72 (m, 6H).

### Step 4: Methyl 5-((4'-(4,4-difluorocyclohexyl)-[1,1'-biphenyl]-4-yl)oxy)-1H-1,2,3-triazole-4-carboxylate (IIc-1)

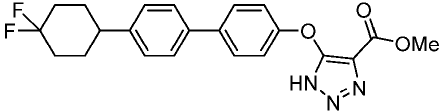

To a room temperature suspension of methyl 5-f[4'-(4,4-difluorocyclohexyl)-[1,1'-biphenyl]-4-yl]oxy}-1-[(4-methoxyphenyl)methyl]-1H-1,2,3-triazole-4-carboxylate (**IIIc-1a-1**) (0.675 g, 1.26 mmol) in DCM (2.2 mL) was added anisole (0.138 mL, 1.266 mmol, 1.000 eq) and TFA (7.6 mL, 99.33 mmol, 78.5 eq). After a period of 4 hrs at 50°C, the reaction mixture was evaporated to dryness under reduced pressure to provide a solid. Hexane (25 mL) was added to the solid and decanted. The resulting solid was triturated (stirred overnight) with EtOAc (100 mL), filtered and triturated (stirred for 30 minutes) with EtOH (10 mL) to give the title compound (0.260 g, 0.629 mmol) (49.7%). ¹H NMR (300 MHz, CDCl₃) δ 7.61 - 7.56 (m, 2H), 7.54 - 7.49 (m, 2H), 7.31 - 7.24 (m, 4H), 3.96 (s, 3H), 2.73 - 2.59 (m, 1H), 2.31 - 2.15 (m, 2H), 2.04 - 1.74 (m, 6H).

### Step 5: 5-((4'-(4,4-Difluorocyclohexyl)-[1,1'-biphenyl]-4-yl)oxy)-1H-1,2,3-triazole-4-carboxylic acid (Ic-1)

To a solution of methyl 5-{[4'-(4,4-difluorocyclohexyl)-[1,1'-biphenyl]-4-yl]oxy}-1H-1,2,3-triazole-4-carboxylate (**IIc-1**) (0.260 g, 0.553 mmol) in THF (1.0 mL) was added solution of NaOH (1 M) (1.7 mL, 1.72 mmol, 3.10 eq). The reaction mixture was stirred at 50°C for 6 hrs. The reaction mixture was acidified with 1.0 M HCl (pH = 3) and suspension was stirred for 10 minutes. The solid was filtered, rinsed with water and dried. The product was dissolved in hot mixture of 10% water in EtOH and after standing the title compound was obtained as a white solid (0.099 g, 0.248 mmol) (44.8%). ¹H NMR (300 MHz, DMSO-d₆) δ 7.18 (d, *J* = 8.2 Hz, 2H), 7.12 (d, *J* = 7.9 Hz, 2H), 6.88 (d, *J* = 7.7 Hz, 2H), 6.67 (d, *J* = 8.4 Hz, 2H), 2.30 - 2.26 (m, 1H), 1.71 - 1.55 (m, 3H), 1.55 - 1.37 (m, 3H), 1.35 - 1.15 (m, 2H); MS: ESI+ [M+] 399.89.

### Example 11: Inhibition of glycolate oxidase

The catalytic reactions used for assaying glycolate oxidase activity in the presence of compounds are outlined in **FIG. 9****.** Glycolate oxidase (GO)-catalyzed conversion of glycolate to glyoxylate (top reaction), with the concomitant reduction of the cofactor flavin mononucleotide (FMN), uses molecular oxygen (O₂) for recovering its oxidative state, releasing hydrogen peroxide (H₂O₂). The Trinder reaction (bottom reaction), in which horseradish peroxidase (HRP) uses hydrogen peroxide, 4-aminoantipyrine and a phenol derivative (sulphonated DCIP) to generate a quinoneimine dye that is spectrophotometrically measured.

**Human glycolate oxidase (hGO) expression:** BL21 (DE3) *E. coli* transformed with recombinant pET-15b expression vector with the N-terminal His-tag human Hao1 cDNA was grown in LB medium in the presence of 0.1 mg/ml ampicillin. For purification of recombinant human glycolate oxidase (hGO) expressed in BL21 *E. coli,* bacteria pellets were thawed and resuspended in 2 ml lysis buffer (50 mM NaH₂PO₄, 300 mM NaCl, 10 mM imidazole, 50 µM FMN, pH 7.5), and then treated for 30 minutes with 1 mM PMSF for protease inhibition, 0.1% Triton X-100 and 0.2 mg/ml lysozyme to break cellular membranes. After sonication, cells were centrifuged and the supernatant containing the total cellular extract (pre-column fraction) was loaded into a Ni-NTA agarose column and incubated for 30 minutes at 4°C to allow binding of the 6 histidine tail of recombinant GO protein to the nickel ions. The column was washed with two bed volumes of lysis buffer with 20 mM imidazole to eliminate unbound proteins (wash fraction). GO was eluted using the same buffer with 300 mM imidazole. Fraction containing purified GO was dialyzed against 300 ml of dialysis buffer (50 mM NaH₂PO₄, 300 mM NaCl, pH 7.5) at 4°C in agitation overnight, and then kept at 4°C in darkness. Protein were quantified by the bicinchoninic acid (BCA) assay.

**Enzymatic assays:** Enzymatic activity of hGO was determined in the presence of glycolate as substrate (40 mM glycolic acid) and phosphate buffer (50 mM KPO₄, 0.1 mM EDTA, pH 7). The production of glyoxylate was indirectly measured by the quantification of hydrogen peroxide formed during the first oxidation reaction. This hydrogen peroxide reacted with 4.9 mM 4-aminoantipyrine and 0.1 mM sulphonated 2,4-dichlorophenolindophenol in a coupled horseradish peroxidase (HRP) reaction that yields a quinoneimine dye (FIG. 3) measured at 515 nm (Trinder reaction). Enzymatic activity was calculated at 1 minute after initiation of the Trinder reaction. Results of the enzymatic assay for the compounds are shown in Table 2.

**Table 2: In vitro inhibition of human glycolate oxidase by compounds**

| **Formula** | **% Inhibition** |
|---|---|
| **Ib-1** | +++ |
| **Ic-1** | +++ |
| + = IC50≥200 nM | |
| ++ = 100 nM≥IC50 ≤ 200 nM | |
| +++ = IC50< 100 nM | |

Although the foregoing disclosure has been described in some detail by way of illustration and example for purposes of clarity of understanding, one of skill in the art will appreciate that certain changes and modifications may be practiced within the scope of the appended claims. Where a conflict exists between the instant application and a reference provided herein, the instant application shall dominate.

## Claims

1. A process for preparing a compound represented by formula (II): 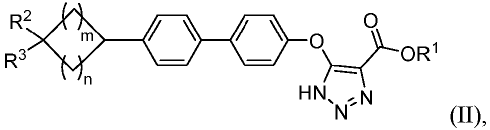 a tautomer thereof, or a salt thereof, comprising:
a) contacting a compound of formula (IV): 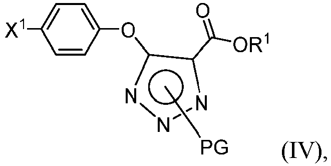 or a salt thereof, with a compound of formula (V): 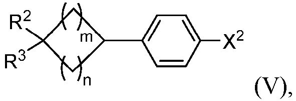 a first transition-metal catalyst, and a first base in a first solvent to form a compound of
formula (III): 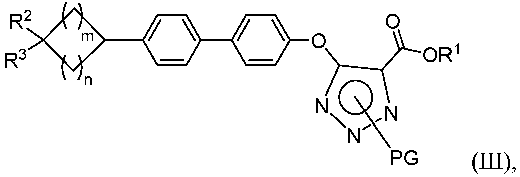 or a salt thereof; and
b) removing the PG group of the compound of formula (III) or the salt thereof to provide the compound of formula (II), the tautomer thereof, or the salt thereof,
wherein:
subscripts m and n are each independently 1 or 2;
R¹ is C₁₋₆ alkyl;
R² and R³ are each independently H or halogen;
X¹ represents a boron-containing group;
X² is halogen or a sulfonate; 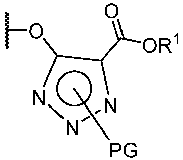 in formula (IV) or (III) is represented by the formula: 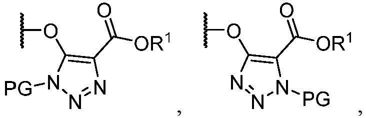
or a mixture thereof; and
PG is an amine-protecting group.

2. The process of claim 1, wherein:
(i) the amine-protecting group is [2-(trimethylsilyl)ethoxy]methyl, 4-methoxybenzyl, or 2,4-dimethoxybenzyl, preferably wherein the amine-protecting group is 4-methoxybenzyl; and/or
(ii) X¹ is represented by the formula:
1) -BY₂, wherein Y is -OH, C₁₋₆ alkyl, C₁₋₆ alkoxy, C₆₋₁₀ aryloxy, or a carboxylate group;
2) -BY, wherein Y is a bidentate C₂₋₈ alkoxy group, a bidentate C₆₋₁₀ aryloxy group, or
a bidentate carboxylate group;
3) a 9-borabicyclo[3,3,1]nonane (9-BBN) group;
4) -BY₃M, wherein Y is F or C₁₋₆ alkoxy and M is an alkaline metal ion, an ammonium ion, or a phosphonium ion; or
5) -BYM, wherein Y is a tridentate C₃₋₁₀ alkoxy group and M is an alkaline metal ion, an ammonium ion, or a phosphonium ion,
preferably wherein X¹ is -B(OH)₂, -B(OEt)₂, -B(O*i*Pr)₂,
-BF₃M, -B(O*i*Pr)₃M, -B(O*i*Pr)₃M, 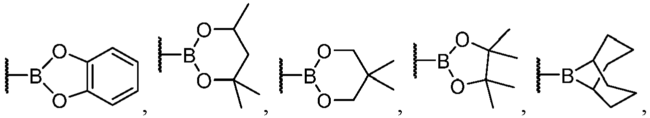 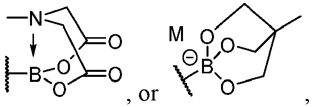 wherein M is Li⁺, Na⁺, or K⁺,
more preferably wherein X¹ is represented by the formula: 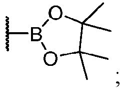 and/or
(iii) X² is Cl, Br, I, OMs, OTs, or OTf, preferably wherein X² is Br; and/or
(iv) the first transition-metal catalyst is a first palladium catalyst, a ruthenium catalyst, a rhodium catalyst, a cobalt catalyst, a nickel catalyst, an iron catalyst, a copper catalyst, or a combination thereof,
preferably wherein the first palladium catalyst is Pd(acac)₂, [Pd(allyl)Cl]₂, Pd(CH₃CN)₂Cl₂, Pd(dba)₂, Pd(CH₃COO)₂, Pd₂(dba)₃, Pd₂(dba)₃·CHCl₃, Pd(PPh₃)₄, Pd(OAc)₂, Pd(PCy₃)₂Cl₂, Pd(PPh₃)₂Cl₂, Pd[P(o-tol)₃]₂Cl₂, Pd(amphos)Cl₂, Pd(dppf)Cl₂, Pd(dppf)Cl₂·CH₂Cl₂, Pd(dtbpf)Cl₂, Pd(CH₃CN)₄(BF₄)₂, PdCl₂, XPhos-Pd-G3, Pd-PEPPSI^{™}-IPr, Pd-PEPPSI^{™}-SIPr, or Pd-PEPPSI^{™}-IPent,
more preferably wherein the first palladium catalyst is Pd(dppf)Cl₂, Pd(dppf)Cl₂·CH₂Cl₂, or Pd(dtbpf)Cl₂; and/or
(v) the first transition-metal catalyst is in a substoichiometric amount; and/or
(vi) the first base is sodium carbonate, potassium carbonate, cesium carbonate, sodium phosphate tribasic, potassium phosphate tribasic, sodium acetate, potassium acetate, cesium acetate, or a combination thereof, preferably wherein the first base is potassium carbonate; and/or
(vii) the compound of formula (V) is in an amount of from 1.0 to 2.0 equivalents, from 1.1 to 2.0 equivalents, from 1.1 to 1.5 equivalents, or 1.2 equivalents, relative to the compound of formula (IV), preferably wherein the compound of formula (V) is in an amount of 1.2 equivalents relative to the compound of formula (IV); and/or
(viii) the first solvent is water, C₁₋₄ alcohol, benzene, toluene, dioxane, tetrahydrofuran (THF), 2-methyl-tetrahydrofuran (MeTHF), acetonitrile (ACN), N-methylpyrrolidone (NMP), N,N-dimethylformamide (DMF), N,N-dimethylacetamide (DMAC), dimethoxyethane (DME), ethylene glycol, or a combination thereof, preferably wherein the first solvent comprises dioxane and water.

3. The process of claim 1 or 2, wherein step a) is conducted at a temperature of from 60°C to 110°C, from 70°C to 110°C, from 70°C to 100°C, from 70°C to 90°C, or 80°C, preferably wherein step a) is conducted at a temperature of 80°C.

4. The process of any of claims 1 to 3, wherein in step b), the PG group is removed by treating with a first acid in a second solvent, preferably wherein the first acid is trifluoroacetic acid, and optionally wherein:
- the second solvent is dichloromethane or 1,2-dichloroethane, and/or
- wherein a reaction mixture of step b) further comprises a transferring agent, preferably wherein the transferring agent is anisole.

5. The process of any of claims **1** to **4,** further comprising:
c) contacting the compound of formula (II), the tautomer thereof, or the salt thereof with a second base in a third solvent; and
d) acidifying with a second acid to provide a compound of formula (I): 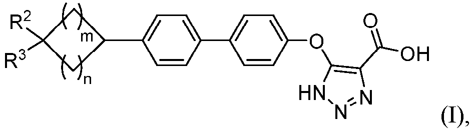 a tautomer thereof, or a salt thereof.

6. The process of claim **5,** wherein:
(i) the second base is lithium hydroxide, sodium hydroxide, or potassium hydroxide, preferably wherein the second base is sodium hydroxide; and/or
(ii) the third solvent is water, C₁₋₄ alcohol, dioxane, tetrahydrofuran (THF), 2-methyl-tetrahydrofuran (MeTHF), acetonitrile (ACN), dimethoxyethane (DME), or a combination thereof, preferably wherein the third solvent comprises tetrahydrofuran and water; and/or
(iii) the second acid is HCl; and/or
(iv) the step d) is conducted in an aqueous solution.

7. The process of any one of claims **1** to **6,** wherein:
(i) the compound of formula (II) is represented by the formula selected from the group consisting of: 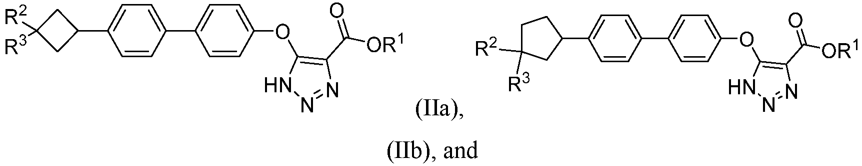 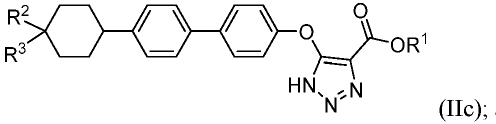 and/or
(ii) R¹ is methyl; and/or
(iii) R² and R³ are each independently halogen, preferably wherein R² and R³ are each F.

8. The process of claim 7, wherein the compound of formula (II) is represented by the formula:

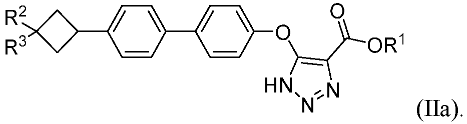

9. The process of any one of claims **5** to **8,** wherein the compound of formula (I) is represented by the formula selected from the group consisting of:

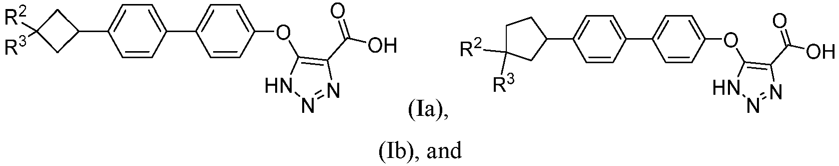

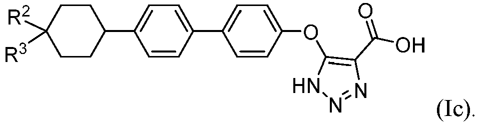

10. The process of claim 9, wherein the compound of formula (I) is represented by the formula:

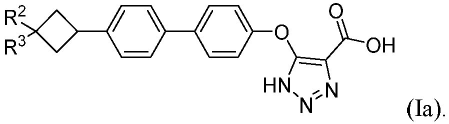

11. The process of any one of claims 1 to 10, further comprising prior to step a):
a1) contacting a compound of formula (VI): 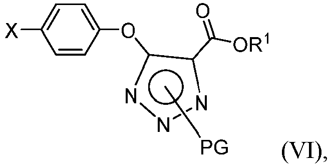 or a salt thereof, with a boron reagent, a second palladium catalyst, and a third base in a fourth solvent to form the compound of formula (IV) or the salt thereof, wherein X is Cl, Br, or I.

12. The process of claim **11,** wherein:
(i) the boron reagent is tetrahydroxydiboron, bis(catecholato)diboron, bis(hexylene glycolato)diboron, bis(neopentyl glycolato)diboron, or bis(pinacolato)diboron, preferably wherein the boron reagent is bis(pinacolato)diboron; and/or
(ii) the second palladium catalyst is Pd(dppf)Cl₂, Pd(dppf)Cl₂·CH₂Cl₂, or Pd(dtbpf)Cl₂; and/or
(iii) the third base is potassium acetate; and/or
(iv) the fourth solvent is 1,4-dioxane; and/or
(v) the compound of formula (VI) is represented by the formula:

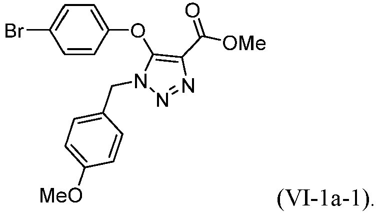

13. A process according to claim **1** for preparing a compound represented by the formula (Ia-1): 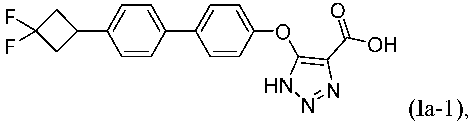 a tautomer thereof, or a salt thereof, comprising:
a1) converting a compound of formula (VI-1a-1): 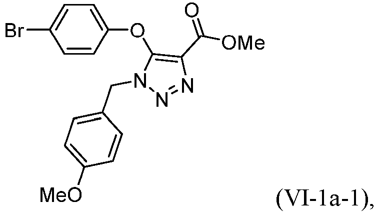 or a salt thereof, with bis(pinacolato)diboron, Pd(dppf)Cl₂·CH₂Cl₂, and
potassium acetate in 1,4-dioxane to a compound of formula (IV-1a-2): 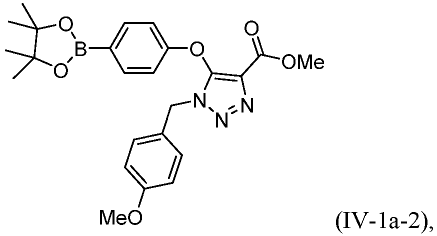 or a salt thereof;
a) contacting the compound of formula (IV-1a-2) or the salt thereof with 1-bromo-4-(3,3-difluorocyclobutyl)benzene, Pd(dppf)Cl₂·CH₂Cl₂, and potassium carbonate in a mixture of 1,4-dioxane and water to form a compound of
formula (IIIa-1a-1): 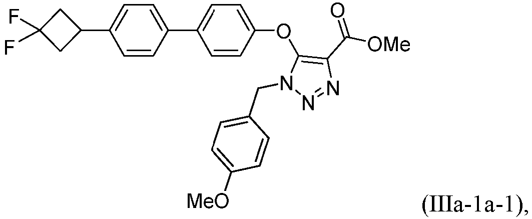 or a salt thereof;
b) treating the compound of formula (IIIa-1a-1) or the salt thereof, with trifluoroacetic acid and anisole in dichloromethane to provide a compound of formula (IIa-1): 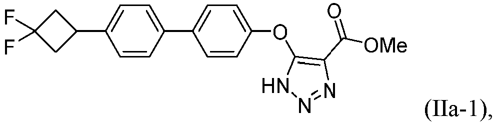 a tautomer thereof, or a salt thereof;
c) saponifying the compound of formula (IIa-1), the tautomer thereof, or the salt thereof with an aqueous solution of sodium hydroxide in tetrahydrofuran; and
d) acidifying with an aqueous solution of HCl to provide the compound of formula (Ia-1), the tautomer thereof, or the salt thereof.

14. The process of claim **13,** wherein:
(1) step a1) is conducted at a temperature of 80°C; step a) is conducted at a temperature of 80°C; step b) is conducted at a temperature of 50°C; and step c) is conducted at a temperature of 55°C; and step d) is conducted at a temperature of 20-25°C; and/or
(2) in step a), 1-bromo-4-(3,3-difluorocyclobutyl)benzene is in an amount of from 1.0 to 1.5 equivalents relative to the compound of formula (IV-1a-2); and/or
(3) in step a), Pd(dppf)Cl₂·CH₂Cl₂ is in an amount of from 0.05 to 0.1 equivalent relative to the compound of formula (IV-1a-2); and/or
(4) in step a), potassium carbonate is in an amount of from 2.0 to 4.0 equivalents relative to the compound of formula (IV-1a-2); and/or
(5) the compound of formula (IV-1a-2) produced by step a1) is directly used in the next step without further purification; and/or
(6) the compound of formula (IIIa-1) is isolated in a combined yield of at least 50% from both steps a1) and a); and/or
(7) the compound of formula (IIa-1) produced by step b) is directly used in next step without further purification; and/or
(8) step d) is conducted by acidifying an aqueous extract of a reaction mixture of step c).

15. The process of claim 14, wherein the process further comprises:
e) converting the compound of formula (Ia-1), the tautomer thereof, or the salt thereof, to a mono-sodium salt of the compound of formula (Ia-1) represented by the formula: 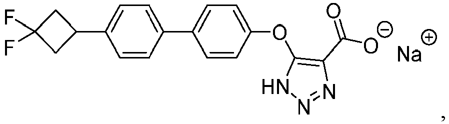 a di-sodium salt of the compound of formula (Ia-1) represented by the formula: 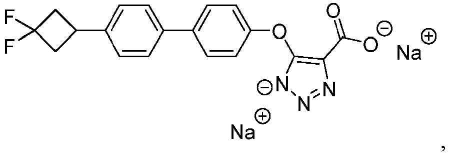 or a mixture thereof, or a tautomer thereof;
preferably wherein step e) is conducted by:
i) treating the compound of formula (Ia-1) or a tautomer thereof with an aqueous solution of sodium hydroxide in water;
ii) forming a slurry having a pH value of 9.5; and
iii) lyophilizing the slurry to provide the mono-sodium salt of the compound of formula (Ia-1) represented by the formula: 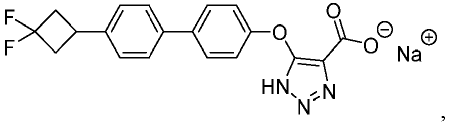 or a tautomer thereof,
wherein sodium hydroxide is in an amount of less than 1.0 equivalent relative to the compound of formula (Ia-1), on a salt-free and anhydrous basis, preferably wherein sodium hydroxide is in an amount of 0.88 equivalent relative to the compound of formula (Ia-1), on a salt-free and anhydrous basis.

## Patentansprüche

1. Verfahren zum Herstellen einer Verbindung, die durch Formel (II) dargestellt wird: 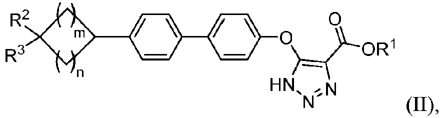 eines Tautomeres davon oder eines Salzes davon, umfassend:
a) Inberührungbringen einer Verbindung der Formel (IV): 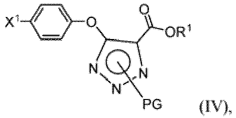 oder eines Salzes davon mit einer Verbindung der Formel (V): 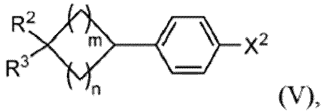 eines ersten Übergangsmetallkatalysators und einer ersten Basis in einem ersten Lösungsmittel, um eine Verbindung der Formel (III) auszubilden: 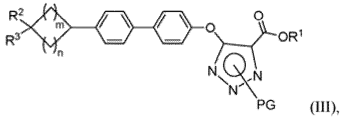 oder eines Salzes davon; und
b) Entfernen der PG-Gruppe der Verbindung der Formel (III) oder des Salzes davon, um die Verbindung der Formel (II), des Tautomeres davon oder des Salzes davon bereitzustellen,
wobei:
die Indizes m und n jeweils unabhängig voneinander 1 oder 2 sind;
R¹ C₁₋₆-Alkyl ist;
R² und R³ jeweils unabhängig voneinander H oder Halogen sind;
X¹ eine borhaltige Gruppe darstellt;
X² Halogen oder ein Sulfonat ist; 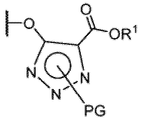 in Formel (IV) oder (III) durch die Formeln: 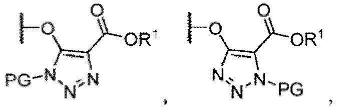
oder eine Mischung davon dargestellt wird; und
PG eine Aminschutzgruppe ist.

2. Verfahren nach Anspruch 1, wobei:
(i) die Aminschutzgruppe [2-(Trimethylsilyl)ethoxy]methyl, 4-Methoxybenzyl oder 2,4-Dimethoxybenzyl ist, vorzugsweise wobei die Aminschutzgruppe 4-Methoxybenzyl ist; und/oder
(ii) X¹ durch die Formel dargestellt wird:
1) -BY₂, wobei Y -OH, C₁₋₆-Alkyl, C₁₋₆-Alkoxy, C₆₋₁₀-Aryloxy oder eine Carboxylatgruppe ist;
2) -BY, wobei Y eine zweizahnige C₂₋₈-Alkoxygruppe, eine zweizahnige C₆₋₁₀-Aryloxygruppe oder
eine zweizahnige Carboxylatgruppe ist;
3) eine 9-Borabicyclo[3,3,1]nonan(9-BBN)-Gruppe ist;
4) -BY₃M, wobei Y F oder C₁₋₆-Alkoxy ist und M ein Alkalimetallion, ein Ammoniumion oder ein Phosphoniumion ist; oder
5) -BYM, wobei Y eine dreizahnige C₃₋₁₀-Alkoxygruppe ist und M ein Alkalimetallion, ein Ammoniumion oder ein Phosphoniumion ist,
vorzugsweise wobei X¹ -B(OH)₂, -B(OEt)₂, -B(O*i*Pr)₂, -BF₃M, -B(O*i*Pr)₃M, - B(O*i*Pr)₃M, 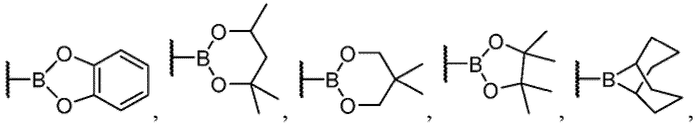 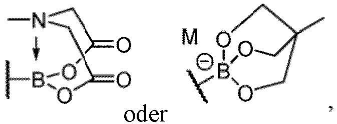 ist, wobei M Li⁺, Na⁺ oder K⁺ ist,
stärker bevorzugt wobei X¹ durch die Formel dargestellt wird: 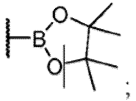 und/oder
(iii) X² Cl, Br, I, OMs, OTs oder OTf ist, vorzugsweise wobei X² Br ist; und/oder
(iv) der erste Übergangsmetallkatalysator ein erster Palladiumkatalysator, ein Rutheniumkatalysator, ein Rhodiumkatalysator, ein Kobaltkatalysator, ein Nickelkatalysator, ein Eisenkatalysator, ein Kupferkatalysator oder eine Kombination davon ist,
vorzugsweise wobei der erste Palladiumkatalysator Pd(acac)₂, [Pd(Allyl)Cl]₂, Pd(CH₃CN)₂Cl₂, Pd(dba)₂, Pd(CH₃COO)₂, Pd₂(dba)₃, Pd₂(dba)₃·CHCl₃, Pd(PPh₃)₄, Pd(OAc)₂, Pd(PCy₃)₂Cl₂, Pd(PPh₃)₂Cl₂, Pd[P(o-tol)₃]₂Cl₂, Pd(Amphos)Cl₂, Pd(dppf)Cl₂, Pd(dppf)Cl₂·CH₂Cl₂, Pd(dtbpf)Cl₂, Pd(CH₃CN)₄(BF₄)₂, PdCl₂, XPhos-Pd-G3, Pd-PEPPSI^{™}-IPr, Pd-PEPPSI^{™}-SIPr, oder Pd-PEPPSI^{™}-IPent ist,
stärker bevorzugt wobei der erste Palladiumkatalysator Pd(dppf)Cl₂, Pd(dppf)Cl₂·CH₂Cl₂ oder Pd(dtbpf)Cl₂ ist; und/oder
(v) der erste Übergangsmetallkatalysator in einer substöchiometrischen Menge vorliegt; und/oder
(vi) die erste Base Natriumcarbonat, Kaliumcarbonat, Cäsiumcarbonat, tribasisches Natriumphosphat, tribasisches Kaliumphosphat, Natriumacetat, Kaliumacetat, Cäsiumacetat oder eine Kombination davon ist, vorzugsweise wobei die erste Base Kaliumcarbonat ist; und/oder
(vii) die Verbindung der Formel (V) in einer Menge von 1,0 bis 2,0 Äquivalenten, von 1,1 bis 2,0 Äquivalenten, von 1,1 bis 1,5 Äquivalenten oder 1,2 Äquivalenten bezogen auf die Verbindung der Formel (IV) vorliegt, vorzugsweise wobei die Verbindung der Formel (V) in einer Menge von 1,2 Äquivalenten bezogen auf die Verbindung der Formel (IV) vorliegt; und/oder
(viii) das erste Lösungsmittel Wasser, C₁₋₄-Alkohol, Benzol, Toluol, Dioxan, Tetrahydrofuran (THF), 2-Methyltetrahydrofuran (MeTHF), Acetonitril (ACN), N-Methylpyrrolidon (NMP), N,N-Dimethylformamid (DMF), N,N-Dimethylacetamid (DMAC), Dimethoxyethan (DME), Ethylenglykol oder eine Kombination davon ist, vorzugsweise wobei das erste Lösungsmittel Dioxan und Wasser umfasst.

3. Verfahren nach Anspruch 1 oder 2, wobei Schritt a) bei einer Temperatur von 60 °C bis 110 °C, von 70 °C bis 110 °C, von 70 °C bis 100 °C, von 70 °C bis 90 °C oder 80 °C durchgeführt wird, vorzugsweise wobei Schritt a) bei einer Temperatur von 80 °C durchgeführt wird.

4. Verfahren nach einem der Ansprüche 1 bis 3, wobei in Schritt b) die PG-Gruppe durch Behandeln mit einer ersten Säure in einem zweiten Lösungsmittel entfernt wird, vorzugsweise wobei die erste Säure Trifluoressigsäure ist, und optional wobei:
- das zweite Lösungsmittel Dichlormethan oder 1,2-Dichlorethan ist und/oder
- wobei eine Reaktionsmischung von Schritt b) ferner ein Übertragungsmittel umfasst, vorzugsweise wobei das Übertragungsmittel Anisol ist.

5. Verfahren nach einem der Ansprüche 1 bis 4, das ferner umfasst:
c) Inberührungbringen der Verbindung der Formel (II), des Tautomeres davon oder des Salzes davon mit einer zweiten Base in einem dritten Lösungsmittel; und
d) Ansäuern mit einer zweiten Säure, um eine Verbindung der Formel (I): 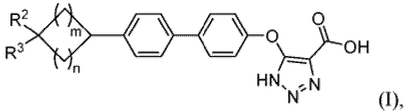 ein Tautomer davon oder ein Salz davon bereitzustellen.

6. Verfahren nach Anspruch 5, wobei:
(i) die zweite Base Lithiumhydroxid, Natriumhydroxid oder Kaliumhydroxid ist, vorzugsweise wobei die zweite Base Natriumhydroxid ist; und/oder
(ii) das dritte Lösungsmittel Wasser, C₁₋₄-Alkohol, Dioxan, Tetrahydrofuran (THF), 2-Methyltetrahydrofuran (MeTHF), Acetonitril (ACN), Dimethoxyethan (DME) oder eine Kombination davon ist, vorzugsweise wobei das dritte Lösungsmittel Tetrahydrofuran und Wasser umfasst; und/oder
(iii) die zweite Säure HCl ist; und/oder
(iv) der Schritt d) in einer wässrigen Lösung durchgeführt wird.

7. Verfahren nach einem der Ansprüche 1 bis 6, wobei:
(i) die Verbindung der Formel (II) durch die Formel dargestellt wird, die aus der Gruppe ausgewählt ist, bestehend aus: 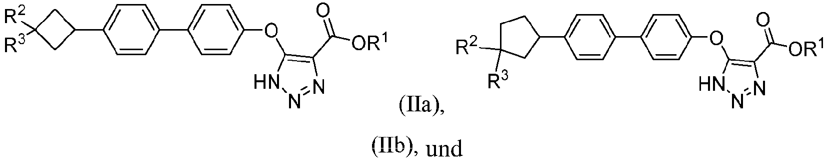 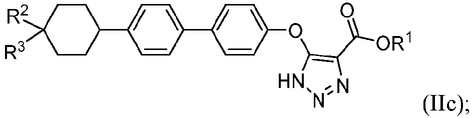 und/oder
(ii) R¹ Methyl ist; und/oder
(iii) R² und R³ jeweils unabhängig voneinander Halogen sind, vorzugsweise wobei R² und R³ jeweils F sind.

8. Verfahren nach Anspruch 7, wobei die Verbindung der Formel (II) durch die Formel dargestellt wird:

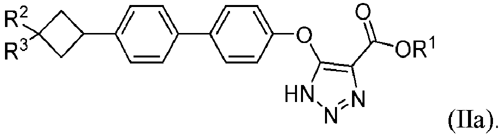

9. Verfahren nach einem der Ansprüche 5 bis 8, wobei die Verbindung der Formel (I) durch die Formel dargestellt wird, ausgewählt aus der Gruppe, bestehend aus:

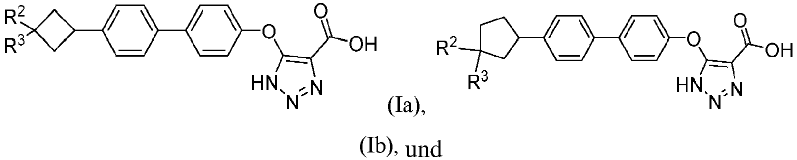

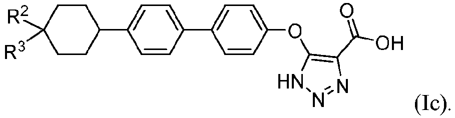

10. Verfahren nach Anspruch 9, wobei die Verbindung der Formel (I) durch die Formel dargestellt wird:

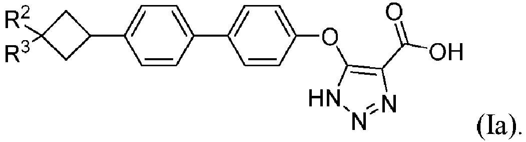

11. Verfahren nach einem der Ansprüche 1 bis 10, ferner umfassend vor Schritt a):
a1) Inberührungbringen einer Verbindung der Formel (IV): 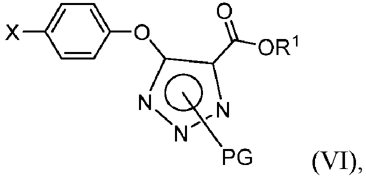
oder eines Salzes davon mit einem Borreagenz, einem zweiten Palladiumkatalysator und einer dritten Base in einem vierten Lösungsmittel, um die Verbindung der Formel (IV) oder das Salz davon auszubilden, wobei X Cl, Br oder I ist.

12. Verfahren nach Anspruch 11, wobei:
(i) das Borreagenz Tetrahydroxydibor, Bis(catecholato)dibor, Bis(hexylenglycolato)dibor, Bis(neopentylglycolato)dibor oder Bis(pinacolato)dibor ist, vorzugsweise wobei das Borreagenz Bis(pinacolato)dibor ist; und/oder
(ii) der zweite Palladiumkatalysator Pd(dppf)Cl₂, Pd(dppf)Cl₂·CH₂Cl₂ oder Pd(dtbpf)Cl₂ ist; und/oder
(iii) die dritte Base Kaliumacetat ist; und/oder
(iv) das vierte Lösungsmittel 1,4-Dioxan ist; und/oder
(v) die Verbindung der Formel (VI) durch die Formel dargestellt wird:

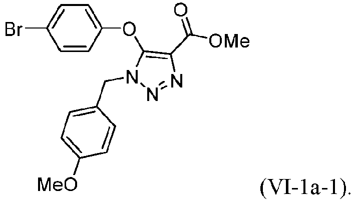

13. Verfahren nach Anspruch 1 zum Herstellen einer Verbindung, die durch die Formel (Ia-1) dargestellt wird: 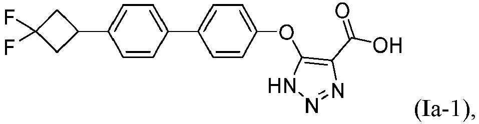 eines Tautomeres davon oder eines Salzes davon, umfassend:
a1) Umwandeln einer Verbindung der Formel (VI-1a-1): 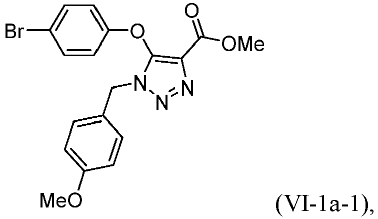 oder eines Salzes davon mit Bis(pinacolato)dibor, Pd(dppf)Cl₂·CH₂Cl₂ und Kaliumacetat in 1,4-Dioxan in eine Verbindung der Formel (IV-1a-2): 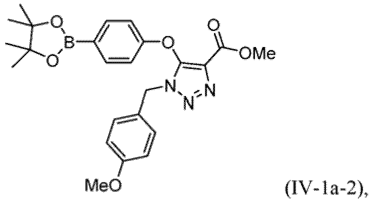 oder ein Salz davon;
a) Inberührungbringen der Verbindung der Formel (IV-la-2) oder des Salzes davon mit 1-Brom-4-(3,3-difluorcyclobutyl)benzol, Pd(dppf)Cl₂·CH₂Cl₂ und Kaliumcarbonat in einer Mischung aus 1,4-Dioxan und Wasser, um eine Verbindung der Formel (IIIa-1a-1): 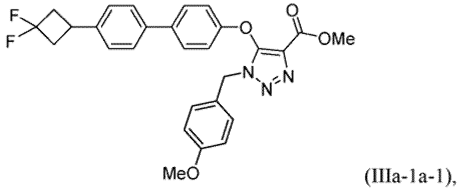 oder ein Salz davon auszubilden;
b) Behandeln der Verbindung der Formel (IIIa-1a-1) oder des Salzes davon mit Trifluoressigsäure und Anisol in Dichlormethan, um eine Verbindung der Formel (IIa-1): 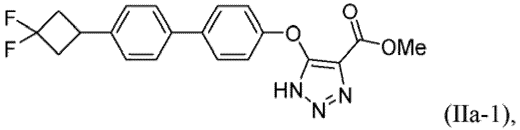 ein Tautomer davon oder ein Salz davon bereitzustellen;
c) Verseifen der Verbindung der Formel (IIa-1), des Tautomeres davon oder des Salzes davon mit einer wässrigen Lösung von Natriumhydroxid in Tetrahydrofuran; und
d) Ansäuern mit einer wässrigen Lösung von HCl, um die Verbindung der Formel (Ia-1), das Tautomer davon oder das Salz davon bereitzustellen.

14. Verfahren nach Anspruch 13, wobei:
(1) Schritt a1) bei einer Temperatur von 80 °C durchgeführt wird; Schritt a) bei einer Temperatur von 80 °C durchgeführt wird; Schritt b) bei einer Temperatur von 50 °C durchgeführt wird; und Schritt c) bei einer Temperatur von 55 °C durchgeführt wird; und Schritt d) bei einer Temperatur von 20-25 °C durchgeführt wird; und/oder
(2) in Schritt a) 1-Brom-4-(3,3-difluorcyclobutyl)benzol in einer Menge von 1,0 bis 1,5 Äquivalenten bezogen auf die Verbindung der Formel (IV-1a-2) vorliegt; und/oder
(3) in Schritt a) Pd(dppf)Cl₂·CH₂Cl₂ in einer Menge von 0,05 bis 0,1 Äquivalenten bezogen auf die Verbindung der Formel (IV-1a-2) vorliegt; und/oder
(4) in Schritt a) Kaliumcarbonat in einer Menge von 2,0 bis 4,0 Äquivalenten bezogen auf die Verbindung der Formel (IV-1a-2) vorliegt; und/oder
(5) die in Schritt a1) hergestellte Verbindung der Formel (IV-1a-2) ohne weitere Reinigung direkt in dem nächsten Schritt verwendet wird; und/oder
(6) die Verbindung der Formel (IIIa-1) in einer kombinierten Ausbeute von wenigstens 50 % aus beiden Schritten a1) und a) isoliert wird; und/oder
(7) die in Schritt b) hergestellte Verbindung der Formel (IIa-1) ohne weitere Reinigung direkt in dem nächsten Schritt verwendet wird; und/oder
(8) Schritt d) durch Ansäuern eines wässrigen Extrakts einer Reaktionsmischung aus Schritt c) durchgeführt wird.

15. Verfahren nach Anspruch 14, wobei das Verfahren ferner umfasst:
e) Umwandeln der Verbindung der Formel (Ia-1), des Tautomeres davon oder des Salzes davon in ein Mononatriumsalz der Verbindung der Formel (Ia-1), die durch die Formel dargestellt wird: 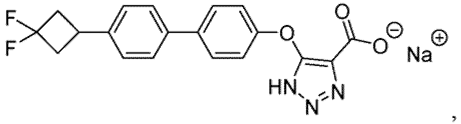 ein Dinatriumsalz der Verbindung der Formel (Ia-1), die durch die Formel dargestellt wird: 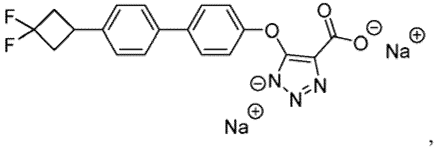 oder einer Mischung davon oder eines Tautomeren davon;
vorzugsweise wobei Schritt e) durchgeführt wird durch:
i) Behandeln der Verbindung der Formel (Ia-1) oder eines Tautomeres davon mit einer wässrigen Lösung von Natriumhydroxid in Wasser;
ii) Ausbilden einer Aufschlämmung mit einem pH-Wert von 9,5; und
iii) Gefriertrocknen der Aufschlämmung, um das Mononatriumsalz der Verbindung der Formel (Ia-1), das durch die Formel dargestellt wird: 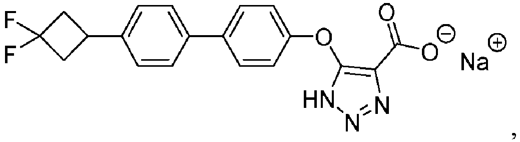 oder eines Tautomeren davon,
wobei Natriumhydroxid in einer Menge von weniger als 1,0 Äquivalent bezogen auf die Verbindung der Formel (Ia-1) auf salzfreier und wasserfreier Basis vorliegt, vorzugsweise wobei Natriumhydroxid in einer Menge von 0,88 Äquivalent bezogen auf die Verbindung der Formel (Ia-1) auf salzfreier und wasserfreier Basis vorliegt.

## Revendications

1. Procédé de préparation d'un composé représenté par la formule (II) : 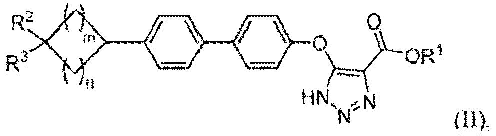 d'un tautomère de celui-ci ou d'un sel de celui-ci, comprenant :
a) la mise en contact d'un composé de formule (IV) : 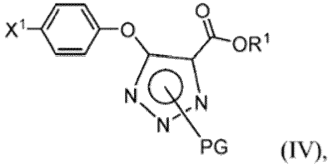 ou d'un sel de celui-ci, avec un composé de formule (V) : 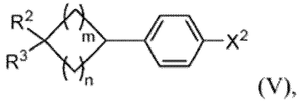 un premier catalyseur à base de métal de transition et une première base dans un premier solvant pour former un composé de formule (III) : 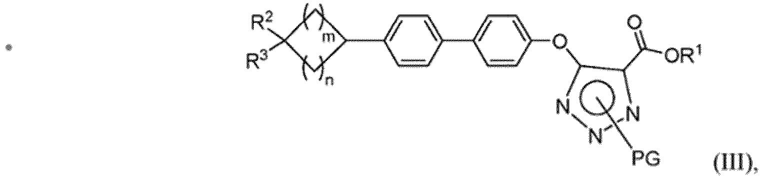 ou un sel de celui-ci ; et
b) l'élimination du groupe PG hors du composé de formule (III) ou du sel de celui-ci pour fournir le composé de formule (II), un tautomère ou sel de celui-ci,
où:
les indices m et n valent chacun indépendamment 1 ou 2 ;
R¹ représente alkyle en C₁₋₆ ;
R² et R³ représentent chacun indépendamment H ou un halogène ;
X¹ représente un groupe contenant du bore ;
X² représente un halogène ou un sulfonate ; 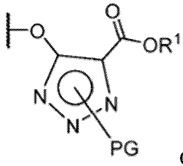 dans la formule (IV) ou (III) est représenté par la formule : 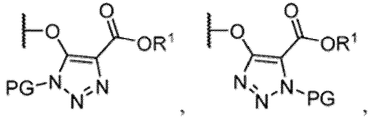
ou un mélange de ceux-ci ; et
PG est un groupe protecteur d'amine.

2. Procédé selon la revendication 1, dans lequel :
(i) le groupe protecteur d'amine est [2-(triméthylsilyl)éthoxy]méthyle, 4-méthoxybenzyle ou 2,4-diméthoxybenzyle, ledit groupe protecteur d'amine étant de préférence 4-méthoxybenzyle ; et/ou
(ii) X¹ est représenté par la formule :
1) -BY₂, où Y représente -OH, un groupe alkyle en C₁₋₆, alcoxy en C₁₋₆, aryloxy en C₆₋₁₀ ou carboxylate ;
2) -BY, où Y représente un groupe alcoxy en C₂₋₈ bidenté, un groupe aryloxy en C₆₋₁₀ bidenté, ou
un groupe carboxylate bidenté ;
3) un groupe 9-borabicyclo[3,3,1]nonane (9-BBN) ;
4) -BY₃M, où Y représente F ou alcoxy en C₁₋₆ et M représente un ion de métal alcalin, un ion ammonium ou un ion phosphonium ; ou
5) -BYM, où Y représente un groupe alcoxy tridenté en C₃₋₁₀ et M représente un ion de métal alcalin, un ion ammonium ou un ion phosphonium,
X1 représentant de préférence -B(OH)₂, -B(OEt)₂, -B(O*i*Pr)₂, -BF₃M, -B(O*i*Pr)₃M, - B(O*i*Pr)₃M, 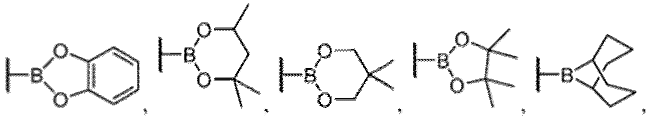 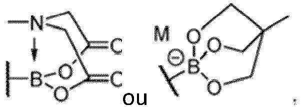 où M représente Li⁺, Na⁺ ou K⁺;
X¹ étant de préférence représenté par la formule : 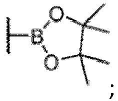 et/ou
(iii) X² représente Cl, Br, I, OMs, OTs ou Otf ; X² représentant de préférence Br ; et/ou
(iv) le premier catalyseur à base de métal de transition est un premier catalyseur au palladium, un catalyseur au ruthénium, un catalyseur au rhodium, un catalyseur au cobalt, un catalyseur au nickel, un catalyseur au fer, un catalyseur au cuivre ou une combinaison de ceux-ci,
ledit premier catalyseur au palladium étant de préférence Pd(acac)₂, [Pd(allyl)Cl]₂, Pd(CH₃CN)₂Cl₂, Pd(dba)₂, Pd(CH₃COO)₂, Pd₂(dba)₃, Pd₂(dba)₃·CHCl₃, Pd(PPh₃)₄, Pd(OAc)₂, Pd(PCy₃)₂Cl₂, Pd(PPh₃)₂Cl₂, Pd[P(o-tol)₃]₂Cl₂, Pd(amphos)Cl₂, Pd(dppf)Cl₂, Pd(dppf)Cl₂·CH₂Cl₂, Pd(dtbpf)Cl₂, Pd(CH₃CN)₄(BF₄)₂, PdCl₂, XPhos-Pd-G3, Pd-PEPPSI^{™}-IPr, Pd-PEPPSI^{™}-SIPr ou Pd-PEPPSI^{™}-IPent,
ledit premier catalyseur au palladium étant de préférence Pd(dppf)Cl₂, Pd(dppf)Cl₂·CH₂Cl₂ ou Pd(dtbpf)Cl₂ ; et/ou
(v) le premier catalyseur à base de métal de transition est présent en quantité sous-stœchiométrique ; et/ou
(vi) la première base est le carbonate de sodium, le carbonate de potassium, le carbonate de césium, le phosphate de sodium tribasique, le phosphate de potassium tribasique, l'acétate de sodium, l'acétate de potassium, l'acétate de césium ou une combinaison de ceux-ci, ladite première base étant de préférence le carbonate de potassium ; et/ou
(vii) le composé de formule (V) est présent en une quantité de 1,0 à 2,0 équivalents, de 1,1 à 2,0 équivalents, de 1,1 à 1,5 équivalent ou de 1,2 équivalent par rapport au composé de formule (IV), ledit composé de formule (V) étant de préférence présent en une quantité de 1,2 équivalent par rapport au composé de formule (IV) ; et/ou
(viii) le premier solvant est de l'eau, l'alcool en C₁₋₄, le benzène, le toluène, le dioxane, le tétrahydrofurane (THF), le 2-méthyltétrahydrofurane (MeTHF), l'acétonitrile (ACN), la N-méthylpyrrolidone (NMP), le N,N-diméthylformamide (DMF), le N,N-diméthylacétamide (DMAC), le diméthoxyéthane (DME), l'éthylène glycol, ou une combinaison de ceux-ci, ledit premier solvant comprenant de préférence le dioxane et de l'eau.

3. Procédé selon la revendication 1 ou 2, dans lequel l'étape a) est réalisée à une température allant de 60 °C à 110 °C, de 70 °C à 110 °C, de 70 °C à 100 °C, de 70 °C à 90 °C, ou de 80 °C, l'étape a) étant de préférence réalisée à une température de 80 °C.

4. Procédé selon l'une quelconque des revendications 1 à 3, dans lequel, à l'étape b), le groupe PG est éliminé par traitement avec un premier acide dans un deuxième solvant, de préférence de l'acide trifluoroacétique, et éventuellement dans lequel :
le deuxième solvant est le dichlorométhane ou le 1,2-dichloroéthane, et/ou
dans lequel un mélange réactionnel de l'étape b) comprend en outre un agent de transfert, ledit agent de transfert étant de préférence l'anisole.

5. Procédé selon l'une quelconque des revendications 1 à 4, comprenant en outre :
c) la mise en contact du composé de formule (II), d'un tautomère ou sel de celui-ci avec une deuxième base dans un troisième solvant ; et
d) une acidification avec un deuxième acide pour fournir un composé de formule (I) : 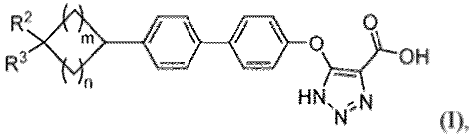
un tautomère de celui-ci, ou un sel de celui-ci.

6. Procédé selon la revendication 5, dans lequel :
(i) la deuxième base est l'hydroxyde de lithium, l'hydroxyde de sodium ou l'hydroxyde de potassium, ladite deuxième base étant de préférence l'hydroxyde de sodium ; et/ou
(ii) le troisième solvant est de l'eau, un alcool en C₁₋₄, le dioxane, le tétrahydrofurane (THF), le 2-méthyltétrahydrofurane (MeTHF), l'acétonitrile (ACN), le diméthoxyéthane (DME) ou une combinaison de ceux-ci, ledit troisième solvant comprenant de préférence le tétrahydrofurane et de l'eau ; et/ou
(iii) le deuxième acide est HCl ; et/ou
(iv) l'étape d) est réalisée dans une solution aqueuse.

7. Procédé selon l'une quelconque des revendications 1 à 6, dans lequel :
(i) le composé de formule (II) est représenté par la formule choisie dans le groupe constitué de : 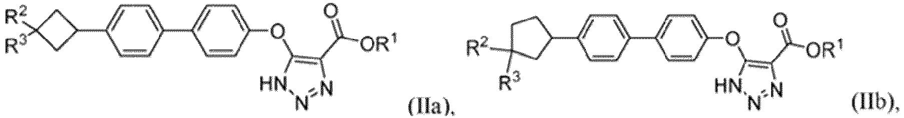 et 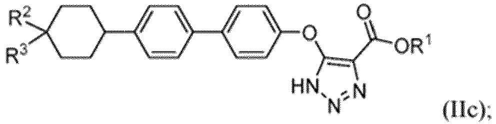 et/ou
(ii) R¹ représente un méthyle ; et/ou
(iii) R² et R³ représentent chacun indépendamment un halogène, R² et R³ représentant de préférence chacun F.

8. Procédé selon la revendication 7, dans lequel le composé de formule (II) est représenté par la formule :

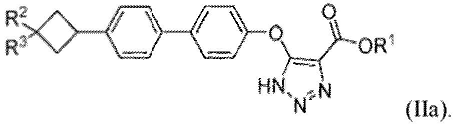

9. Procédé selon l'une quelconque des revendications 5 à 8, dans lequel le composé de formule (I) est représenté par la formule choisie dans le groupe constitué de : 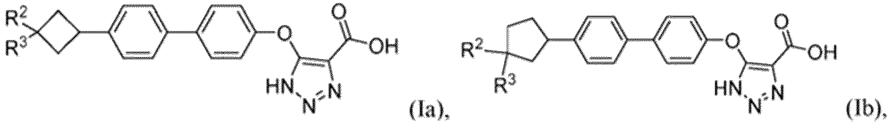 et

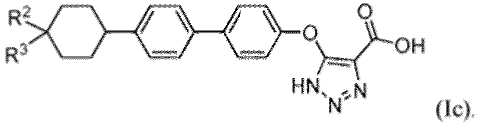

10. Procédé selon la revendication 9, dans lequel le composé de formule (I) est représenté par la formule :

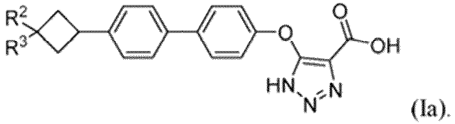

11. Procédé selon l'une quelconque des revendications 1 à 10, comprenant en outre, avant l'étape a) :
a1) la mise en contact d'un composé de formule (VI) : 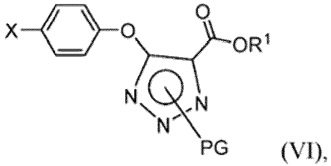 ou d'un sel de celui-ci, avec un réactif au bore, un deuxième catalyseur au palladium et une troisième base dans un quatrième solvant pour former le composé de formule (IV) ou un sel de celui-ci ; X représentant CI, Br ou I.

12. Procédé selon la revendication 11, dans lequel :
(i) le réactif au bore est le tétrahydroxydibore, le bis(catécholato)dibore, le bis(hexylène glycolato)dibore, le bis(néopentyl glycolato)dibore ou le bis(pinacolato)dibore ; ledit réactif au bore étant de préférence le bis(pinacolato)dibore ; et/ou
(ii) le deuxième catalyseur au palladium est Pd(dppf)Cl₂, Pd(dppf)Cl₂·CH₂Cl₂ ou Pd(dtbpf)Cl₂ ; et/ou
(iii) la troisième base est l'acétate de potassium ; et/ou
(iv) le quatrième solvant est le 1,4-dioxane ; et/ou
(v) le composé de formule (VI) est représenté par la formule :

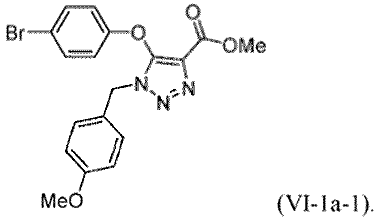

13. Procédé selon la revendication 1 pour la préparation d'un composé représenté par la formule (la-1) : 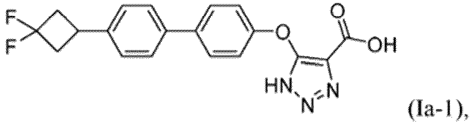 d'un tautomère de celui-ci ou d'un sel de celui-ci, comprenant :
a1) la conversion d'un composé de formule (VI-1a-1) : 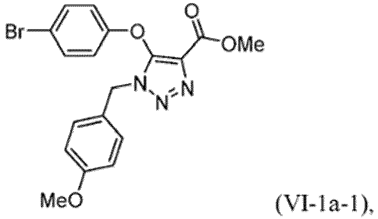 ou d'un sel de celui-ci, avec du bis(pinacolato)dibore, du Pd(dppf)Cl₂·CH₂Cl₂ et de l'acétate de potassium dans du 1,4-dioxane en un composé de formule (IV-1a-2) : 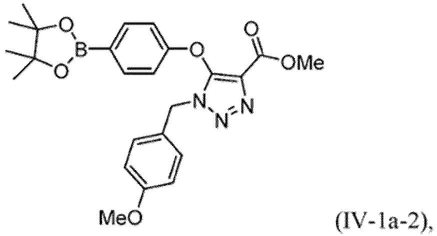 ou un sel de celui-ci ;
a) la mise en contact du composé de formule (IV-1a-2) ou du sel de celui-ci avec du 1-bromo-4-(3,3-difluorocyclobutyl)benzène, du Pd(dppf)Cl₂·CH₂Cl₂ et du carbonate de potassium dans un mélange de 1,4-dioxane et d'eau pour former un composé de formule (Illa-1a-1) : 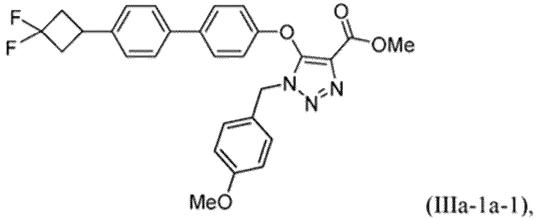 ou un sel de celui-ci ;
b) le traitement du composé de formule (IIIa-1a-1) ou du sel de celui-ci avec de l'acide trifluoroacétique et de l'anisole dans du dichlorométhane pour fournir un composé de formule (Ila-1) : 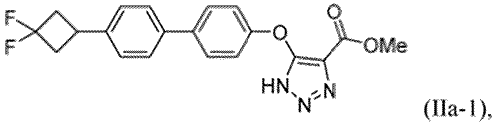 un tautomère de celui-ci ou un sel de celui-ci ;
c) la saponification du composé de formule (Ila-1), du tautomère ou sel de celui-ci avec une solution aqueuse d'hydroxyde de sodium dans du tétrahydrofurane ; et
d) une acidification avec une solution aqueuse de HCl pour fournir le composé de formule (la-1), le tautomère ou sel de celui-ci.

14. Procédé selon la revendication 13, dans lequel :
(1) l'étape a1) est réalisée à une température de 80 °C ; l'étape a) est réalisée à une température de 80 °C ; l'étape b) est réalisée à une température de 50 °C ; l'étape c) est réalisée à une température de 55 °C ; et l'étape d) est réalisée à une température de 20 à 25 °C ; et/ou
(2) à l'étape a), le 1-bromo-4-(3,3-difluorocyclobutyl)benzène est présent en une quantité de 1,0 à 1,5 équivalent par rapport au composé de formule (IV-1a-2) ; et/ou
(3) à l'étape a), Pd(dppf)Cl₂·CH₂Cl₂ est présent en une quantité de 0,05 à 0,1 équivalent par rapport au composé de formule (IV-1a-2) ; et/ou
(4) à l'étape a), le carbonate de potassium est présent en une quantité de 2,0 à 4,0 équivalents par rapport au composé de formule (IV-1a-2) ; et/ou
(5) le composé de formule (IV-1a-2) produit par l'étape a1) est utilisé directement dans l'étape suivante, sans purification supplémentaire ; et/ou
(6) le composé de formule (IIIa-1) est isolé avec un rendement combiné d'au moins 50 % à l'issue des deux étapes a1) et a) ; et/ou
(7) le composé de formule (IIa-1) produit par l'étape b) est utilisé directement dans l'étape suivante, sans purification supplémentaire ; et/ou
(8) l'étape d) est réalisée par acidification d'un extrait aqueux d'un mélange réactionnel de l'étape c).

15. Procédé selon la revendication 14, ledit procédé comprenant en outre :
e) la conversion du composé de formule (la-1), du tautomère ou sel de celui-ci en un sel monosodique du composé de formule (la-1) représenté par la formule : 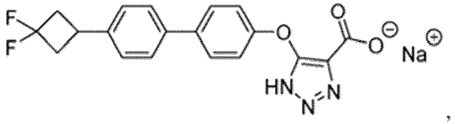 un sel disodique du composé de formule (la-1) représenté par la formule : 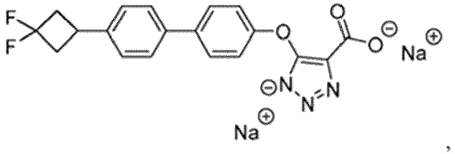 ou un mélange de ceux-ci, ou un tautomère de ceux-ci ;
l'étape e) étant de préférence réalisée par :
i) traitement du composé de formule (la-1) ou d'un tautomère de celui-ci avec une solution aqueuse d'hydroxyde de sodium dans de l'eau ;
ii) formation d'une suspension ayant un pH de 9,5 ; et
iii) lyophilisation de la suspension pour fournir le sel monosodique du composé de formule (la-1) représenté par la formule : 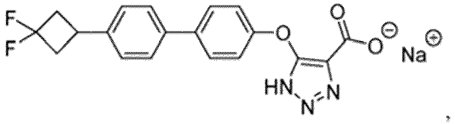 ou un tautomère de celui-ci,
l'hydroxyde de sodium étant présent en une quantité inférieure à 1,0 équivalent par rapport au composé de formule (la-1), sur une base sans sel et anhydre ; l'hydroxyde de sodium étant de préférence présent en une quantité de 0,88 équivalent par rapport au composé de formule (la-1), sur une base sans sel et anhydre.
